(19) Europäisches Patentamt
European Patent Office
Office européen des brevets

(11) **EP 4 711 442 A1**

(12) **EUROPEAN PATENT APPLICATION**

(43) Date of publication:
**18.03.2026 Bulletin 2026/12**

(21) Application number: **25197101.6**

(22) Date of filing: **20.08.2025**

(51) International Patent Classification (IPC):
**C12M 3/06** (2006.01) **C12M 1/00** (2006.01)

(52) Cooperative Patent Classification (CPC):
**C12M 23/16; C12M 23/58**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **15.09.2024 US 202463694903 P**
**19.08.2025 CA 3283419**

(71) Applicant: **Quantitative Biosciences, Inc.**
**San Diego, CA 92121 (US)**

(72) Inventors:
• **COOKSON, Natalie Anne**
**Encinitas, 92024 (US)**

• **COOKSON, Scott Warren**
**Encinitas, 92024 (US)**
• **CSICSERY, Nicholas Sigmund**
**San Diego, 92131 (US)**
• **FERRY, Michael Stephen**
**San Diego, 92127 (US)**
• **MATHER, William H.**
**Birmingham, 48009 (US)**
• **SAVAGE, April Heather**
**San Diego, 92103 (US)**
• **JOHNSON, Ryan Alexander**
**Irvine, 92618 (US)**

(74) Representative: **Latscha Schöllhorn Partner AG**
**Grellingerstrasse 60**
**4052 Basel (CH)**

(54) **MICROFLUIDIC CHIP FOR THE PARALLEL CULTURE OF MICROBIAL BIOSENSOR STRAINS**

(57) Disclosed is a microfluidic chip for parallel culture of recombinant biosensing microbes containing a novel design of cell trap complexes set in paired arrays for monitoring process water composition(s) for comparison against reference water(s).

**EP 4 711 442 A1**

**Description**

STATEMENT OF GOVERNMENT LICENSE RIGHTS

This invention was made with government support under SBIR STTR Award Number DE-SC0018575, awarded by the United States Department of Energy. The government has certain rights in the invention.

BACKGROUND OF THE INVENTION

1. Field of the Invention

This invention relates to microfluidic devices and platforms useful for housing biosensor microorganisms and the monitoring of analytes in aqueous samples.

2. Discussion of the Related Art

Access to clean, reliable water supplies is critical to the quality of life and the economy, yet a vast array of contaminants including heavy metals, nutrients, and emerging contaminants of concern threaten the drinking water of millions of people across the United States and other countries, which can cause critical health problems to those who are, often unknowingly, affected. (See, e.g., Mueller et al., "The widespread and unjust drinking water and clean water crisis in the United States," Nature Commun. (2021), 12:3544 , doi.org/10.1038/s41467-021-23898-z; Allaire et al., "National trends in drinking water quality violations," PNAS 115(9):2078-2083 (2018), pnas.org/doi/epdf/10.1073/pnas.1719805115).

Human activities have also accelerated the rate and extent of eutrophication of many freshwater and coastal marine ecosystems throughout the world by both point-source discharges and non-point loadings of limiting nutrients, such as nitrogen and phosphorus, into aquatic ecosystems, with dramatic adverse consequences for drinking water sources, fisheries, and recreational water bodies. (See, e.g., Chislock, M. F., Doster, E., Zitomer, R. A. & Wilson, A. E. (2013) "Eutrophication: Causes, Consequences, and Controls in Aquatic Ecosystems," Nature Education Knowledge 4(4):10, nature.com/scitable/knowledge/library/eutrophication-causes-consequences-and-controls-in-aquatic-102364466; Dodds et al., "Eutrophication of U.S. Freshwaters: Analysis of Potential Economic Damages," Environ. Sci. & Technol. 43(1): (2009) doi: 10.1021/es801217q).

The contaminants at various sites range from common water toxins, such as arsenic and cadmium, to excess nutrients (nitrogenous and phosphorus) and even radionuclides like uranium. Measuring contamination in the environment is critical to human health, but current testing is mostly limited to sporadic sample collection for laboratory analysis. Not only is such assessment costly, but it is inefficient, making it difficult to monitor water quality, e.g., of agricultural run-off and industrial water inputs and outputs with high spatial or temporal resolution.

Current microfluidic devices and analytical methods employing them do not capture the full complexity of how contaminants behave in the environment. (See, e.g., Hasty et al., "Microbial Microfluidic Biosensor," US11209412B2 and US2022/057378A1; Cardemil, C. et al., "Bioluminescent Escherichia coli strains for the quantitative detection of phosphate and ammonia in coastal and suburban watersheds," DNA Cell Biol, vol. 29, no. 9, pp. 519-31

(2010), doi: 10.1089/dna.2009.0984; DeAngelis et al., "Two novel bacterial biosensors for detection of nitrate availability in the rhizosphere," Appl. Environ. Microbiol. (2005), 71(12):8537-47, doi: 10.1128/AEM.71.12.8537-8547.2005; Diawara et al., "Arsenic, cadmium, lead, and mercury in surface soils, Pueblo, Colorado: implications for population health risk," Environ. Geochem. Health (2006), 28(4):297-315, doi: 10.1007/s10653-005-9000-6. Epub 2006 Jun 4; National Research Council of the National Academies, "Alternatives for Managing the Nation's Complex Contaminated Groundwater Sites" (2013); Bricker et al., "Effects of nutrient enrichment in the nation's estuaries: A decade of change," Harmful Algae 8(1):21-32 (2008), doi: 10.1016/j.hal.2008.08.028; Daunert et al., "Genetically engineered whole-cell sensing systems: coupling biological recognition with reporter genes," Chemical Reviews, 2000, 100(7):2705-2737).

When culturing bacterial strains, biological or experimental requirements often dictate a specific chemical composition for the selected growth media. The inclusion, exclusion, or concentration of specific micronutrients can significantly affect observed results. For example, across engineered *E. coli* strains, optimal growth and other observed phenotypes depend heavily on which carbon, nitrogen, or phosphorous sources and concentrations are used. While achieving desired media compositions is trivial for individual batch cultures, problems of scale rapidly present themselves when moving towards parallel continuous culture of many strains.

Past microfluidic solutions have enabled the continuous parallel culture of dozens or up to thousands of unique bacterial strains within a small physical footprint. However, existing microfluidic devices that enable continuous microchemostat growth subject all strains to the same growth conditions. While it is possible to run multiple devices in parallel, each addressing a specific growth condition, equipment availability and scaling issues hinder this approach. (See, e.g., Hasty et al., "Microbial Microfluidic Biosensor," US11209412B2 and US2022/057378A1; Daunert et al., "Spores for the stabilization and onsite application of bacterial whole-cell biosensing systems," US8389263B2 and US2010/0304379A1).

Therefore, it is a desideratum to design a single-device microfluidic solution, enabling the parallel culture of multiple microbial biosensor strains under numerous conditions simultaneously and on a continuous basis.

This the present invention provides.

SUMMARY OF THE INVENTION

The present invention relates to a microfluidic chip (100) for parallel culture, and housing, of recombinant biosensing microbes (i.e., "biosensors"), e.g., a panel of recombinant *Escherichia coli* strains, for monitoring one or a plurality of analytes of interest in aqueous samples of "process waters."

In one aspect, the microfluidic device can house many different biosensor strains," each with the ability to detect a water contaminant or analyte, optionally on a continuous basis for extended periods, with high sensitivity and selectivity,

The inventive microfluidic chip (100) for parallel culture of recombinant biosensing microbes, includes, encompassed by a chip solid matrix (796):

(a) one or more paired microbial process monitoring arrays (110) and microbial reference monitoring arrays (120), each reference monitoring array corresponding to one of the process monitoring array(s), each process monitoring array (110) and each reference monitoring array (120) comprising an upstream end and a downstream end, and further comprising:

(i) an upstream array inlet channel (132, 432, 532), fluidly connected and upstream to one or more upstream manifold channels (200, 402, 502, 602), and thence fluidly connected to:

(ii) a network of branch channels (210, 310, 410, 510, 610), branching in parallel within the process monitoring array(s) (110) and reference monitoring array(s) (120), wherein a cell trap complex (130, 230, 430, 530, 630) is in fluid connection to each branch channel (210, 310, 410, 510, 610), and is capable of receiving fresh liquid cell culture medium from the branch channel; and wherein each branch channel is fluidly connected via a downstream manifold channel (220, 403, 503, 603), which is configured to receive cell-conditioned medium outflow from the branch channel (210, 310, 410, 510, 610), wherein the downstream manifold channels are configured to convey the cell-conditioned medium outflow further downstream to:

(iii) a downstream array outlet channel (135, 235, 435, 535), which is configured to fluidly convey the cell-conditioned medium outflow;

(b) for conveying liquid to the process monitoring array(s) (110), a first inlet port (150, 415, 515), fluidly connected via a first resistance-tuned inlet channel (155), upstream to the first upstream array inlet channel (132, 432, 532), which is fluidly connected to the process monitoring array (110);

(c) for conveying liquid to the reference monitoring array(s) (120), a second inlet port (160, 415, 515), fluidly connected via a second resistance-tuned inlet channel (165), upstream to the second upstream array inlet channel (132, 432, 532), which is fluidly connected to the reference monitoring array (120); and

(d) a waste outlet channel (138, 438, 538), which is configured to fluidly receive the cell-conditioned medium outflow from the downstream array outlet channels (135, 235, 435, 535) and to fluidly convey the outflow to a downstream waste port (140, 470, 570).

The microfluidic chip of the present invention is also characterized in that each cell trap complex (130, 230, 430, 530, 630) comprises:

(i) a cell spotting region (300, 600);

(ii) an adjacent primary cell trap (312, 612), fluidly connected to the cell spotting region by a first migration channel (320, 620) having a broader end (322) and a narrower end (324), wherein the first migration channel tapers from the cell spotting region (300, 600) at the broader end to the primary cell trap (312, 612) at the narrower end, each primary cell trap having a side distal from the cell spotting region open to an adjacent microfluidic branch channel (210, 310, 410, 510, 610);

(iii) a second migration channel (332, 632), fluidly branching from the narrower end (324) of the tapering first migration channel; and

(iv) a plurality of downstream cell traps (340, 640) in a series adjacent to the primary cell trap, each downstream cell trap having a side with an opening to the second migration channel (332, 632) and having a side opposite the second migration channel open to the adjacent microfluidic branch channel (210, 310, 410, 510, 610), each downstream cell trap being capable of housing a viable microbial colony.

In many useful embodiments, the primary cell traps and the downstream cell traps have a transparent ceiling to enable light emissions to be imaged by an external detector (180).

The foregoing summary is not intended to define every aspect of the invention, and additional aspects are described in other sections, such as the Detailed Description of Embodiments. The entire document is intended to be related as a unified disclosure, and it should be understood that all combinations of features described herein are contemplated, even if the combination of features are not found together in the same sentence, or paragraph, or section of this document. In addition to the foregoing, the invention includes, as an additional aspect, all embodiments of the invention narrower in scope in any way than the variations defined by specific paragraphs above. For example, certain aspects of the invention are described as a genus, and it should be understood that every member of a genus is, individually, an aspect of the invention. Also, aspects described as a genus or selecting a member of a genus, should be understood to embrace combinations of two or more members of the genus.

Although the applicant(s) invented the full scope of the invention described herein, the applicants do not intend to claim subject matter described in the prior art work of others. Therefore, in the event that statutory prior art within the scope of a claim is brought to the attention of the applicants by a Patent Office or other entity or individual, the applicant(s) reserve the right to exercise amendment rights under applicable patent laws to redefine the subject matter of such a claim to specifically exclude such statutory prior art or obvious variations of statutory prior art from the scope of such a claim. Variations of the invention defined by such amended claims also are intended as aspects of the invention.

BRIEF DESCRIPTION OF THE DRAWINGS

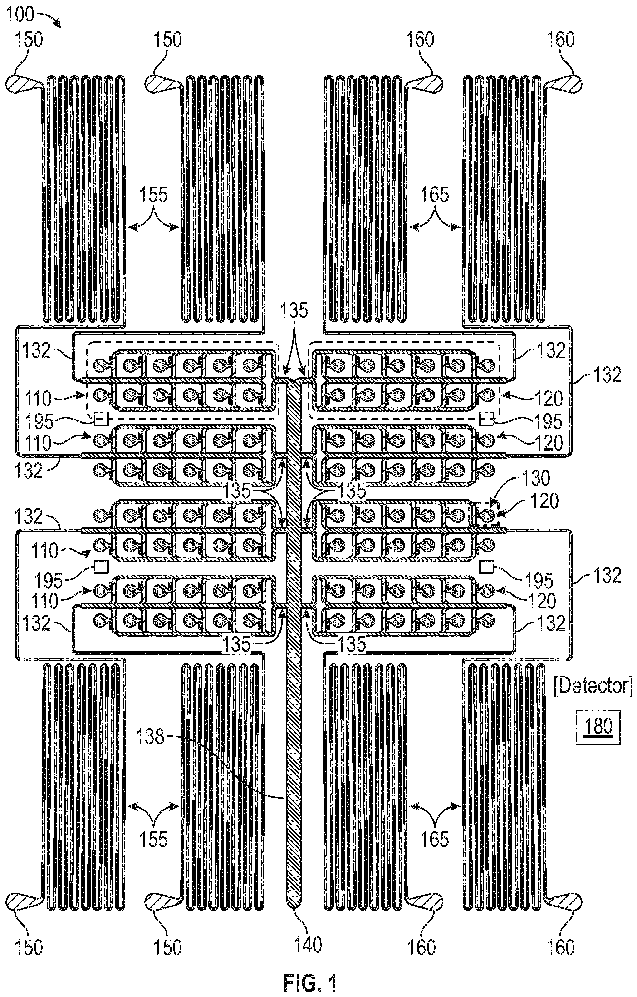

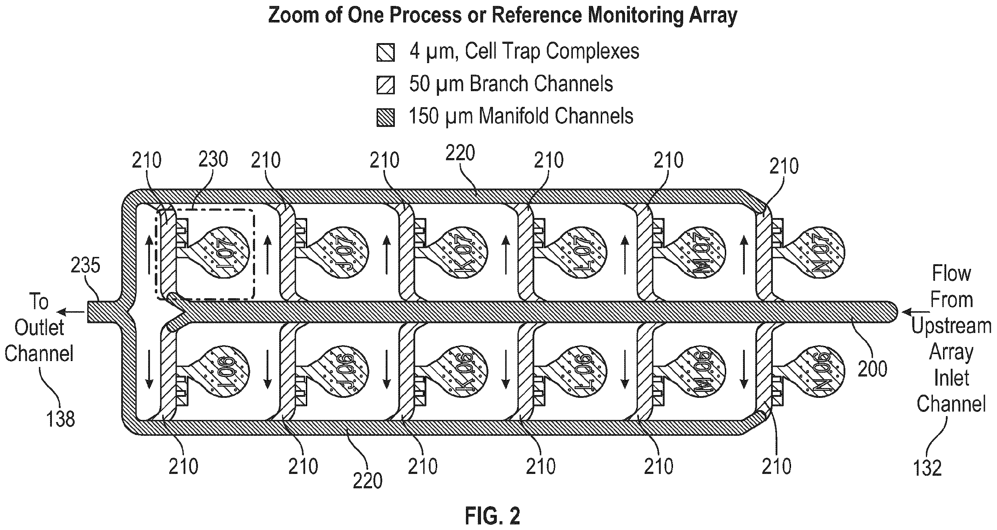

Figure 1 shows a schematic representation of the general architecture of an embodiment of the inventive microfluidic device (100) designed with 4 pairs of process and reference monitoring arrays. Figure 2 shows an expanded view of an exemplary monitoring array (110, 120). The array is structured the same whether it is a process monitoring array (110) or a reference monitoring array (120).

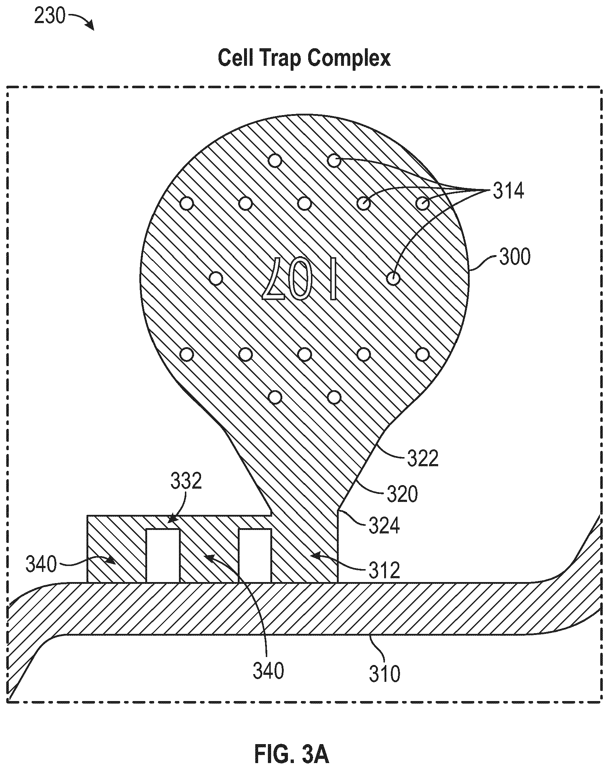

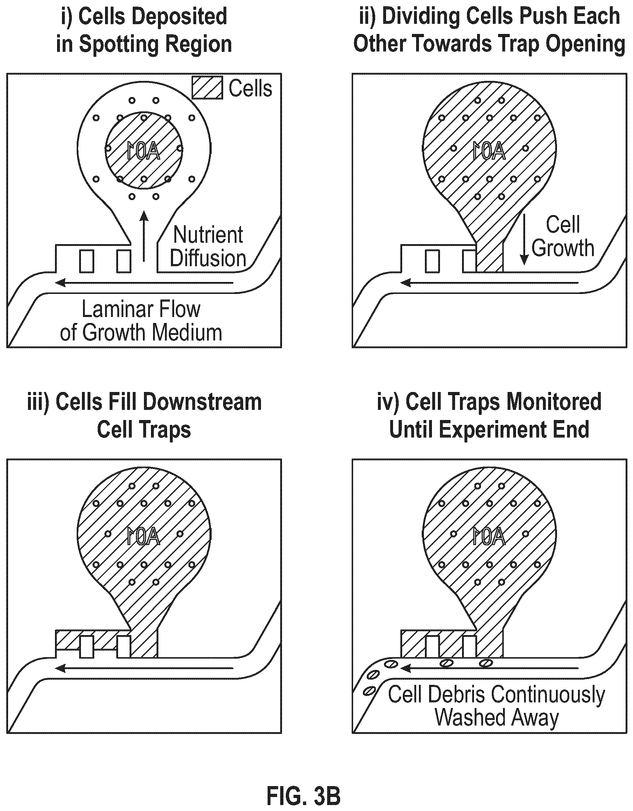

Figure 3A-C depicts a more detailed view of the structure and function of the regions contained within the cell trap complexes.

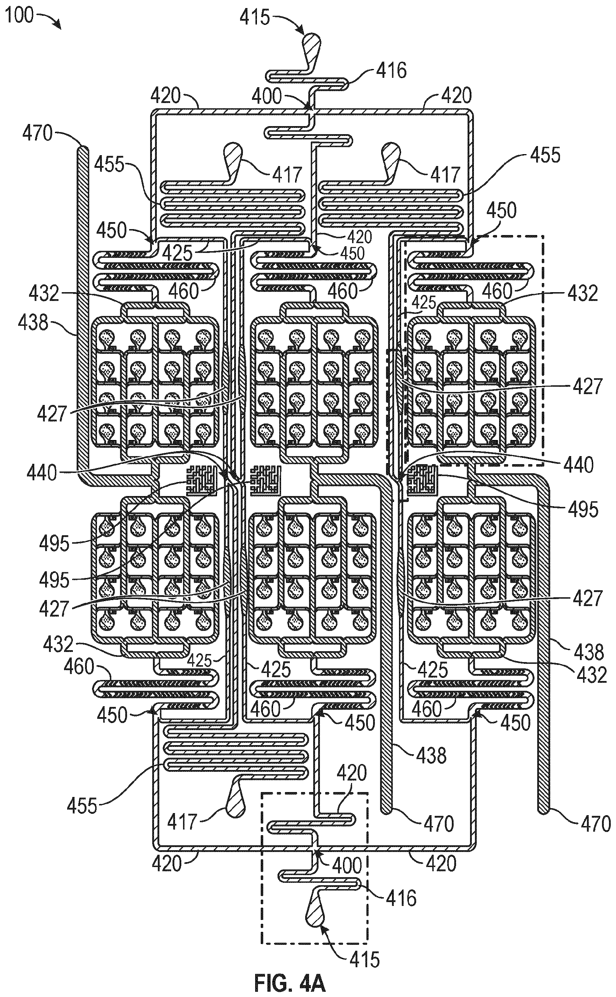

Figure 4A illustrates an embodiment of the inventive chip designed with 3 pairs of process and reference monitoring arrays, where each process water monitoring array receives the same process water from an exterior source, and each reference monitoring array receives the same reference water that matches the background chemical composition of the paired process water.

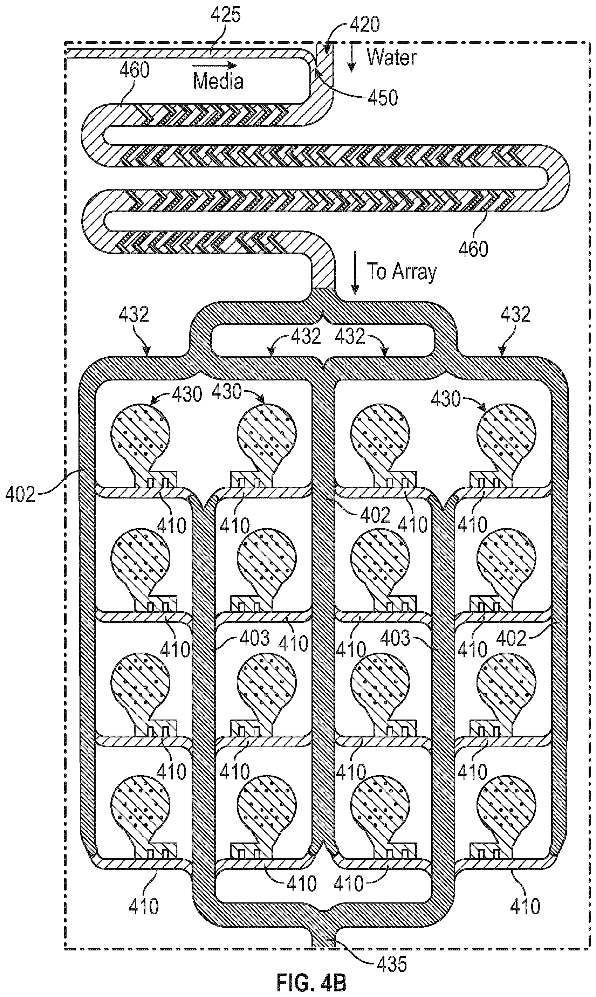

Figure 4B shows an expanded view of a monitoring array and mixing channel (460). In the embodiment of Figure 4A, the cell trap complex (430) remains structurally unchanged from that shown in Figure 3A-C, and the fundamental structure of the monitoring array shown in Figure 2 is the same, albeit with additional upstream manifold channels (402) supplying media to the branch channels (410) which exits trough the downstream manifold channels (403).

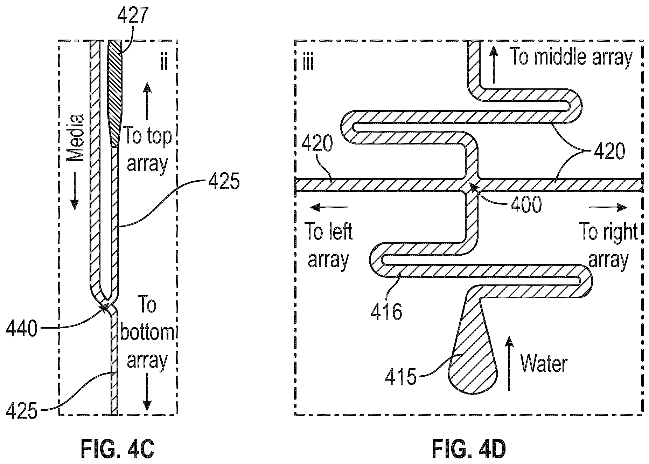

Figure 4C shows an expanded view of one of the media junctions (440) at which each of the three media sources is split into two post-media junction media channels (425).

Figure 4D shows an expanded view of a water junction (400), at which the flow of one of the two water sources (i.e., process water or reference water) is split to flow into three post-water junction water channels (420). The water arrives at the water junction after flowing from an inlet port (415) and via a primary water inlet channel (416).

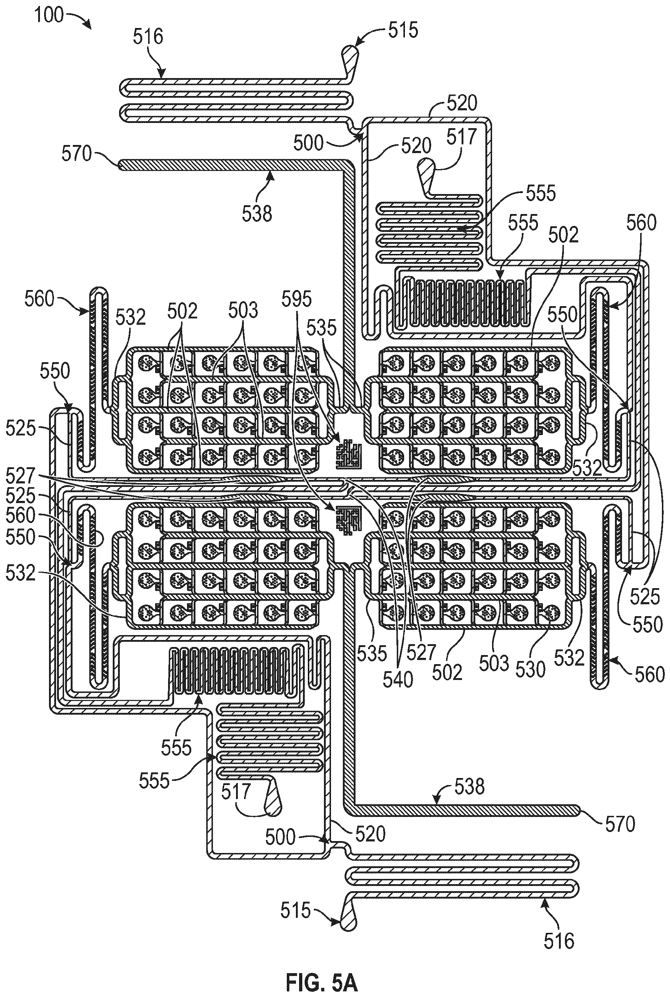

Figure 5A shows a schematic of another embodiment of the chip design with two pairs of microbial process monitoring arrays (110) and microbial reference monitoring arrays (120), wherein each water monitoring array receives water from an exterior source via an inlet port (515).

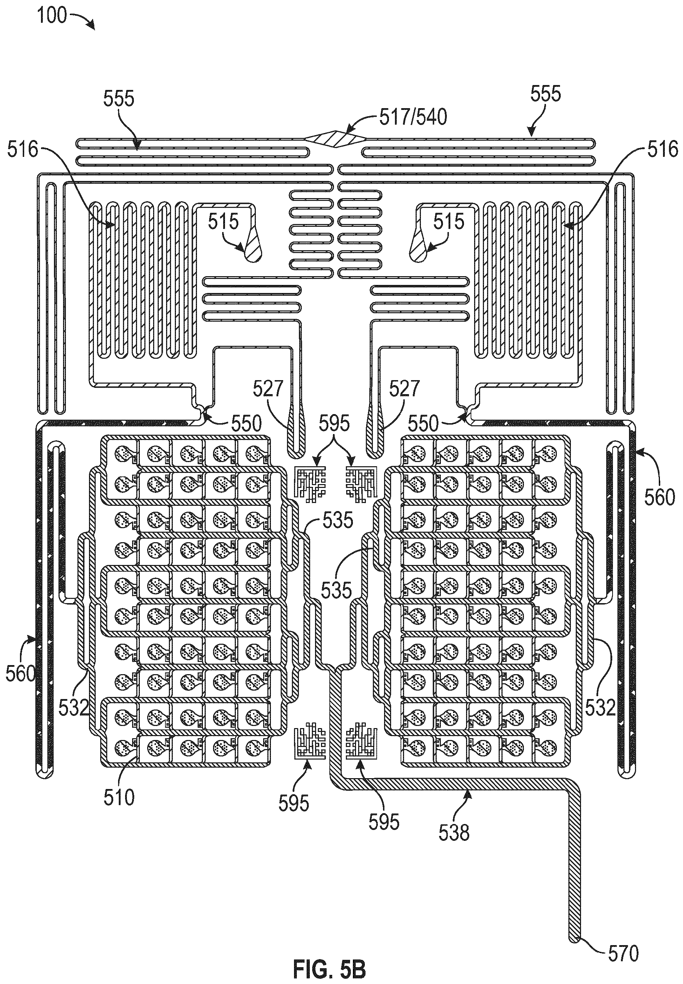

Figure 5B shows a schematic of another embodiment of the chip design with one pair of a microbial process monitoring array (110) and a microbial reference monitoring array (120), wherein each water monitoring array receives water from an exterior source via an inlet port (515).

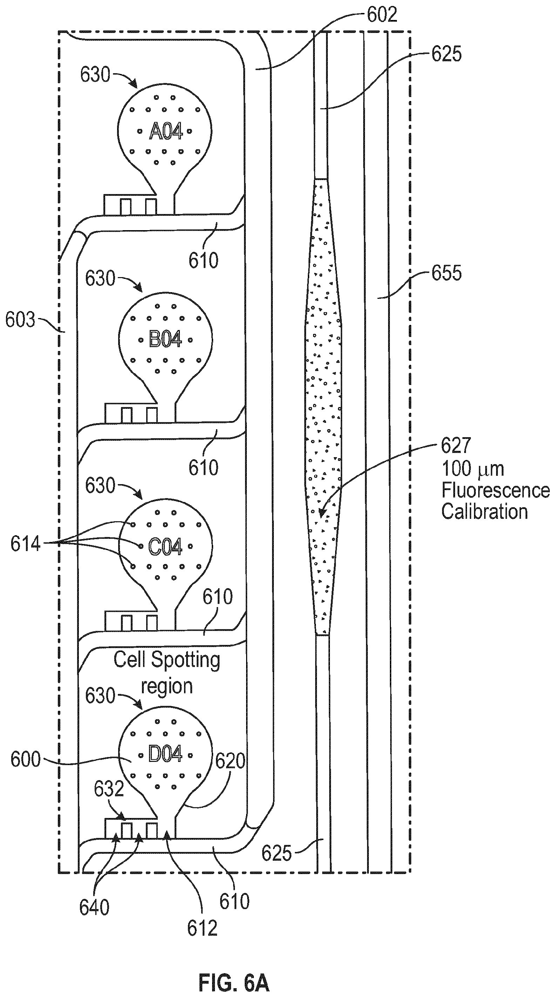

Figure 6A-B shows expanded schematic views from different perspectives, featuring a light emission calibration channel (627); in some embodiments the light emission of interest is fluorescence, and this structure (627) can also be called a "fluorescence calibration channel," which is also illustrated in Figure 4A (427) and Figure 5A-B (527). In this example, support pillars (614) are shown in the cell spotting region (600), but other embodiments of the chip (100) made from glass or other rigid material, such as a thermoplastic material, do not require support pillars.

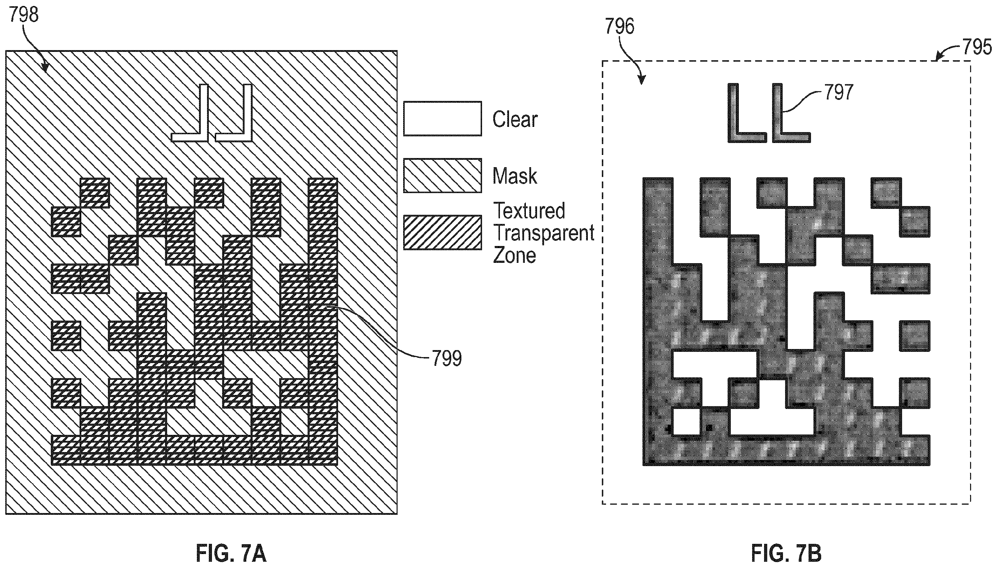

Figure 7A-B shows a schematic enlargement of a data matrix (795) that is useful for assessing the chip orientation for imaging and data analysis. In Figure 7A, the hatched textured transparent zone (799) of the mask (798) is shown with horizontal lines (as well as with leftward diagonal striations); these horizontal lines represent a high contrast, light-refractive pattern on the mask (798), which, in the microfluidic chip fabrication process, is translated into the data matrix (795) in an external surface of the chip solid matrix (796), as shown in Figure 7B.

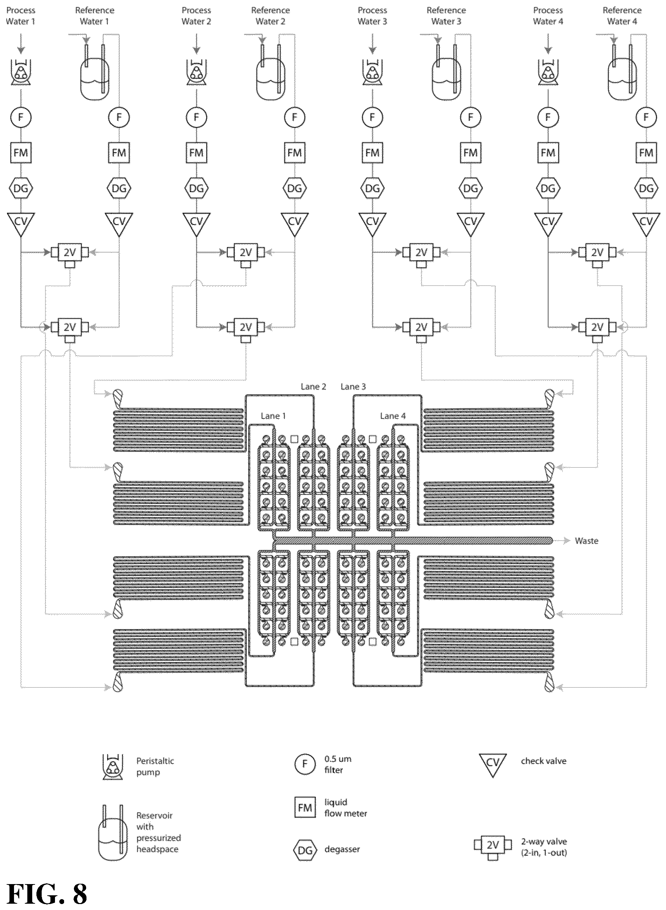

Figure 8 illustrates schematically an embodiment of a hardware assembly connecting the inlet ports (illustrated in Figure 8 as striated bulb-like structures at the left and right edges of the chip), in an embodiment of the microfluidic chip as shown in Figure 1, with the upstream water sources and/or reservoirs containing process waters or reference waters, respectively. In this particular embodiment, the defined culture medium is pre-mixed into the process and reference waters. In other embodiments (see, e.g., Figure 4A, Figure 5A-B), mixing occurs on-chip, with separate medium reservoirs being connected to the media inlet port(s) of the microfluidic chip. Additional outputs of the MATLAB optimizer code were used to determine the quantity of each "ingredient" chemical to add to construct each synthetic reference water shown in Figure 8.

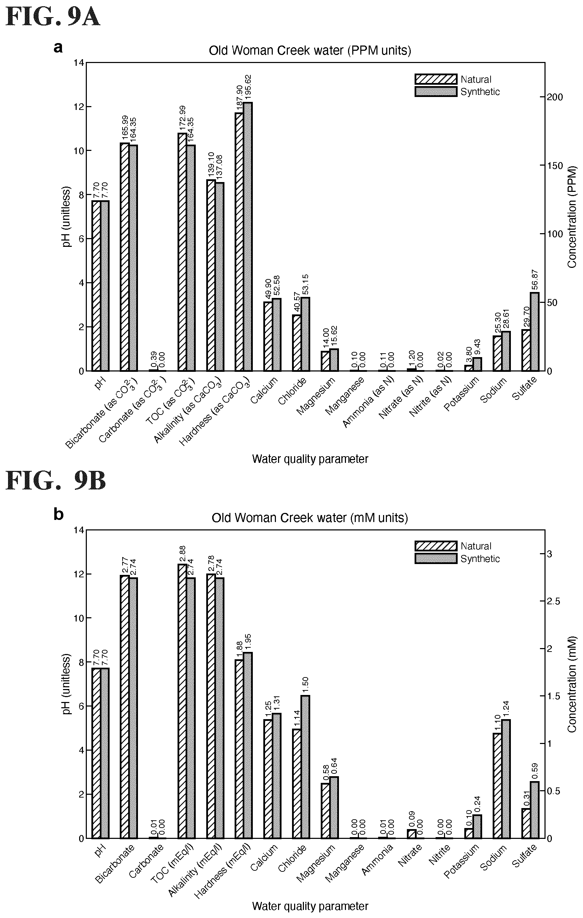

Figure 9A-B illustrates a comparison of naturally occurring (hatched bars) and synthetic (solid bars) Old Woman Creek (OWC) waters. We formulated a synthetic water recipe to match average values of a set of water quality parameters collected at the OWC Wetland Mouth site by the Ohio Department of Natural Resources during the years 1987-2022. Figure 9A shows parameters in concentration units of PPM. Figure 9B shows parameters in concentration units of mM.

## DETAILED DESCRIPTION OF EMBODIMENTS

The section headings used herein are for organizational purposes only and are not to be construed as limiting the subject matter described.

Definitions

Unless otherwise defined herein, scientific and technical terms used in connection with the present application shall have the meanings that are commonly understood by those of ordinary skill in the art. Further, unless otherwise required by context, singular terms shall include pluralities and plural terms shall include the singular. Thus, as used in this specification and the appended claims, the singular forms "a", "an" and "the" include plural referents unless the context clearly indicates otherwise. For example, reference to "a protein" includes a plurality of proteins; reference to "a cell" includes populations of a plurality of cells.

The present invention involves a "microfluidic device," or a "microfluidic chip," or simply a "chip" (terms used interchangeably herein) for parallel culture of recombinant biosensing microbes. For purposes of the invention, a "microbe" or "microbial cell" (used interchangeably herein) is a microscopic prokaryotic cell or eukaryotic cell that is cultured individually, or as part of a population of like cells of a cell line or strain cultured in a bioreactor or colony, but not as part of a tissue within a multicellular organism. The microfluidic chips of the invention are designed to house colonies of recombinant biosensing microbes. Examples of "microbial cells" include cultured bacterial cells, yeast cells, algal or microalgal cells, mammalian (e.g., HEK 293 or CHO) cells, or insect cells.

"Process water" is an aqueous chemically complex mixture, or preferably a solution, from an exterior source, containing an analyte(s) of interest. The process water is typically drawn from an exterior source, such as an environment of interest, e.g., a natural, agricultural, or industrial process water; advantageously, but not necessarily, the microfluidic device of the invention is placed in situ in the environment of interest. If the process water is a suspension, containing particulates and/or microbial cells, the process water must be filtered, to remove these particulates and microbes in the mixture. The flow of process water from the environment into the inlet port(s) of the inventive chip can be induced by using positive pressure (e.g., by using a bladder pump) or negative pressure (e.g., by using a peristaltic pump). However, applying negative pressure to the process water can cause the outgassing of dissolved gas from the process water, and, thus, passive or active degassing of the process water may be needed to remove gas bubbles from the flow stream upstream of the microfluidic chip. The complex mixture of process water may contain salts, minerals, organics, and particulates. When particles are present in process water, they are generally removed by filtration (e.g., 0.2 $\mu$m pore size) upstream of the microfluidic chip to prevent clogging of microchannels. Exemplary sources of process water include aqueous solutions used in or resulting from pharmaceutical production processes and cell cultivation, or environmental waters, such as groundwater or agricultural run-off, or process waters, input to, or output from, wastewater treatment processes.

"Reference water" is a synthetic formulated aqueous chemically complex mixture, or preferably a solution, that matches the background chemical composition of the process water to which it is paired, without the analyte(s) of interest. Being formulated and defined, the reference water can be said to be from an "interior source." Each reference water is paired with a process water and is formulated to serve as a reference for sensing analytes in that process water. The transcriptional response of an engineered microbial biosensor strain is highly complex and influenced by many factors in its environment; therefore, we match the reference water background to the process water as closely as possible in order to preclude nonspecific cellular responses due to differences in other water quality parameters. By measuring the differential response of the strain between process and reference waters, we remove nonspecific responses due to the sensing medium. Typically, the background chemical composition of the process water is known, or else it can be determined by analysis. In particular, we aim to match common water quality parameters such as pH, bicarbonate (as $CO_3^{2-}$), carbonate (as $CO_3^{2-}$), total organic carbon (TOC, as $CO_3^{2-}$), alkalinity (as $CaCO_3$), hardness (as $CaCO_3$), calcium, chloride, magnesium, manganese, ammonia (as N), nitrate (as N), nitrite (as N), potassium, sodium, and sulfate. An example of the formulating of a reference water is shown hereunder in Figure 9A-B. The water, and all other ingredients that are used to make formulations of reference waters, are preferably of a high level of purity meeting the applicable legal or pharmacopoeial standards required, such as the minimum quality of source or feed water for the generation of Water for Injection is Drinking Water as defined by the U.S. Environmental Protection Agency (EPA), EU, Japan, or WHO. For example, we used the Millipore Direct-Q® 5 UV Remote Water Purification System as a source of purified water for reference water formulations. American Chemical Society (ACS) reagent grade chemical ingredients were employed in making reference waters, generally greater than 97% pure, typically greater than 99% pure. For example, the "OWC" reference water was formulated (as illustrated in Figure 9A-B), with the following ingredients:

12.82 mg $MgSO_4$ (Sigma-Aldrich #746452);
8.00 mg $CaSO_4$ (Sigma-Aldrich #237132);
125.42 mg $CaCO_3$ (Sigma-Aldrich #398101);
51.05 mg $MgCl_2$ (Sigma-Aldrich #M8266);
104.55 mg $NaHCO_3$ (Fisher Scientific #S233);
24.14 mg $KHCO_3$ (Sigma-Aldrich #237205);
35.14 $\mu$l 36.9% HCl (Sigma-Aldrich #320331);
23.45 $\mu$l 97% $H_2SO_4$ (Sigma-Aldrich #258105);
remaining 1-L volume filled with Milli-Q water.

To measure the differential response of the microbial biosensor strains between process water and reference water, the microbial biosensor strains are alternately exposed to the process water and the reference water, over a pre-selected time period. This is accomplished by the inventive microfluidic chip design which contains replicate microbial process monitoring array(s) and microbial reference monitoring array(s), where each array receives water (i.e., process water or reference water) from a dedicated inlet port. Upstream of the microfluidic chip, valving is used to alternate the flows of process water and reference water to, respectively, the first and second inlet ports of the chip, which "alternatively receive" process water or reference water at a preselected periodicity. Thus, the cell trap complexes of each array are alternately exposed to either process water or reference water, and the microbial biosensor strain response can be interpreted as a system response to a continuous sequence of step functions (i.e., a rectangular wave when taken in aggregate) changing between two states in water composition. "Step function" and "rectangular wave" are terms understood in the art of signal analysis, commonly used in the field of electrical engineering to describe how a system translates an input signal to an output signal. Due to the typical timescale of a cellular transcriptional response, a pre-selected switching period (typically a period 1 to 8 hours) is selected to allow the cells to generate a measurable transcriptional response before stepping back to the alternative water source. "Cell-conditioned medium outflow" is the aqueous flow outward from the cell trap complexes of the chip; cell-conditioned medium outflow includes both cell-conditioned culture medium containing metabolic waste from cells retained in the cell trap complexes and sloughed cells from cellular outgrowth of the exponentially-expanding culture mass in each cell trap complex. Conditioned medium outflow flows out of the microbial process monitoring array(s) and microbial reference monitoring array(s), ultimately as effluent from the waste outlet channel(s) and the downstream waste port(s) of the chip. "Orientation" means registration of data from the external detector for analysis. The orientation of the microfluidic chip can be determined by comparison to a keyframe image.

An "analyte" is a chemical substance that is the subject of identification and/or measurement, e.g., ammonium, nitrate, nitrite, phosphate, a manganese dication, an iron cation, or a combination of any of these. In some embodiments, one or more paired microbial process monitoring arrays (110) and microbial reference monitoring arrays (120), contains recombinant microbial biosensor strains, such as but not limited to, *Escherichia coli* strains, for monitoring a plurality of analytes of interest in aqueous samples of "process water(s)". (See, e.g., Cookson et al., "Engineered Biosensor Strains of E. Coli for Continuous Aerobic Detection of Analytes," PCT/US2024/025712). Each array can include a set of one engineered microbial strain, at least 2 different engineered microbial strains, 3 different engineered microbial strains, 4 different engineered microbial strains, 5 different engineered microbial strains, 6 different engineered microbial strains, or 7 different engineered microbial strains, or more. Each microbial biosensor strain may be engineered for sensitivity to a particular analyte of interest. Embodiments of the inventive microfluidic devices can be stored with the microbial strains already in place in the cell traps in lyophilized (freeze-dried) form, or otherwise dehydrated, e.g., by air-drying. (See, e.g., Hasty et al., "Microbial Microfluidic Biosensor," US11209412B2). After being in a dehydrated state for at least 30-60 days, we have found that the engineered microbial biosensor strains are capable of being revived under aqueous physiological conditions, and they capable of expressing the optically detectable marker in the presence of at least one of the plurality of analytes of interest. Alternatively, the engineered microbial biosensor strains can be added to the cell spotting region (300), e.g., by hand or by the use of an automated pin tool, hours or minutes before the microfluidic device is placed *in situ* for water monitoring.

The microfluidic devices, or "chips," are suitable for continuously monitoring analyte levels in aqueous samples; in such devices, the strains are distinguishable from each other by being arrayed in separate addressable chambers or colonies within the device, and each strain housed in its preselected chamber or colony having the ability to detect one of the different analytes, and/or different concentration ranges of an analyte. Such microfluidic biosensor devices can typically run freely for 30-60 days without intervention. (See, e.g., Hasty et al., "Microbial Microfluidic Biosensor," US11209412B2). In some embodiments, the panel and/or the microfluidic biosensor containing the inventive panel can run for 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30, or up to 60 days, or any number of days defined by a range between any two aforementioned values, without intervention. In some embodiments, the identity of each microbial strain can be distinguished from the other strains in the set of strains by expressing a selectable marker identifiably different from other strains in the set, even if cells of the strain are physically mixed with cells of other strains, e.g., by an identifiable emission wavelength from the electromagnetic spectrum.

A "continuous series" of aqueous samples of process waters or reference waters means a plurality of chronologically sequential liquid aqueous sample doses for detection or monitoring of the presence of a chemical analyte of interest in the aqueous samples via the expression of a pre-selected detectable marker. Optionally, the sample doses are "conditioned" aqueous sample doses. The conditioned aqueous sample doses are generated by serially processing volumes (microliter or larger) of "raw" aqueous sample (e.g., industrial water or environmental water), obtained in chronological succession, through an optional sterile filtration step and/or optional dilution before application to the sensing or detection instrumentation. In a merely illustrative example of an embodiment involving the recombinant *E. coli* strains arrayed in a microfluidic biosensor chip device for housing them, in each raw sample dosing event, the processing of raw aqueous sample (e.g., industrial water or environmental water) involves the following: 25-100 μL of the raw aqueous sample is sterile-filtered, diluted with 300-375 μL of sterile diluent, and mixed in an intermediate vessel maintained at a 1.0-1.5 mL total volume; a continuous stream of "conditioned" liquid aqueous sample is pumped from this intermediate vessel through an active degasser and thence into the microfluidic chip. Flows of the liquid process water and reference water and diluent (e.g., defined culture medium) can be driven by pumping. Useful pumps include, but are not limited to, peristaltic pumps or centrifugal pumps.

The diluent can be, optionally, ultrapure water, a defined growth medium, an acid, a base, or a pH buffer, depending on downstream needs. If an intermediate vessel is employed (e.g., reservoirs with pressurized headspace, as shown in Figure 8) the liquid can be mixed via magnetic stirrer or bubbling of inert gas. As part of the processing of liquids, a camera can be used to continuously image the fill level in the intermediate vessel. When the fill level drops below some threshold, a machine learning algorithm analyzing this image stream can be employed to automatically initiate a dosing event, which triggers the flow of both raw aqueous sample and diluent at the appropriate volumetric mixing ratio. Flow meters placed inline in the raw aqueous sample and diluent streams can be implemented to ensure an accurate mixing ratio in the intermediate vessel. An electronic computerized "controller" or "microcontroller" or "digital control unit," terms used interchangeably herein, can be employed to automatically direct the activity of the pump(s), meter(s) or sensor(s), optional valves, and/or data collection. Within the scope of a method for monitoring a plurality of analytes of interest, the continuous series of aqueous samples can be obtained sequentially from the raw aqueous sample over a time period extending from 1 to 5 minutes, or from 5 minutes up to 10 minutes, or from 10 minutes up to 1-4 hours, or from 4 hours up to 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days, or up to 60 days, or more. The raw aqueous sample can optionally not be "conditioned" before being directed to the apparatus and/or instrumentation for detection or monitoring of the expression of the pre-selected detectable marker(s) expressed by the recombinant microbial biosensor strains housed in the microfluidic device of the invention, if the raw aqueous sample has been pre-processed or does not require pre-processing to be compatible with the device and/or instrumentation.

The term "defined dilution ratio" means that an aqueous sample is mixed with fresh defined culture medium in a predetermined dilution ratio of aqueous sample to concentrated culture medium, e.g., a dilution ratio of 20:1, 15:1, 10:1, 9:1, 8:1, 7:1, 6:1, 5:1, 4:1, 3:1, 2:1, 1:1, or any other dilution ratio suitable for the nature of the detectable marker selected, the format or platform housing the panel of strains, the instrumentation used for monitoring the expression of the detectable marker by the panel, and the suspected concentration of the analyte of interest in the aqueous sample; and the skilled person can readily adjust the defined dilution ratio as needed. In a typical example, one can add one (1) part of 5X concentrated defined culture medium to four (4) parts of aqueous sample, resulting in a (1/5)X dilution of concentrated culture medium and a (4/5)X dilution of aqueous sample.

For producing microbial cells volumes to be used as inoculant to be delivered into the cell trap complexes of the microfluidic chip, cell culturing techniques are employed. The term "inoculation of the cells into the cell culture medium" refers to the step of contacting the cells with the cell culture medium under conditions which are suitable for growth and proliferation of the cells.

The cell culture contemplated herein may be any cell culture independently of the kind and nature of the cultured cells and the growth phase of the cultured cells, e.g. adherent or non-adherent cells; growing, or growth-arrested cells.

The term "sterile," as used herein, refers to a substance that is free, or essentially free, of microbial and/or viral contamination. In this respect the "contaminant" means a material that is different from the desired components in a preparation being a cell culture medium or at least a component of a cell culture medium. In the context of "sterile filtration," the term sterile filtration is a functional description that a preparation is filtered through a sterile filter (with a pore size of 0.2 $\mu$m or less) to remove bacterial and/or mycoplasma contaminants.

Filtration can be used to sterilize an aqueous fluid and/or to remove particulates. "Batch filtration," otherwise known as "batch wise filtration" or filtration done in batch mode, refers herein to a process wherein a specific total amount or volume of a preparation, being a cell culture medium or at least a component of a cell culture medium, is filtered in one batch dependent on the capacity of the filter and wherein the filtration process is finalized before the filtrate is directed or fed to the process in which it is used or consumed. The term "continuous filtration" or "online filtration" or "in line filtration" refers to a filtration process, wherein the specific total amount or volume of a preparation, being a cell culture medium or at least a component of a cell culture medium, is filtered through the sterile filter (e.g., having a microbe-filtering or virus-filtering pore size, as may be desired) continuously dependent on the capacity of the sterile filter and wherein the filtration process is still going on when the filtrate is already directed or fed to the process in which it is used or consumed. For example, a continuous filtration step can be used to filter and sterilize the aqueous sample where it first enters a sensor system, such as, into the inventive microfluidic chip housing a microbial biosensor strain(s). In one embodiment, a tangential flow filter with 500 kDa pore size is used; the continuous relatively fast flow of unfiltered sample tangential to the filter membrane acts to "sweep away" particles in the sample that would otherwise blind the filter membrane.

The "cell culture supernatant" is the extracellular medium in which the cells are cultured. This medium is not to be confused with feed medium that may be added to the culture after inoculation of the cells into the cell culture medium and cell growth has been commenced. A "cell culture" means the cell culture supernatant and the cells cultured therein. Conventionally, *E. coli* cells and/or yeast cells, are cultured at 37°C $\pm$ 1°C or ambient temperature. Mammalian cells are typically cultured at 37°C $\pm$ 1°C.

By "culturing at" or "maintaining at" a temperature, pH, or oxygenation level, we are referring to the temperature, pH, or oxygenation level to which the process control systems are set (in other words, the intended target temperature, pH, or oxygenation level). The culture conditions, such as temperature (typically, but not necessarily, about 20-37°C), pH (typically, but not necessarily, a cell culture medium is maintained within the range of about pH 6.5-7.5, as modified consistent with the present invention), oxygenation, and the like, will be apparent to the ordinarily skilled artisan. Clearly, there will be small variations of the temperature of a culture over time, and from location to location through the culture vessel. Digital control units and sensory monitors are available commercially or can be constructed by the skilled artisan. Alternative digital control units (DCU) that control and monitor the cell culture process are available commercially, made by companies such as B. Braun, New Brunswick, or Sartorius. For in-flask batch culture with shaker, numerous models of suitable cell culture incubators with built-in environmental controls are commercially available, e.g., by Thermo Fisher Scientific. In a typical useful embodiment, a constant culture temperature of 37°C is maintained, using a proportional-integral-derivative controller (PID controller, also known as a "three-term" controller) to control a thermoelectric module with input from a 10K thermistor. Such a system can both heat and cool. Alternatively, a system with a resistive heater can only heat.

The ordinarily skilled artisan is familiar with useful culture conditions, such as temperature, pH (typically, but not necessarily, the cell culture medium is maintained within the range of about pH 6.5-7.5), oxygenation, and the like. "Culturing at" or "maintaining at" a temperature that is set at X±Y°C, means that the set point is at a value of from X+Y°C to X-Y°C. For example, where X is 37.0±0.9°C, the set-point is set at a value of from 37.9 to 36.1°C. For each of the preferred values of X, e.g., X=31, X=32, X=33, X=34, X=35, X=36, or X=37, the set-point is at a value within the range X±0.9°C, ±0.8°C, ±0.7°C, ±0.6°C, ±0.5°C, ±0.4°C, ±0.3°C, ±0.2°C, or ±0.1°C. (See, e.g., Oguchi et al., pH Condition in temperature shift cultivation enhances cell longevity and specific hMab productivity in CHO culture, Cytotechnology. 52(3):199-207 (2006); Al-Fageeh et al., The cold-shock response in cultured mammalian cells: Harnessing the response for the improvement of recombinant protein production, Biotechnol. Bioeng. 93:829-835 (2006); Marchant, R.J. et al., Metabolic rates, growth phase, and mRNA levels influence cell-specific antibody production levels from in vitro cultured mammalian cells at sub-physiological temperatures, Mol. Biotechnol. 39:69-77 (2008)).

For any given set-point, slight variations in temperature may occur. Typically, such variation may occur because heating and cooling elements are only activated after the temperature has deviated somewhat from the set-point. In that case, the set-point is X±Y and the heating or cooling element is activated when the temperature varies by ±Z°C, as appropriate. Typically, the permissible degree of deviation of the temperature from the set-point before heating or cooling elements are activated may be programmed in the process control system. Temperature may be controlled to the nearest ±0.5°C, ±0.4°C, ±0.3°C, ±0.2°C, or even ±0.1°C by heating and cooling elements controlled by thermostats. Larger differentials in temperature may also be programmed, such as ±1.0°C, ±0.9°C, ±0.8°C, ±0.7°C, or ±0.6°C. The temperature may also be controlled by immersion of the culture vessel in a heating bath at a particular temperature.

Conceivably, there is no variation from the set-point because the heating is applied continually. Another source of variation arises due to measurement error in the temperature of the cell culture supernatant. Typical thermometers used in cell culture equipment may have a variability of $\pm0.3°C$, or $\pm0.2°C$, or even $\pm0.1°C$.

Where the temperature set-point is set at a value within the range $X\pm Y°C$, and the tolerance of the temperature is $\pm Z°C$ (i.e. a heater or cooler is activated when the temperature deviates by $\pm Z°C$, as appropriate) this can also be expressed as a set-point of $(X-Y$ to $X+Y)\pm Z°C$. For each possible value of X, all combinations of $\pm Y°C$. and $\pm Z°C$, as indicated above, are envisaged.

"Culturing at" or "maintaining at" a set point of a particular desired pH value, means that the process control systems are set to that desired pH value, in other words that the set point of pH is the intended, target, pH. "Culturing at" or "maintaining at" a pH that is set at $X\pm Y$, means that the set point is at a value of from $X+Y$ to $X-Y$ pH units. For each of the preferred values of X, the set-point is at a value within the range pH $X\pm0.05$, $\pm0.04$, $\pm0.03$, $\pm0.02$ or $\pm0.01$.

Where the pH set-point is set at a value within the range $X\pm Y$, and the tolerance is $\pm Z$, this can also be expressed as a set-point of $(X-Y$ to $X+Y)\pm Z$. For each possible value of X, all combinations of $\pm Y$ and $\pm Z$, as indicated above.

For any given pH set-point, slight variations in pH may occur. Typically, such variation can occur because means which control pH are only activated after the pH has deviated somewhat from the set-point. Typically, the pH is controlled to the nearest $\pm0.05$, $\pm0.04$, $\pm0.03$, $\pm0.02$, or $\pm0.01$. Typically, sparging with $CO_2$ provides additional acid in cell culture. Liquid acids, e.g., HCl or $H_3PO_4$, are commonly used in microbial cultures. Sodium carbonate is usually the source of added alkali used to maintain pH for cell culture, and $NH_4OH$ is often selected to add alkali in microbial culture.

For producing cell culture volumes of microbial cells for delivery to, and housing in, embodiments of the microfluidic chip intended to house colonies of mammalian biosensing cells, the cell culture supernatant typically has a $CO_2$ concentration of 1 to 10% (v/v), for example, 4.0-9.0% (v/v), 5.5-8.5% (v/v), or about 6-8% (v/v). Conventionally, $CO_2$ concentration is higher than this due to the $CO_2$ produced by the cells not being removed from the cell culture supernatant. Maintaining the $CO_2$ concentration at 10% or lower is reported to increase the yield of recombinant protein expression; it helps the dCO2 (or $pCO_2$) to be kept low if the feed medium is degassed (for example by bubbling air through it) as well as the cell culture supernatant in the bioreactor being sparged. (See, Giovagnoli et al., Cell Culture Processes, US2009/0176269, US2016/0244506, US9359629, EP2235197, EP2574676). Degassing of natural/environmental aqueous samples before routing them to a microfluidic device (or "chip") is also important, because outgassing within some sensor systems can generate bubbles that act as high resistance blockages to fluid flow into or within a microfluidic chip, which can divert flows and affect cell growth and sensing. Degassing is especially important when sensing in a background of photosynthetic culture medium, as these can contain levels of dissolved oxygen above 200%.

Ways of monitoring culture parameters of temperature, pH and $CO_2$ concentration are well-known in this art and generally rely on probes that are inserted into the bioreactor, or included in loops through which the culture medium is circulated, or inserted into extracted samples of culture medium. Suitable monitoring equipment and appropriate alternatives are commercially available or can be constructed by the skilled artisan. Alternative gas analyzers are commercially available, such as RapidLab® 248 (Siemens) and others made by Nova® Biomedical, Radiometer America and Roche Diagnostics. Mass flow controllers can also be used to control gas and liquid additions in labs that are properly equipped. A suitable in-line dCO$_2$ (or $pCO_2$) sensor and its use are described in Pattison et al (2000) Biotechnol. Prog. 16:769-774. A suitable in-line pH sensor is Mettler Toledo InPro 3100/125/Pt100 (Mettler-Toledo Ingold, Inc., Bedford, Mass.). A suitable off-line system for measuring dCO$_2$ (or $pCO_2$), in addition to pH and $pO_2$ is the BioProfile pHOx (Nova Biomedical Corporation, Waltham Mass.). In this system, or dCO2 (or $pCO_2$) is measured by potentiometric electrodes within the range 3-200 mmHg with an imprecision resolution of 5%. The pH may be measured in this system at a temperature of 37°C, which is close to the temperature of the cell culture supernatant in the bioreactor. Ways of altering the specified parameter in order to keep it at the predefined level are also well-known. For example, keeping the temperature constant usually involves heating or cooling the bioreactor or the feed medium (if it is a fed-batch or continuous process); keeping the pH constant usually involves choosing and supplying enough of an appropriate buffer (typically bicarbonate) and adding acid, such as hydrochloric acid, or alkali, such as sodium hydroxide, sodium carbonate or a mixture thereof, to the feed medium as necessary; and keeping the $CO_2$ concentration constant usually involves adjusting the sparging rate (see further below), or regulating the flow of $CO_2$ in the head space. It is possible that the calibration of an in-line pH probe may drift over time, such as over periods of days or weeks, during which the cells are cultured. In that event, it may be beneficial to reset the in-line probe by using measurements obtained from a recently calibrated off-line probe. A suitable off-line probe is the BioProfile pHOx (Nova Biomedical Corporation, Waltham Mass.)

For producing cell culture volumes of microbial cells for delivery to, and housing in, embodiments of the microfluidic chip intended to house colonies of mammalian biosensing cells, mammalian cell cultures must be provided with molecular oxygen for the cells to grow. Normally, this can be provided by forcing oxygen into the culture through injection ports. It is also necessary to remove the $CO_2$ that accumulates due to the respiration of the cells. This is achieved by "sparging," i.e., passing a gas through the bioreactor in order to entrain and flush out the $CO_2$. Conventionally, this can also be done using oxygen. However, the inventors have found that it is advantageous to use air instead. It has been found that usually a conventional inert gas such as nitrogen is less effective at sparging $CO_2$ than using air. Given that air is about 20% (v/v) oxygen, one might have thought that five times as much air would be used. However, this has been found to be inadequate in large scale cultures, particularly in cultures at 2500 L scale. In a 2500 L bioreactor, 7 to 10 times as much air, preferably about 9 times as much air, is used. For example, under standard conditions, the 2500 L bioreactor is sparged with $O_2$ at a 10-$\mu$m bubble size at a rate of 0.02 VVH (volume $O_2$ per volume of culture per hour). The same 2500 L bioreactor used according to the method of the invention would be sparged with air at a 10-$\mu$m bubble size at a rate of 0.18 VVH. Hence, the use of surprisingly high volumes of air has been found to provide adequate oxygen supply and to remove the unwanted $CO_2$ in mammalian cell cultures. Flushing the bioreactor head space with air or pure oxygen is also a useful mechanism for removing excess $CO_2$.

The term "buffer" or "buffered solution" refers to solutions which resist changes in pH by the action of its conjugate acid-base range. Examples of useful buffers that control pH at ranges of about pH 4 to about pH 8 include phosphate, bicarbonate, acetate, MES, citrate, Tris, bis-tris, histidine, arginine, succinate, citrate, glutamate, and lactate, or a combination of two or more of these, or other mineral acid or organic acid buffers. Salts containing sodium, ammonium, and potassium cations are often used in making a buffered solution.

A component or a subpart structure of the inventive microfluidic chip, device or system of the invention, is said to be "fluidly connected" to (another chip substructure), or "fluidly receives" or "fluidly conveys" an aqueous liquid, when the component or subpart structure is capable (in the presence of liquid and appropriate pressure (P)) of receiving or conveying such aqueous liquid, in a downstream direction from a more upstream component or subpart structure of the microfluidic chip, device, or system. When aqueous liquid flows within the chip, in a downstream direction, from one step or component or subpart structure via an aperture, closed channel or conduit, junction, or other like microfluidic structure, between the components or subpart structures, and without manual loading or unloading between any of these steps or substructures, it is said to be flowing "fluidly." The entire system of microchannels within the microfluidic chip of the invention "fluidly conveys," or is capable of conveying (in the actual presence of liquid and appropriate pressure (P)), the aqueous liquid from an inlet port, downstream within the microfluidic chip and its various channels and junctions, ultimately to a waste port.

A protein of interest, such as a fluorescent marker or other detectable marker, is used in the practice of the invention, whether a variant or parent protein, is typically produced by recombinant expression technology. The term "recombinant" indicates that the material (e.g., a nucleic acid or a polypeptide) has been artificially or synthetically (i.e., non-naturally) altered by human intervention. The alteration can be performed on the material within, or removed from, its natural environment or state. For example, a "recombinant nucleic acid" is one that is made by recombining nucleic acids, e.g., during cloning, DNA shuffling or other well-known molecular biological procedures. Examples of such molecular biological procedures are found in Maniatis et al., Molecular Cloning. A Laboratory Manual. Cold Spring Harbor Laboratory, Cold Spring Harbor, N.Y. (1982). A "recombinant DNA molecule," is comprised of segments of DNA joined together by means of such molecular biological techniques.

The term "recombinant protein" or "recombinant polypeptide" as used herein refers to a protein molecule, e.g., an antibody, an enzyme, a transcription factor, a detectable marker (e.g., a fluorescent protein or a luminescent protein), which is expressed using a recombinant DNA molecule. A "recombinant host cell" or an "engineered" cell (terms used interchangeably herein) is a cell that contains and/or expresses a recombinant nucleic acid, e.g., a recombinant microbial biosensor cell, such as, but not limited to, an engineered *Escherichia coli* cell.

The term "naturally occurring," where it occurs in the specification in connection with raw aqueous sample or process waters, biological materials such as microbial cells, polypeptides, nucleic acids, and the like, refers to materials which are found in nature. Recombinant cells, recombinant cell lines, and their progeny, are not naturally occurring cells, having been engineered by human manipulation.

The term "control sequence" or "control signal" refers to a polynucleotide sequence that can, in a particular host cell, affect the expression and processing of coding sequences to which it is ligated. The nature of such control sequences may depend upon the host organism. In particular embodiments, control sequences for prokaryotes may include a promoter, a ribosomal binding site, and a transcription termination sequence. Control sequences may include promoters comprising one or a plurality of recognition sites for transcription factors, activator sequences, transcription enhancer (or enhancer-like) sequences or elements, polyadenylation sites, and transcription termination sequences. Control sequences can include leader sequences and/or fusion partner sequences. Promoters and enhancers consist of short arrays of DNA that interact specifically with cellular proteins involved in transcription (Maniatis, et al., Science 236:1237 (1987)). The selection of a particular promoter and enhancer depends on what cell type is to be used to express the protein of interest. Some promoters and enhancers have a broad host range while others are functional in a limited subset of cell types (for review see Voss, et al., Trends Biochem. Sci., 11:287 (1986) and Maniatis, et al., Science 236:1237 (1987)).

A "promoter" is a region of DNA including a site at which RNA polymerase binds to initiate transcription of messenger RNA by one or more downstream structural genes. Promoters are located near the transcription start sites of genes, on the same strand and upstream on the DNA (towards the 5' region of the sense strand). Promoters are typically about 100-1000 base pairs (bp) in length.

The term "modification of expression" from a promoter means either higher measurable expression (i.e.,

activation or induction) or lower measurable expression (i.e., repression), compared to the level of expression in the absence of an analyte of interest. For example, the measurable expression difference can be by at least 1.5-fold, or more, or by at least two-fold, or more, or by at least three-fold, or more, or by at least five-fold, or by at least ten-fold, or more, compared to the level of expression in the absence of an analyte of interest.

The term "modification" when used in connection with proteins of interest, such as activator or repressor proteins, include, but are not limited to, one or more amino acid changes (including substitutions, insertions or deletions); chemical modifications; covalent modification by conjugation to therapeutic or diagnostic agents; labeling (e.g., with radionuclides or various enzymes); covalent polymer attachment such as PEGylation (derivatization with polyethylene glycol) and insertion or substitution by chemical synthesis of non-natural amino acids. By methods known to the skilled artisan, proteins, can be "engineered" or modified for improved target affinity, selectivity, stability, and/or manufacturability before the coding sequence of the "engineered" protein is included in the expression cassette.

"Physiological conditions" are conditions of pH, temperature, nutrients, and the like, that allow the microbial cells, e.g., *E. coli* cells, to express a recombinant expression cassette. "Aerobic" physiological conditions are those above the Pasteur Point for a particular microbial organism (in this case a strain of *E. coli*), i.e., the partial pressure of oxygen (in equilibrium with a solution; "PO$_2$") is above the PO$_2$ at which a facultative aerobic organism switches to anaerobic metabolism, which is typically below a value of approximately 0.01 (1%) of the present atmospheric oxygen level ("PAL"). (See, Stolper et al., "Aerobic growth at nanomolar oxygen concentrations," PNAS 107(44):18755-18760 (2010), pnas.org/cgi/doi/10.1073/pnas.1013435107P). Typically, an oxygen sensor is useful to indicate whether aerobic conditions prevail.

In general, a promoter is capable of operating under "aerobic" physiological conditions, if transcription from the promoter can occur when the redox potential (i.e., oxidation/reduction potential; also known as "ORP" or E$_h$) in the aqueous environment surrounding the cell is E$_h$ > 50-300 mV. The redox potential is measured in millivolts (mV) relative to a standard hydrogen electrode and is commonly measured using a platinum electrode with a saturated calomel electrode as reference. In well-oxidized water, as long as oxygen concentrations stay above about 1 mg O$_2$/L, the redox potential will be highly positive (i.e., above 300-500 mV). In reduced environments, such as in the deep water of stratified lakes or the sediment of eutrophic lakes, the redox potential will be low, even a negative value. (See, e.g., M. Søndergaard, In: "Encyclopedia of Inland Waters," Ed. Gene E. Likens, Academic Press (2009), pp. 852-859; C. Tobias et al., "Coastal Wetlands: An Integrated Ecosystem Approach," Second Edition, Eds. Gerardo M.E. Perillo et al., Elsevier (2019), pp. 539-596). ORP measurements are quick and easy with a ORP probe, but ORP values can give a distorted proxy value of oxygenation in the presence of certain chemicals, e.g., hypochlorite, often found in raw aqueous samples from industrial or water processing plants.

A "domain" or "region" (used interchangeably herein) of a polynucleotide is any portion of the entire polynucleotide, up to and including the complete polynucleotide, but typically comprising less than the complete polynucleotide. A domain can, but need not, fold independently (e.g., DNA hairpin folding) of the rest of the polynucleotide chain and/or be correlated with a particular biological, biochemical, or structural function or location, such as a coding region or a regulatory region.

A "domain" or "region" (used interchangeably herein) of a protein is any portion of the entire protein, up to and including the complete protein, but typically comprising less than the complete protein. A domain can, but need not, fold independently of the rest of the protein chain and/or be correlated with a particular biological, biochemical, or structural function or location (e.g., a ligand binding domain, or a cytosolic, transmembrane or extracellular domain).

The term "identity" refers to a relationship between the sequences of two or more polypeptide molecules or two or more nucleic acid molecules, as determined by aligning and comparing the sequences. "Percent identity" means the percent of identical residues between the amino acids or nucleotides in the compared molecules and is calculated based on the size of the smallest of the molecules being compared. For these calculations, gaps in alignments (if any) must be addressed by a particular mathematical model or computer program (i.e., an "algorithm"). Methods that can be used to calculate the identity of the aligned nucleic acids or polypeptides include those described in Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje, G., 1987, Sequence Analysis in Molecular Biology, New York: Academic Press; Sequence Analysis Primer, (Gribskov, M. and Devereux, J., eds.), 1991, New York: M. Stockton Press; and Carillo et al., 1988, SIAM J. Applied Math. 48:1073. For example, sequence identity can be determined by standard methods that are commonly used to compare the similarity in position of the amino acids of two polypeptides. Using a computer program such as BLAST or FASTA, two polypeptide or two polynucleotide sequences are aligned for optimal matching of their respective residues (either along the full length of one or both sequences, or along a pre-determined portion of one or both sequences). The programs provide a default opening penalty and a default gap penalty, and a scoring matrix such as PAM 250 (a standard scoring matrix; see Dayhoff et al., in Atlas of Protein Sequence and Structure, vol. 5, supp. 3 (1978)) can be used in conjunction with the computer program. For example, the percent identity can then be calculated as: the total number of identical matches multiplied by 100 and then divided by the sum of the length of the longer sequence within the matched span and the number of gaps introduced into the longer sequences in order to align the two sequences. In calculating percent identity, the sequences being compared are aligned in a way that gives the largest match between the sequences.

The GCG program package is a computer program that can be used to determine percent identity, which package includes GAP (Devereux et al., 1984, Nucl. Acid Res. 12:387; Genetics Computer Group, University of Wisconsin, Madison, Wis.). The computer algorithm GAP is used to align the two polypeptides or two polynucleotides for which the percent sequence identity is to be determined. The sequences are aligned for optimal matching of their respective amino acid or nucleotide (the "matched span", as determined by the algorithm). A gap opening penalty (which is calculated as 3.times. the average diagonal, wherein the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm. In certain embodiments, a standard comparison matrix (see, Dayhoff et al., 1978, Atlas of Protein Sequence and Structure 5:345-352 for the PAM 250 comparison matrix; Henikoff et al., 1992, Proc. Natl. Acad. Sci. U.S.A. 89:10915-10919 for the BLOSUM 62 comparison matrix) is also used by the algorithm.

Recommended parameters for determining percent identity for polypeptides or nucleotide sequences using the GAP program include the following:

Algorithm: Needleman et al., 1970, J. Mol. Biol. 48:443-453;
Comparison matrix: BLOSUM 62 from Henikoff et al., 1992, supra;
Gap Penalty: 12 (but with no penalty for end gaps)
Gap Length Penalty: 4
Threshold of Similarity: 0
Certain alignment schemes for aligning two amino acid sequences may result in matching of only a short region of the two sequences, and this small aligned region may have very high sequence identity even though there is no significant relationship between the two full-length sequences. Accordingly, the selected alignment method (GAP program) can be adjusted if so desired to result in an alignment that spans at least 50 contiguous amino acids of the target polypeptide.

An "enhancer" is a short (50-1500 bp) region of DNA that can be bound with one or more activator proteins (transcription factors) to activate transcription of a gene.

The terms "in operable combination", "in operable order" and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced. For example, a control sequence in a vector that is "operably linked" to a protein coding sequence is ligated thereto so that expression of the protein coding sequence is achieved under conditions compatible with the transcriptional activity of the control sequences.

"Polypeptide" and "protein" are used interchangeably herein and include a molecular chain of two or more amino acids linked covalently through peptide bonds. The terms do not refer to a specific length of the product. Thus, "peptides," and "oligopeptides," are included within the definition of polypeptide. The terms include post-translational modifications of the polypeptide, for example, glycosylations, acetylations, phosphorylations and the like. In addition, protein fragments, analogs, mutated or variant proteins, fusion proteins and the like are included within the meaning of polypeptide. The terms also include molecules in which one or more amino acid analogs or non-canonical or unnatural amino acids are included as can be expressed recombinantly using known protein engineering techniques. In addition, proteins can be derivatized as described herein and by other well-known organic chemistry techniques.

A "variant" of a polypeptide comprises an amino acid sequence wherein one or more amino acid residues are inserted into, deleted from and/or substituted into the amino acid sequence relative to another polypeptide sequence. Variants can include fusion proteins.

As used herein "soluble" when in reference to a protein produced by recombinant DNA technology in a host cell is a protein that exists in aqueous solution; if the protein contains a twin-arginine signal amino acid sequence the soluble protein is exported to the periplasmic space in gram negative bacterial hosts or by bacterial host possessing the appropriate genes (e.g., the *kil* gene). Thus, a soluble protein is a protein which is not found in an inclusion body inside the host cell. Alternatively, depending on the context, a soluble protein is a protein which is not found integrated in cellular membranes, or, *in vitro,* is dissolved, or is capable of being dissolved in an aqueous buffer under physiological conditions without forming significant amounts of insoluble aggregates (i.e., forms aggregates less than 10%, and typically less than about 5%, of total protein) when it is suspended without other proteins in an aqueous buffer of interest under physiological conditions, such buffer not containing an ionic detergent or chaotropic agent, such as sodium dodecyl sulfate (SDS), urea, guanidinium hydrochloride, or lithium perchlorate. In contrast, an insoluble protein is one which exists in denatured form inside cytoplasmic granules (called an inclusion body) in the host cell, or again depending on the context, an insoluble protein is one which is present in cell membranes, or in an *in vitro* aqueous buffer under physiological conditions forms significant amounts of insoluble aggregates (i.e., forms aggregates equal to or more than about 10% of total protein) when it is suspended without other proteins (at physiologically compatible temperature) in an aqueous buffer of interest under physiological conditions, such buffer not containing an ionic detergent or chaotropic agent, such as sodium dodecyl sulfate (SDS), urea, guanidinium hydrochloride, or lithium perchlorate.

The term "polynucleotide" or "nucleic acid" includes both single-stranded and double-stranded nucleotide polymers containing two or more nucleotide residues. The nucleotide residues comprising the polynucleotide can be ribonucleotides or deoxyribonucleotides or a modified form of either type of nucleotide. Said modifications include base modifications such as bromouridine and inosine derivatives, ribose modifications such as 2',3'-dideoxyribose, and internucleotide linkage modifications such as phosphorothioate, phosphorodithioate, phosphoroselenoate, phosphorodiselenoate, phosphoroanilothioate, phosphoraniladate and phosphoroamidate.

The term "oligonucleotide" means a polynucleotide comprising 200 or fewer nucleotide residues. In some embodiments, oligonucleotides are 10 to 60 bases in length. In other embodiments, oligonucleotides are 12, 13, 14, 15, 16, 17, 18, 19, or 20 to 40 nucleotides in length. Oligonucleotides may be single stranded or double stranded, e.g., for use in the construction of a mutant gene. Oligonucleotides may be sense or antisense oligonucleotides. An oligonucleotide can include a label, including a radiolabel, a fluorescent label, a hapten or an antigenic label, for detection assays. Oligonucleotides may be used, for example, as PCR primers, cloning primers or hybridization probes.

A "polynucleotide sequence" or "nucleotide sequence" or "nucleic acid sequence," as used interchangeably herein, is the primary sequence of nucleotide residues in a polynucleotide, including of an oligonucleotide, a DNA, and RNA, a nucleic acid, or a character string representing the primary sequence of nucleotide residues, depending on context. From any specified polynucleotide sequence, either the given nucleic acid or the complementary polynucleotide sequence can be determined. Included are DNA or RNA of genomic or synthetic origin which may be single- or double-stranded, and represent the sense or antisense strand. Unless specified otherwise, the left-hand end of any single-stranded polynucleotide sequence discussed herein is the 5' end; the left-hand direction of double-stranded polynucleotide sequences is referred to as the 5' direction. The direction of 5' to 3' addition of nascent RNA transcripts is referred to as the transcription direction; sequence regions on the DNA strand having the same sequence as the RNA transcript that are 5' to the 5' end of the RNA transcript are referred to as "upstream sequences;" sequence regions on the DNA strand having the same sequence as the RNA transcript that are 3' to the 3' end of the RNA transcript are referred to as "downstream sequences."

As used herein, an "isolated nucleic acid molecule" or "isolated nucleic acid sequence" is a nucleic acid molecule that is either (1) identified and separated from at least one contaminant nucleic acid molecule with which it is ordinarily associated in the natural source of the nucleic acid or (2) cloned, amplified, tagged, or otherwise distinguished from background nucleic acids such that the sequence of the nucleic acid of interest can be determined. An isolated nucleic acid molecule is other than in the form or setting in which it is found in nature. However, an isolated nucleic acid molecule includes a nucleic acid molecule contained in cells that ordinarily express the immunoglobulin (e.g., antibody) where, for example, the nucleic acid molecule is in a chromosomal location different from that of natural cells.

As used herein, the terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of ribonucleotides along the mRNA chain, and also determines the order of amino acids along the polypeptide (protein) chain. The DNA sequence thus codes for the RNA sequence and for the amino acid sequence.

The term "gene" is used broadly to refer to any nucleic acid associated with a biological function. Genes typically include coding sequences and/or the regulatory sequences required for expression of such coding sequences. The term "gene" applies to a specific genomic or recombinant sequence, as well as to a cDNA or mRNA encoded by that sequence. Genes also include non-expressed nucleic acid segments that, for example, form recognition sequences for other proteins. Non-expressed regulatory sequences including transcriptional control elements to which regulatory proteins, such as transcription factors, bind, resulting in transcription of adjacent or nearby sequences.

"Expression of a gene" or "expression of a nucleic acid" means transcription of DNA into RNA (optionally including modification of the RNA, e.g., splicing), translation of RNA into a polypeptide (possibly including subsequent post-translational modification of the polypeptide), or both transcription and translation, as indicated by the context.

An "expression cassette" refers to a nucleic acid construct, which when introduced into a host cell, results in transcription and/or translation of a RNA or polypeptide, respectively. The expression cassette includes a gene encoding a protein of interest. It includes a promoter, operable in an *E. coli* cell, for mRNA transcription, one or more gene(s) encoding protein(s) of interest and a mRNA termination. An expression cassette can usefully include among the coding sequences, a gene useful as a selective marker and/or a detectable marker or reporter. In the expression cassette promoter is operably linked 5' to an open reading frame encoding an exogenous protein of interest; and a polyadenylation site is operably linked 3' to the open reading frame. Other suitable control sequences can also be included as long as the expression cassette remains operable. The open reading frame can optionally include a coding sequence for more than one protein of interest.

In some embodiments, a "detectable marker" expressed by a microbial biosensor strain is a protein, e.g., a fluorescent protein. In some embodiments, the fluorescent protein is selected from the group consisting of green fluorescent protein, a yellow fluorescent protein, a cyan fluorescent protein, a red-shifted green fluorescent protein (rs-GFP), and miniSOG. In some embodiments, the detectable protein is a luminescent protein. In some embodiments, the luminescent protein is bacterial luciferase (Lux).

As used herein the term "coding region" or "coding sequence" when used in reference to a structural gene refers to the nucleotide sequences which encode the amino acids found in the nascent polypeptide as a result of translation of an mRNA molecule. The coding region is bounded, in eukaryotes, on the 5' side by the nucleotide triplet "ATG" which encodes the initiator methionine and on the 3' side by one of the three triplets which specify stop codons (i.e., TAA, TAG, TGA).

Recombinant expression technology typically involves the use of a recombinant expression vector comprising an expression cassette and a host cell comprising the recombinant expression vector with the expression cassette or at least the expression cassette, which may for example, be integrated into the host cell genome.

The term "vector" means any molecule or entity (e.g., nucleic acid, plasmid, bacteriophage or virus) used to transfer protein coding information into a host cell.

The term "expression vector" or "expression construct" as used herein refers to a recombinant DNA molecule containing a desired coding sequence and appropriate nucleic acid control sequences necessary for the expression of the operably linked coding sequence in a particular host cell. An expression vector can include, but is not limited to, sequences that affect or control transcription and translation of a coding region operably linked thereto. Nucleic acid sequences necessary for expression in prokaryotes include a promoter, optionally an operator sequence, a ribosome binding site and possibly other sequences. A secretory signal peptide sequence can also, optionally, be encoded by the expression vector, operably linked to the coding sequence of interest, so that the expressed polypeptide can be secreted by the recombinant host cell, for more facile isolation of the polypeptide of interest from the cell, if desired. Such techniques are well-known in the art. (See, e.g., Goodey, Andrew R.; et al., Peptide and DNA sequences, U.S. Pat. No. 5,302,697; Weiner et al., Compositions and methods for protein secretion, U.S. Pat. No. 6,022,952 and U.S. Pat. No. 6,335,178; Han et al., "Novel signal peptides improve the secretion of recombinant Staphylococcus aureus Alpha toxinH35L in Escherichia coli," AMB Express (2017) 7:93 Published online 2017 May 12, doi:10.1186/s13568-017-0394-1). For expression of multi-subunit proteins of interest, separate expression vectors in suitable numbers and proportions, each containing a coding sequence for each of the different subunit monomers, can be used to transform a host cell. In other embodiments, a single expression vector can be used to express the different subunits of the protein of interest.

The term "host cell" or "engineered cell" means a cell that has been transformed, or is capable of being transformed, with a nucleic acid and thereby expresses a gene or coding sequence of interest. The term includes the progeny of the parent cell, whether or not the progeny is identical in morphology or in genetic make-up to the original parent cell, so long as the gene of interest is present. The selection of a particular host is dependent upon a number of factors recognized by the art. These include, for example, compatibility with the chosen expression vector, toxicity of the peptides encoded by the DNA molecule, rate of transformation, ease of recovery of the peptides, expression characteristics, bio-safety and costs. A balance of these factors must be struck with the understanding that not all hosts may be equally effective for the expression of a particular DNA sequence. Modifications can be made at the DNA level, as well. The peptide-encoding DNA sequence may be changed to codons more compatible with the chosen host cell. Codons can be substituted to eliminate restriction sites or to include silent restriction sites, which may aid in processing of the DNA in the selected host cell. Next, the transformed host is cultured and purified. Host cells may be cultured under conventional fermentation conditions so that the desired compounds are expressed. Such fermentation conditions are well-known in the art.

The term "transfection" means the uptake of foreign or exogenous DNA by a eukaryotic cell, and a cell has been "transfected" when the exogenous DNA has been introduced inside the eukaryotic cell membrane. A number of transfection techniques are well-known in the art and are disclosed herein. See, e.g., Graham et al., 1973, Virology 52:456; Sambrook et al., 2001, Molecular Cloning: A Laboratory Manual, supra; Davis et al., 1986, Basic Methods in Molecular Biology, Elsevier; Chu et al., 1981, Gene 13:197. Such techniques can be used to introduce one or more exogenous DNA moieties into suitable host cells.

The term "transformation" refers to a change in a prokaryotic cell's genetic characteristics, and a cell has been transformed when it has been modified to contain new DNA or RNA. For example, a cell is transformed where it is genetically modified from its native state by introducing new genetic material via transfection, transduction, or other techniques. Following transfection or transduction, the transforming DNA may recombine with that of the cell by physically integrating into a chromosome of the cell, or may be maintained transiently as an episomal element without being replicated, or may replicate independently as a plasmid. A cell is considered to have been "stably transformed" when the transforming DNA is replicated with the division of the cell.

Microbial host cells can be usefully grown in batch culture, fed-batch culture, intensified fed-batch culture (product retention perfusion), or in continuous culture systems employing liquid aqueous medium. Host cells are generally cultured as suspension cultures. That is to say, the cells are suspended in a liquid cell culture medium, rather than adhering to a solid support. In other embodiments, the host cells can be cultured on solid or semi-solid aqueous culture medium, for example, containing agar or agarose, to form a medium, carrier (or microcarrier) or substrate surface to which the cells adhere and form an adhesion layer. Another useful mode of production is a hollow fiber bioreactor with an adherent cell line. Porous microcarriers can be suitable and are available commercially.

"Cell culture medium" or "culture medium" or "growth medium" (used interchangeably herein, whether in singular or plural), is defined, for purposes of the invention, as a sterile medium suitable for growth of cells, in *in vitro* cell culture. Any medium capable of supporting growth of the appropriate cells in cell culture can be used. Suitably, the culture medium has an osmolality of between 210 and 650 mOsm, preferably 270 to 450 mOsm, more preferably 300 to 350 mOsm. (See, e.g., Cayley S et al., "Large changes in cytoplasmic biopolymer concentration with osmolality indicate that macromolecular crowding may regulate protein-DNA interactions and growth rate in osmotically stressed Escherichia coli K-12," J. Mol. Recognit. 17(5):488-96 (2004)). Preferably, the osmolality of the cell culture supernatant is maintained within one or more of these ranges throughout the culturing of host cells. The cell culture medium can be based on any basal medium, such as Luria-Bertani (LB) broth, M9 minimal medium, HM9 minimal medium, generally known to the skilled worker, and/or media further described herein. Commercially available media are suitable for culturing various host cells, or can be modified appropriately to suit the cell line employed. The basal medium can comprise a number of ingredients, including amino acids, vitamins, organic and inorganic salts, and sources of carbohydrate, each ingredient being present in an amount which supports the cultivation of a cell which is generally known to the person skilled in the art. The medium can contain auxiliary substances, such as buffer substances like sodium bicarbonate, antioxidants, stabilizers to counteract mechanical stress, or protease inhibitors. Any of these media may be supplemented, as necessary, with physiologically acceptable salts, such as sodium chloride, calcium, magnesium, and phosphate salts, including salts of amino acids, such as, but not limited to, a lysine, histidine, or proline salt; with buffers, such as HEPES and/or sodium bicarbonate; nucleotides, such as adenosine and thymidine; antibiotics, such as gentamicin, neomycin, tetracycline, puromycin, or kanamycin; trace elements (defined as inorganic compounds usually present at final concentrations in the micromolar range); and glucose or an equivalent carbon and/or energy source, such that the physiological conditions of the cell in, or on, the medium promote expression of the protein of interest by the host cell; any other necessary supplements may also be included at appropriate concentrations that would be known to those skilled in the art.

Detailed Embodiments of the Microfluidic Chip of the Invention

Figure 1 shows a microfluidic device (or "chip"; 100) design with four pairs of microbial process monitoring and microbial reference monitoring arrays, where each process monitoring array (110) can receive a unique process water from an exterior source, and each reference monitoring array (120) can receive a unique reference water that matches the background chemical composition of the paired process water. In an embodiment of the inventive chip design, shown in Figure 1, defined growth medium, required to support culturing of the engineered biosensing cells in the cell trap complexes (130, 230), must be pre-mixed into each process water and reference water prior to flowing into the chip's inlet ports (150, 160). The growth medium can be unique for each pair of process monitoring array (110) and reference monitoring array (120) to allow the culturing of a unique strain or species of microbial sensing cell in each array pair.

In the expanded view of an exemplary monitoring array (110, 120), as shown in Figure 2, the array is structured the same whether it is a process monitoring array (110) or a reference monitoring array (120), and in the course of operation of the chip, the process monitoring arrays and reference monitoring arrays are optionally functionally alternated at preselected time intervals, as detailed in Example 2 and Example 4, hereinbelow. The process monitoring arrays (110) or reference monitoring arrays (120) are structurally the same across all chip embodiments, although the number of cell trap complexes (130, 230) in an array can be varied as desired, with the upstream manifold channels (200) fluidly conveying to them, or the downstream manifold channels (220) fluidly conveying from these cell trap complexes, being increased or decreased accordingly, as needed (see, e.g., Figure 4A-B and Figure 5A-B). However, each cell trap complex (130, 230) will be fluidly connected to only a single branch channel (210, 410, 510).

In the exemplary array shown in Figure 2, reference water or process water, as the case may be, is mixed with cell culture medium. The aqueous mixture then flows sequentially downstream from the inlet ports (150, 160), into resistance-tuned inlet channel(s) (155, 165), thence through upstream array inlet channel(s) (132), and thence into the cell trap manifold through the upstream manifold channel(s) (200), which directs the flow across a manifold of branch channels (210), into which the water/culture medium mixture is supplied to the actively growing cells in the cell trap complexes (130, 230). Conditioned growth medium, containing cellular debris from the cell trap complexes, is released downstream from the cell trap complexes (130, 230) to flow into the downstream manifold channel(s) (220), which convey the liquid flow into a downstream array outlet channel (135, 235), whence the mixture of water, medium, and cell debris is carried through the waste outlet channel (138) to the downstream waste port (140) for exhaustion from the chip.

The monitoring array (110, 120) as a whole is designed such that all of its cell trap complexes (130, 230) receive the fresh water and culture medium mixture at the same flow rate, and also such that waste, i.e., conditioned medium and cell debris from each of the cell trap complexes, flows directly to the downstream manifold channel(s) (220) and waste outlet channels (138), without passing over, and potentially contaminating, neighboring cell trap complexes. This enables unique strains to be spotted in each cell spotting region (300, 600) without the risk of cross-contamination.

Figure 3A-C depicts a more detailed view of the structure and function of the regions contained within the cell trap complexes (130, 230, 430, 530, 630) including the cell spotting regions (300, 600), primary cell traps (312, 612), and downstream cell traps (340, 640).

Figure 3A shows the spotting region (300) in which cells are deposited before revival on growth media; the spotting region is fluidly connected to a first migration channel (320, 620) having a broader end (322) and a narrower end (324), which is fluidly connected to a primary cell trap (312, 612) and to a second migration channel (332, 632), which is fluidly connected to a plurality of downstream cell traps (340, 640). Within the spotting regions (300), support pillars (314, 614) are present to prevent the collapse of the chip solid matrix (796) in some embodiments of the inventive chip, in what is a relatively wide and shallow region of the chip.

Figure 3B illustrates microbial growth in the cell traps (340, 640) where the deposited cells directly interface with nutrient cell culture medium flowing through the branch channel (310). Figure 3B(i)-(iv) shows in sequential details how (i) cells deposited in the spotting region (300, 600) receive nutrients from a branch channel (210, 310, 410, 510, 610); (ii) the deposited cells begin their growth outwards towards the primary cell trap (312); and (iii) via the second migration channel (332), the cells continue to grow into the downstream cell traps (340), (iv) eventually filling the entire cell trap spaces up to the interface with the branch channel (310). As illustrated in Figure 3B(iv), the cells continue to grow into the branch channel (310), and the flow in the branch channel (310) washes cell debris away, maintaining the culture in the cell trap complexes (130, 230, 430, 530, 630) at a constant volume.

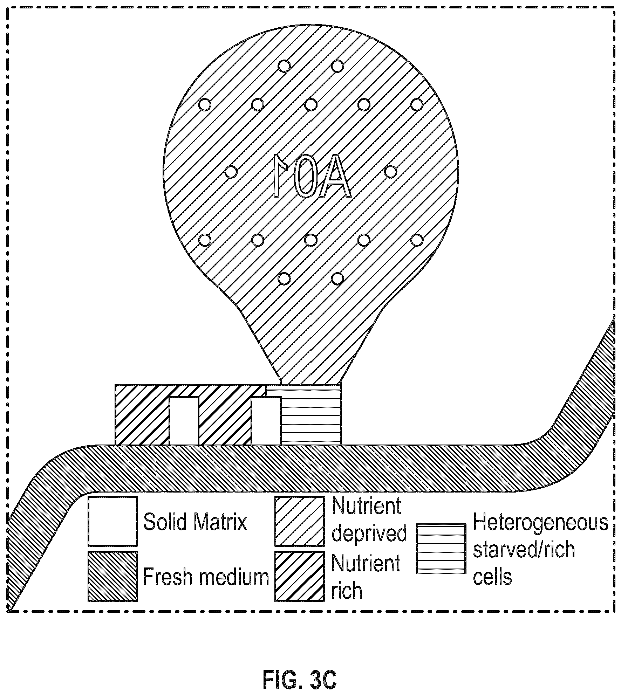

Figure 3C highlights the general nutrient availability to cells in the primary cell traps (312, 612) and downstream cell traps (340, 640) and in the spotting region (300, 600), once the cells have grown to fill the region (as shown in Figure 3B(iv)). Fresh medium from the branch channel (310) diffuses towards the back of the spotting region and is consumed by the growing bacteria. Cells closest to the branch channel are in nutrient rich conditions and have a faster growth rate, while the cells farthest from branch channel (310) are the most nutrient deprived and grow slower. This relative slow growth eventually pushes nutrient-deprived cells into the primary cell trap (312), causing the growth phenotype to be a heterogeneous distribution of healthy cells and cells in a more starved state. Meanwhile, the phenotype of cells in the downstream cell traps (340) tend to be more homogenous, only containing a healthy cell population because of the separation of the downstream cell traps from the spotting region (300), with only the narrow second migration channel (332, 632) connecting these chambers.

While cells growing in the downstream cell traps (340, 640) are often those considered the most experimentally important and relevant, all regions of this cell trap complex are measured and can provide useful biological information. For example, some transcriptional promoters can be more active in cellular growth states resembling either exponential or stationary growth, both of which are provided in cell trap regions.

In some embodiments, the growth medium required to support culturing of the engineered sensing cells in the cell trap complexes (230) is mixed with water on-chip, in which defined medium flows into a designated media inlet port and is mixed with a pair of process and reference waters on-chip. Media inlet flow from the port splits at a media junction and flows toward each half of a process and reference monitoring array pair. Media flow then merges with each process and reference water at a mixing junction, mixes during flow through a series of staggered herringbone mixer (SHM) channel features, and feeds the upstream array inlet channel for each monitoring array. The SHM channel structures comprise staggered herringbone features (e.g., 90-150 micrometers in height in embodiments designed for use in housing prokaryotic cells) positioned along a microfluidic channel (e.g., 40-90 micrometers in height in embodiments designed for use in housing prokaryotic cells) that serve to "fold over" the parallel laminar flows of water and media downstream of the mixing junction, thereby increasing the surface area of the flow boundary and thus the diffusion rate across it. To sense one or more analytes in a single process water using unique strains or species of microbial sensing cells with different growth medium requirements, multiple growth media may be mixed with a single pair of process and reference waters on-chip.

For example, Figure 4A shows a chip design with three pairs of microbial process monitoring arrays (110) and microbial reference monitoring arrays (120). Each microbial process monitoring array receives the same process water from an exterior source via an inlet port (415, 515), and also, via a separate inlet port, each microbial reference monitoring array receives the same reference water that matches the background chemical composition of the paired process water. Process water or reference water enters at an inlet port (415, 515) and is fluidly conveyed by the chip through the primary water inlet channel(s) (416, 516), to a flow-splitting water junction (400, 500), from which the chip fluidly conveys the liquid to each post-water junction water channel (420, 520), which is upstream of a mixing junction (450, 550). Process or reference water is merged by the chip with defined media at the mixing junction(s) (450, 550), and mixes in the mixing channel(s) (460, 560) containing the SHMs before flowing via an upstream array inlet channel (132, 432, 532) into the microbial monitoring arrays (110, 120), with their component channels and cell trap complexes (130, 230, 430, 530, 630), as previously described hereinabove. The defined medium initially enters the chip at media inlet port(s) (417, 517), and is thence fluidly conveyed by the chip's primary media inlet channel(s) (455, 555, 655) to the mixing junction(s) (450, 550), where the process water or reference water is encountered. Each pair of microbial process and reference monitoring arrays fluidly conveys liquid away from the monitoring arrays via downstream array outlet channel(s) (135, 235, 435, 535) to a dedicated waste outlet channel (138, 438, 538) and thence to a downstream waste port (140, 470, 570), where liquid flow finally exits the chip. Each pair of microbial process and microbial reference monitoring arrays feed a dedicated waste outlet channel (438) and downstream waste port (470). Summarily, this chip embodiment enables the on-chip mixing of three media sources and two water sources across six total monitoring arrays, each with a unique combination of water and media.

Figure 4B shows an expanded view of a monitoring array (110, 120) and mixing channel (460) upstream to the monitoring array. In this embodiment, the cell trap complexes (430) remain unchanged from the cell trap complexes shown in Figure 3, and the fundamental structure of the monitoring array shown in Figure 2, albeit with additional upstream manifold channels (402, 502, 602) supplying media to the branch channels (210, 310, 410, 510, 610) which fluidly conveys liquid to the downstream manifold channels (220, 403, 503, 603), and thence to a downstream array outlet channel (435), as shown. The primary difference in this embodiment, as described above, is the on-chip mixing after the flows of water and concentrated media, respectively, meet at the mixing junction (450) and are mixed as the fluid travels through the mixing channel containing the SHMs.

Figure 4C shows an expanded view of one of the media junctions (440, 540) at which each of the three media sources is split into two post-media junction media channels (425, 525, 625), one of which channels fluidly conveys liquid to a microbial process monitoring array (110) and the other fluidly conveying liquid to a microbial reference monitoring array (120). A light emission calibration channel (427, 527, 627) is placed along the path of a post-media junction media channel (425, 525, 625) leading to each monitoring array.

Figure 4D shows an expanded view of the structure fluidly conveying one of the two water sources (i.e., process water or reference water) from an inlet port (415, 515) to a primary water inlet channel (416, 516); at a water junction (400, 500) the flow is split to flow into three post-water junction water channels (420, 520), which fluidly convey liquid either to the three microbial process monitoring arrays (110) or the three microbial reference monitoring arrays (120).

As another example of on-chip media and water mixing, Figure 5A shows another embodiment of the chip design with two pairs of microbial process monitoring arrays (110) and microbial reference monitoring arrays (120). Each microbial process monitoring array receives the same process water from an exterior source via an inlet port (415, 515), and also, via a separate inlet port, each microbial reference monitoring array receives the same reference water that matches the background chemical composition of the paired process water. Process water or reference water enters at an inlet port (415, 515), and is fluidly conveyed by the chip through the primary water inlet channel(s) (416, 516), to a flow-splitting water junction (400, 500), from which the chip fluidly conveys the liquid to each post-water junction water channel (420, 520), which is upstream of a mixing junction (450, 550). Process or reference water is merged by the chip with defined media at the mixing junction(s) (450, 550), and these flows mix in the mixing channel(s) (460, 560) containing the SHMs before flowing via an upstream array inlet channel (132, 432, 532) into the microbial monitoring arrays (110, 120), with their component channels and cell trap complexes (130, 230, 430, 530, 630), as previously described hereinabove. The defined culture medium initially enters the chip at media inlet port(s) (417, 517), and the medium is thence fluidly conveyed by the chip's primary media inlet channel(s) (455, 555, 655) to the mixing junction(s) (450, 550), where the process water or reference water is encountered. Each pair of process monitoring and reference monitoring arrays fluidly conveys liquid away from the monitoring arrays via downstream array outlet channel(s) (135, 235, 435, 535) to a dedicated waste outlet channel (138, 438, 538) and thence to a downstream waste port (140, 470, 570), where liquid flow finally exits the chip.

As a further example of on-chip media and water mixing, Figure 5B shows an embodiment of the inventive chip design with one paired microbial process monitoring array (110) and microbial reference monitoring array (120), where the microbial process monitoring array receives process water from an exterior source via an inlet port (415, 515), and the microbial reference monitoring array receives reference water that matches the background chemical composition of the paired process water, also via a separate inlet port (415, 515). The process water or reference water is thence fluidly conveyed by the chip through the primary water inlet channel(s) (416, 516), to a mixing junction (450, 550). Process or reference water is merged by the chip with defined media at the two mixing junction(s) (450, 550), and mixes in the mixing channel(s) (460, 560) containing the SHMs before flowing via an upstream array inlet channel (132, 432, 532) into the microbial monitoring arrays (110, 120), with their component channels and cell trap complexes (130, 230, 430, 530, 630), as previously described hereinabove. Process water or reference water merges with the same defined culture medium at the mixing junction(s) (450, 550), and mixes with the medium in the SHMs of the mixing channel(s) (460, 560) before being fluidly conveyed to the microbial process monitoring array or the microbial reference monitoring array, respectively. **In** this embodiment, the defined medium initially enters the chip at a single media inlet port (517), which also serves as the media junction (540), and the medium is thence fluidly conveyed by the chip's primary media inlet channel(s) (455, 555, 655) to the mixing junction(s) (450, 550), where the process water or reference water is encountered. The paired microbial process monitoring array and microbial reference monitoring array fluidly convey liquid away from the monitoring arrays, via downstream array outlet channels (135, 235, 435, 535), to a dedicated waste outlet channel (138, 438, 538) and thence to a downstream waste port (140, 470, 570), where liquid flow finally exits the chip.

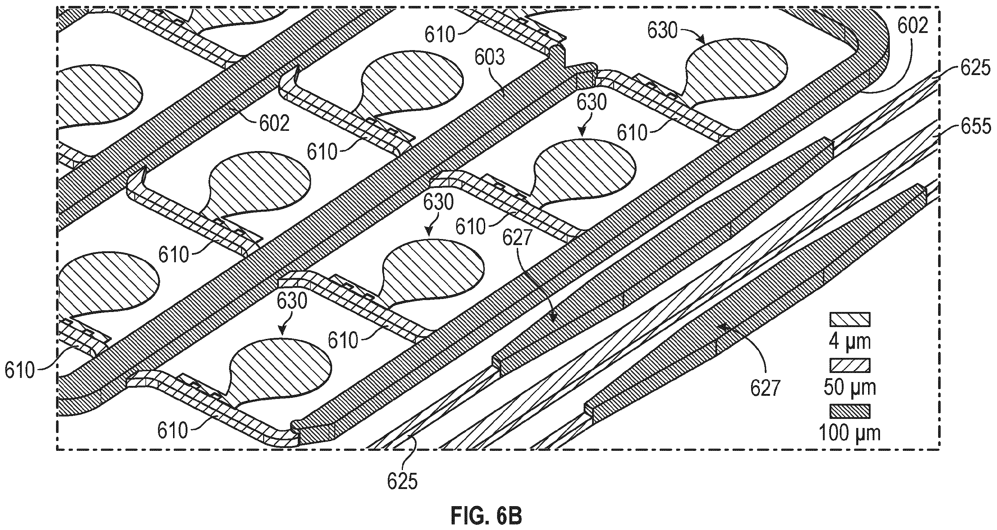

Figure 6A shows an additional feature of these embodiments, also illustrated in Figure 4A and Figure 5A-B: a light emission calibration channel(s) (427, 527, 627). The light emission calibration channel contains medium prior to its on-chip mixing with the process water or reference water, and is at a height to match the spatially neighboring upstream manifold channel (402, 502, 602), 100 $\mu$m in the particular embodiment shown in Figure 6A. Because the fluorescence signal from a channel is proportional to the height of the channel and the intensity of fluorescence of the media flowing through it, the relative fluorescence of the pre-mix medium in the calibration channel to the post-mix medium in the upstream manifold channel can be compared, to ensure that mixing is occurring at the correct and intended ratio. For example, if the defined medium is expected to be diluted by water by a factor of five (i.e., 5X) during on-chip mixing, the measured fluorescence in the light emission calibration channel (427, 527, 627) should be 5 times greater than the fluorescence in the manifold channel. Media fluorescence can be a natural physical property of some growth media, or fluorescence in the medium can be achieved by the addition of fluorescent dyes. Figure 6B shows an isometric view of this same section of the chip to further illustrate the relative channel heights.

Figure 7 shows data matrices (195, 495, 595, 795), an additional chip feature that is useful for assessing the chip orientation during imaging by an external detector (180) and data processing. During image analysis, the location of these data matrices can be used to determine the orientation, rotation, and any potential warping of the physical device. While the data matrix (195, 495, 595, 795) contains information within its arrangement of squares, a human-readable data matrix label (797) can also be included within this information for convenience. Finally, to achieve the contrast necessary to distinguish each square of the data matrix (195, 495, 595, 795) from the solid matrix (796) of the chip (100), a hatched textured transparent zone (799) is used on the photolithography mask (798), instead of making the full chip solid matrix clear, as is done with other device features.

Engineering and Culturing of Recombinant Microbial Biosensor Strains

The microfluidic device of the invention is designed to house functional microbial biosensor strains, which are typically engineered by recombinant expression techniques. (See, e.g., Cookson et al., "Engineered Biosensor Strains of E. Coli for Continuous Aerobic Detection of Analytes," PCT/US2024/025712; Hasty et al., "Microbial Microfluidic Biosensor," US2018149633A1). Briefly, the pooled plasmid constructs (expression vectors) from the cloning step can be stably transformed or transfected into a plurality of host cells. Vectors can be transformed into microbial host cells (e.g., bacterial cells, yeast cells, or algal or microalgal cells) for expression using chemical transformation, electroporation, or biolistic methods. Vectors can be transfected into eukaryotic host cells (e.g., mammalian, e.g., HEK 293 or CHO cell, or insect cells) for expression using a cationic lipid, polyethylenimine, Lipofectamine™, or ExpiFectamine™, or electroporation.

In some embodiments, the host cell, in culture, is a microbial cell or microorganism (used interchangeably herein), e.g., a bacterium, a cyanobacterium, a fungus, a microalga, or an alga. The microbial host cell can be a prokaryote, e.g., a bacterium, such as, but not limited to, *Escherichia coli, Bacillus subtilis, Salmonella sp., Aliivibrio fischeri, Pseudomonas fluorescens, Bacillus* sp., *Cupriavidus metallidurans, Deinococcus radiodurans,* and *Staphylococcus aureus,* or a non-filamentous cyanobacterium, such as *Synechocystis sp.* In some embodiments, the microbial cell is a eukaryote, e.g., a yeast, such as, but not limited to, *Saccharomyces cerevisiae, Pichia pastoris, Kluyveromyces lactis, Hansenula polymorpha, Yarrowia lipolytica,* and *Trichosporon cutaneum.* As an example, synthetic constructs can be transformed into both standard *E.coli* MG1655 (ATCC 700926) and optimized *E.coli* LABEC01 cells for expression of proteins of interest. The *E. coli* MG1655 LABEC01 lab strain can be grown in lysogeny broth (LB) medium and has been adapted for growth in M9 minimal medium. (See, e.g., Hasty et al., WO2016/133830A1, US2018/149633A1).

In some embodiments, the expression cassette is in a plasmid which has been introduced into the microorganism. In some embodiments, the expression cassette is integrated into the genome of the microorganism. In some embodiments, colonies or cultures of microorganisms are lyophilized (freeze-dried). In some embodiments, the colonies or cultures of microbes or microorganisms housed in the microfluidic chips are one or more different species. In some embodiments, the colonies or cultures of microbes or microorganisms are the same species. In some embodiments, the detectable protein is a fluorescent protein. In some embodiments, the fluorescent protein is selected from the group consisting of a green fluorescent protein, a yellow fluorescent protein, a cyan fluorescent protein, a red-shifted green fluorescent protein (rs-GFP), and miniSOG. In some embodiments, the detectable protein is a luminescent protein. In some embodiments, the luminescent protein is bacterial luciferase (Lux). In some embodiments, the microfluidic device comprises a plurality of microbial colonies or cultures wherein each of said plurality of colonies or cultures comprises an expression cassette comprising a biosensor or promoter operably linked to a polynucleotide encoding a detectable protein reporter wherein transcription of the reporter is modulated by the presence of a different analyte than the biosensor or promoter in the other of said plurality of colonies or cultures.

Alternatively, methods for stable genomic integration of expressions cassettes encoding protein(s) of interest can be employed to make a cell line of biosensing mammalian cells. (See, e.g., Zhang, Crispr-Cas Systems and Methods for Altering Expression Of Gene Products, WO2014093661 A2; Frendewey et al., Methods and Compositions for the Targeted Modification of a Genome, US9228208 B2; Church et al., Multiplex Automated Genome Engineering, WO2008052101A2, US8153432 B2; Bradley et al., Methods Cells and Organisms, US2015/0079680 A1; Begemann et al., Compositions and Methods for Modifying Genomes, WO2017141173A2; Gill et al., Nucleic acid-guided nucleases, US9982279 B1; Minshull et al., Enhanced nucleic acid constructs for eukaryotic gene expression, US9428767B2, US9580697B2, US9574209B2; Minshull et al., DNA Vectors, Transposons And Transposases For Eukaryotic Genome Modification, US10041077B2; McGrew et al., Hybrid Promoter and Uses Thereof, US 11028410B2; McGrew et al., Expression from Transposon-Based Vectors and Uses, US 11098310B2; McGrew et al., Inducible Expression From Transposon-Based Vectors and Uses, US 2019/0185881A1). Optionally, the transfectant or transformant cells will be provided with a recombinant expression cassette for a selectable marker, for example, but not limited to, one or more of the following: glutamine synthase, dihydrofolate reductase, puromycin-N acetyl transferase, blasticidin-S deaminase, hygromycin phosphotransferase, aminoglycoside phosphotransferase, nourseothircin N-acetyl transferase, or a protein that binds to zeocin.

Cloning DNA. Cloning of DNA is carried out using standard techniques (see, e.g., Sambrook et al. (1989) Molecular Cloning: A Laboratory Guide, Vols 1-3, Cold Spring Harbor Press, which is incorporated herein by reference). For example, a cDNA library may be constructed by reverse transcription of polyA+ mRNA, preferably membrane-associated mRNA, and the library screened using probes specific for human immunoglobulin polypeptide gene sequences. In one embodiment, however, the polymerase chain reaction (PCR) is used to amplify cDNAs (or portions of full-length cDNAs) encoding an immunoglobulin gene segment of interest (e.g., a light or heavy chain variable segment). The amplified sequences can be readily cloned into any suitable vector, e.g., expression vectors, minigene vectors, or phage display vectors. It will be appreciated that the particular method of cloning used is not critical, so long as it is possible to determine the sequence of some portion of the polypeptide of interest, e.g., antibody sequences.

Sequencing of DNA is carried out using standard techniques (see, e.g., Sambrook et al. (1989) Molecular Cloning: A Laboratory Guide, Vols 1-3, Cold Spring Harbor Press, and Sanger, F. et al. (1977) Proc. Natl. Acad. Sci. USA 74: 5463-5467, which is incorporated herein by reference), or so-called "Next Generation Sequencing" (NGS) techniques. By comparing the sequence of the cloned nucleic acid with published sequences of genes and cDNAs, one of skill will readily be able to determine, depending on the region sequenced. One source of gene sequence information is the National Center for Biotechnology Information, National Library of Medicine, National Institutes of Health, Bethesda, MD. Embodiments of the present invention can involve NGS sequencing, as a preferred method for confirming the presence of all engineered DNA constructs prior to the transfection step(s). (See, e.g., Buermans, H. P. J., & den Dunnen, J. T., Next generation sequencing technology: Advances and applications, Biochimica et Biophysica Acta - Molecular Basis of Disease 1842(10): 1932-1941 (2014)).

Chemical synthesis of parts or the whole of a coding region containing codons reflecting desired protein changes can be cloned into an expression vector by either restriction digest and ligation of 5' and 3' ends of fragments or the entire open reading frame (ORF), containing nucleotide overhangs that are generated by restriction enzyme digestion and which are compatible to the destination vector. The fragments or inserts are typically ligated into the destination vector using a T4 ligase or other common enzyme. Other useful methods are similar to the above except that the cut site for the restriction enzyme is at location different from the recognition sequence. Alternatively, isothermal assembly (i.e., "Gibson Assembly") can be employed, in which nucleotide overhangs are generated during synthesis of fragments or ORFs; digestion by exonucleases is employed. Alternatively, nucleotide overhangs can be ligated ex vivo by a ligase or polymerase or in vivo by intracellular processes.

Alternatively, homologous recombination can be employed, similar to isothermal assembly, except exonuclease activity of T4 DNA ligase can used on both insert and vector and ligation can be performed in vivo.

Another useful cloning method is the so-called "TOPO" method, in which a complete insert containing a 3' adenosine overhang (generated by Taq polymerase) is present, and Topoisomerase I ligates the insert into a TOPO vector.

Another useful cloning method is degenerate or error-prone PCR exploiting degenerate primers and/or a thermally stable low-fidelity polymerase caused by the polymerase within certain reaction conditions. Fragments or inserts are then cloned into an expression vector.

The above are merely examples of known cloning techniques, and the skilled practitioner knows how to employ any other suitable cloning techniques.

Isolated DNA can be operably linked to control sequences or placed into expression vectors, which are then transfected into host cells that do not otherwise produce the protein of interest (e.g., a selectable marker or detectable marker), to direct the synthesis of the protein of interest, in the recombinant host cells. Recombinant production of antibodies is well-known in the art.

Nucleic acid is operably linked when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, operably linked means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

Many vectors are known in the art for recombinant engineering of microbial biosensor strains for use in the microfluidic chip. Vector components can include one or more of the following: a signal sequence (that may, for example, direct secretion of the expressed protein by the recombinant host cells); an origin of replication, one or more selection marker and/or reporter protein encoding genes (that may, for example, encode a fluorescent protein, such as a green fluorescent protein (GFP), an enhanced green fluorescent protein (EGFP), a red-shifted green fluorescent protein (rs-GFP), a yellow fluorescent protein (YFP), a red fluorescent protein (RFP), a cyan fluorescent protein (e.g., CyOFP1), mini Singlet Oxygen Generator (miniSOG), a luminescent protein (e.g., luciferase), or the like, or may confer antibiotic or other drug resistance, or complement auxotrophic deficiencies of the host cells or supply critical nutrients not available in the medium, e.g., dihydrofolate reductase or glutamine synthetase selection markers), an enhancer element, a promoter, and a transcription termination sequence, all of which are well-known in the art.

Useful secretory signal peptide (SP) sequences are known in the art, and these can be added, adjacent or distal, for the purpose of facilitating secretion or other cellular localization of the protein of interest. An example of a useful SP sequence is the IGKV1-39*01 SP signal peptide.

Efficiency of transformation or transfection can also be a consideration in choosing an appropriate expression system. Electroporation can be a suitable method given its effectiveness, relative low cost and the fact that high-throughput during this step is not critical. Additionally, the ratio of immunoglobulin light chain to heavy chain can be varied during the co-transfection to improve expression of certain variants. The product yield for a given protein of interest or variant has to be sufficient to survive numerous handling steps and produce a signal high enough to be detected by the chosen fluorescence detector.

Cell Culture. A protein of interest (POI) is typically obtained by culturing the transfected or transformed host cells under physiological conditions allowing the cells to express recombinant proteins. The culturing of a microbial biosensor strain, such as, an *E. coli biosensor,* can be by any conventional type of culture, such as batch, fed-batch, intensified fed-batch, or continuous. Suitable continuous cultures included repeated batch, chemostat, turbidostat or perfusion culture. For purposes of the present invention, the desired scale of the recombinant expression will be dependent on the type of expression system and the quantity of microfluidic chips to be employed, the number of arrays and cell trap complexes in the chip(s).

A batch culture starts with all the nutrients and cells that are needed, and the culture proceeds to completion, i.e. until the nutrients are exhausted or the culture is stopped for some reason.

A fed-batch culture is a batch process in the sense that it starts with the cells and nutrients but it is then fed with further nutrients in a controlled way. The fed-batch strategy is typically used in bio-industrial processes to reach a high cell density in the bioreactor. The feed solution is usually highly concentrated to avoid dilution of the bioreactor. The controlled addition of the nutrient directly affects the growth rate of the culture and allows one to avoid overflow metabolism (formation of metabolic by-products) and oxygen limitation (anaerobiosis). In most cases the growth-limiting nutrient is glucose which is fed to the culture as a highly concentrated glucose syrup (for example 500-850 g/L).

Different strategies can be used to control the growth in a fed-batch process. For example, any of dissolved oxygen tension (DOT, pO2), oxygen uptake rate (OUR), glucose concentration, lactate concentration, pH and ammonia concentration can be used to monitor and control the culture growth by keeping that parameter constant. In a continuous culture, nutrients are added and, typically, medium is extracted in order to remove unwanted by-products and maintain a steady state. Suitable continuous culture methods are repeated batch culture, chemostat, turbidostat and perfusion culture.

In a repeated batch culture, also known as serial subculture, the cells are placed in a culture medium and grown to a desired cell density. To avoid the onset of a decline phase and cell death, the culture is diluted with complete growth medium before the cells reach their maximum concentration. The amount and frequency of dilution varies widely and depends on the growth characteristics of the cell line and convenience of the culture process. The process can be repeated as many times as required and, unless cells and medium are discarded at subculture, the volume of culture will increase stepwise as each dilution is made. The increasing volume may be handled by having a reactor of sufficient size to allow dilutions within the vessel or by dividing the diluted culture into several vessels. The rationale of this type of culture is to maintain the cells in an exponentially growing state. Serial subculture is characterized in that the volume of culture is always increasing stepwise, there can be multiple harvests, the cells continue to grow and the process can continue for as long as desired.

In the chemostat and turbidostat methods, the extracted medium contains cells. Thus, the cells remaining in the cell culture vessel must grow to maintain a steady state. In the chemostat method, the growth rate is typically controlled by controlling the dilution rate i.e. the rate at which fresh medium is added. The cells are cultured at a sub-maximal growth rate, which is achieved by restricting the dilution rate. The growth rate is typically high. In contrast, in the turbidostat method, the dilution rate is set to permit the maximum growth rate that the cells can achieve at the given operating conditions, such as pH and temperature.

In general, before delivery as inoculant to the cell trap complexes of the microfluidic chip, the transfected or transformed host cells are typically cultured by any conventional type of bioreactor culture, such as batch, fed-batch, intensified fed-batch, or continuous. Suitable continuous cultures included repeated batch, chemostat, turbidostat or perfusion culture with product and cell retention or solely cell retention. Bioreactors for protein production, which can be reusable or single-use bioreactors, typically can contain a volume of liquid culture medium of about 50 L to about 4000 L (e.g., 50 L, 60 L, 75 L, 100 L, 250 L, 500 L, 650 L, 750 L, 1000 L, 1250 L, 1500 L, 1750 L, 2000 L, 2250 L, 2500 L, 2750 L, 3000 L, 3250 L, 3500 L, 3750 L, or 4000 L), as desired.

The ordinarily skilled artisan is familiar with useful culture conditions, such as temperature (typically, but not necessarily, about 37° $\pm$ 1°C), pH-- typically, but not necessarily, the cell culture medium is maintained within the range of about pH 6.5-7.5-oxygenation, and the like.

The host cells used to produce the protein of interest or "POI" (e.g., non-glycosylated or glycosylated proteins) in the invention can be cultured in a variety of media suitable for the type of host cell chosen. Commercially available media such as Ham's F10 (Sigma-Aldrich), Minimal Essential Medium (MEM) (Sigma-Aldrich), RPMI-1640 (Sigma-Aldrich), and Dulbecco's Modified Eagle's Medium ((DMEM), Sigma-Aldrich) can be suitable for culturing the host cells. In addition, any of the media described in Ham et al., Meth. Enz. 58: 44 (1979), Barnes et al., Anal. Biochem. 102: 255 (1980), U.S. Patent Nos. 4,767,704; 4,657,866; 4,927,762; 4,560,655; or 5,122,469; WO90103430; WO 87/00195; or U.S. Patent Re. No. 30,985 can be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as Gentamycin™ drug), trace elements (defined as inorganic compounds usually present at final concentrations in the micromolar range), and glucose or an equivalent energy source, such that the physiological conditions of the cell in, or on, the medium promote expression of the protein of interest by the host cell; any other necessary supplements may also be included at appropriate concentrations that would be known to those skilled in the art.

In some embodiments, recombinant microbial (e.g., *Escherichia coli*) biosensor strains can be cultured in a bioreactor. The bioreactor can be a stainless steel, glass or plastic vessel of 0.01 (i.e., 10-mL) to 10000 (ten thousand) liters capacity, for example, 0.01, 0.015, 0.10, 0.25, 0.30, 0.35, 1, 2, 5, 10, 15, 20, 25, 30, 50, 75, 100, 500, 1000, 2500, 5000 or 8000 liters. The vessel is usually rigid but flexible plastic bags or bioreactor liners can be used. These flexible plastic bioreactor bags and liners are generally of the "single use" type. In an intensified fed-batch culture, culture vessels, reactors or chambers, of any of various capacities are used to grow suspensions of cells. Each culture vessel can be connected via inlets to an array of porous tangential flow filters which in turn are connected via outlets back to the culture vessel. After cell growth, the suspensions of cells and growth medium are pumped through the array of porous tangential flow filters to concentrate the cell suspension. The cell suspension is recycled through the filters and culture vessel allowing a portion of the old growth medium to be removed. A supply of fresh sterile medium is added to the concentrated cell suspension to maintain a nominal volume in the culture vessel. (See, e.g., Zijlstra et al., Process for the culturing of cells, US8119368, US8222001, US8440458).

In a perfusion or continuous culture, the extracted medium is depleted of cells, because most of the cells are retained in the culture vessel, for example, by being retained on a membrane through which the extracted medium flows. However, typically such a membrane retains 100% of cells, and so a proportion are removed when the medium is extracted. Alternatively, sonic cell separation technology achieves separation of cells from the media matrix with high-frequency, resonant ultrasonic waves rather than using a physical barrier, unlike tangential-flow filtration (TFF) or alternating tangential flow filtration (ATF); the cells are held back using an acoustic field as the bioprocess fluid flows through an open channel. The use of acoustic waves allows differentiation of particles of equal size, and thus the technology can be used for the separation of particles from the nano- to macro- scales. (See, e.g., Challenger, C.A., An acoustic wave-based technology for cell harvesting applications may help enable continuous manufacturing, BioPharm International 30(9):30 (2017)). Regardless of the technology employed to separate the cells from the extracted medium, it may not be crucial to operate perfusion cultures at very high growth rates, as the majority of the cells are retained in the culture vessel.

Continuous cultures, particularly repeated batch, chemostat and turbidostat cultures, are typically operated at high growth rates. According to common practice, it is typical to seek to maintain growth rates at maximum, or close to maximum, in an effort to obtain maximum volumetric productivity. Volumetric productivity is measured in units of protein quantity or activity per volume of culture per time interval. Higher cell growth equates to a higher volume of culture being produced per day and so is conventionally considered to reflect a higher volumetric productivity. A suitable fully continuous process can have a perfusion bioreactor coupled to recombinant protein harvesting and protein purification steps, for example, a multi-column chromatography capture step, followed by flow-through virus inactivation, multi-column intermediate purification, a flow-through membrane adsorber polishing step, continuous sterile or viral filtration and a final ultrafiltration step operated in continuous mode. (See, e.g., Crowley et al., Process for cell culturing by continuous perfusion and alternating tangential flow, US8206981).

The cell density is commonly monitored in cell cultures. In principle, a high cell density would be considered to be desirable since, provided that the productivity per cell is maintained, this should lead to a higher productivity per bioreactor volume. However, increasing the cell density can actually be harmful to the cells, and the productivity per cell is reduced. There is therefore a need to monitor cell density. To date, in cell culture processes, this has been done by extracting samples of the culture and analyzing them under a microscope or using a cell counting device such as the CASY TT device sold by Scharfe System GmbH, Reutlingen, Germany. It can be advantageous to analyze the cell density by means of a suitable probe introduced into the bioreactor itself (or into a loop through which the medium and suspended cells are passed and then returned to the bioreactor). Such probes are available commercially from Aber Instruments, for example the Biomass Monitor 220, 210 220 or 230. The cells in the culture act as tiny capacitors under the influence of an electric field, since the non-conducting cell membrane allows a build-up of charge. The resulting capacitance can be measured; it is dependent upon the cell type and is directly proportional to the concentration of viable cells. A probe of 10 to 25 mm diameter uses two electrodes to apply a radio frequency field to the biomass and a second pair of electrodes to measure the resulting capacitance of the polarized cells. Electronic processing of the resulting signal produces an output which is an accurate measurement of the concentration of viable cells. The system is insensitive to cells with leaky membranes, the medium, gas bubbles and debris. Alternatively, cell viability can be measured by use of a vital dye (or vital stain) to stain small-aliquot samples of culture sampled periodically, and microscopically enumerated to determine viable cell count. For example Trypan blue is a vital dye commonly used for this purpose. Automated cell counters supplied by Beckman (e.g., Vi-Cell™ XR) and other companies are available. Examples include cell counting instruments made by other manufacturers, e.g., Nova Biomedical, Olympus, Thermo Fisher Scientific and Eppendorf. Cells can also be counted using flow cytometry or manually by using a hemocytometer

Typically, source cell culture of the biosensing strains that will be used to spot a microfluidic device has an OD600 value of approximately 0.1 to 1.0, which equates to approximately $1.0 \times 10^8$ cells/mL to $1.0 \times 10^9$ cells/mL. (See, e.g., bionumbers.hms.harvard.edu/bionumber.aspx?id=100985&vei=14&trm=OD600+coli&org=) This can also be used as a typical high range for cell density in other liquid cell culture biosensors. Cell densities in the microfluidic device sensors presented here tend to be much higher than in liquid culture due to the close packing of cells in the microfluidic cell traps. If a bacterial cell is estimated to occupy approximately 5.0 $\mu m^3$ of excluded volume on average (approximating a cell as an extruded square shape 5.0 $\mu m$ long with a cross section 1.0 $\mu m$ X 1.0 $\mu m$), then close packing of this shape leads to a maximum cell density of $0.2 \times 10^{12}$ cells/mL. This close packing density estimate is a few orders of magnitude larger than what is typically expected for liquid culture cell density. This increased cell density allows for microfluidic devices to have a higher signal to noise ratio for a given volume relative to liquid cell culture of microbial biosensor strains, since the density of sensing units (cells) is higher in microfluidic cell traps.

Introducing Microbial Biosensor Stains to the Microfluidic Chip and Signal Detection

Recombinant microbial biosensor strains, such as but not limited to, *Escherichia coli* biosensor strains can operate as an assay of analytes when incorporated into the microfluidic chip of the invention, designed to sample the process water, which is mixed and diluted with concentrated defined culture medium. Microbial biosensor strains housed in the microfluidic chip are useful to detect and quantify a signal coming from the expression of a detectable marker in response to the presence of an analyte(s) of interest. For example, in some embodiments, fluidic, electronic, and optical systems can be designed to continuously draw and mix process water with a concentrated defined culture medium within a microfluidic chip housing the cells, and with an external detector (180) periodically acquiring and processing images of the microfluidic chip to detect and quantify the expression of a detectable marker, such as GFP. (See, e.g., Figure 8). Then the GFP signal for each strain can be calibrated to a concentration of the on-target analyte of interest, e.g., a contaminant. In this manner, a panel of different biosensor strains can be employed to continuously monitor a process water for a variety of analytes in the aqueous sample. Optionally, the marker signal data can be gathered and transmitted to an operator or database in real time.

The engineered microbial biosensor strains, e.g., *E. coli* biosensor strains, can be loaded into predetermined addressable locations arrayed in the inventive microfluidic device or chip during its manufacture, or post-manufacture. Culture biomass can be transferred from an agar plate or liquid culture into an array of cell spotting region(s) (300, 600) in the device, either individually and sequentially by hand or in parallel by pin tool. For example, the pin tool can be automated to robotically select and align the source and target colony arrays, retrieve biomass from the source array, and deposit it upon the target array. Following "loading" of the cell spotting region(s) in the microfluidic device, strain viability can be preserved either by air drying or freeze drying. For air drying, the colonies are allowed to dry naturally through evaporation. For freeze drying, the microfluidic chips are frozen, and vacuum pressure is applied to sublimate water vapor. For example, the microfluidic chip monolith containing the loaded cell spotting region(s) can be sealed against a flat surface to form the microfluidic channels using various methods; both chip halves can be treated with oxygen plasma to activate the surfaces before spotting and then brought into contact to form a covalent bond. Alternatively, a pressure-sensitive adhesive can be applied to one chip half and then compressed against the other to rupture adhesive vesicles at the contacting surfaces but not along the open microfluidic channels.

The recombinant microbial biosensor strains, e.g., *E. coli* strains, are typically viable in a microfluidic device when shielded from light at refrigerator temperature (4°C) to room temperature (20°C) for at least 30 days. When the microfluidic chip is booted in preparation for sensing, rich medium (e.g. Lysogeny Broth (LB)) is flowed into the chip to mix with the process water at a predetermined defined dilution ratio and hydrates the arrayed cell spotting region(s). Strains spotted within the cell spotting regions (300, 600) grow to confluence in 12-24 hours, throughout the first migration channel(s) (320, 620), the primary cell trap(s) (312, 612), and the second migration channel(s) (332, 632), and finally in the downstream cell trap(s) (340, 640), with excess culture overflowing each primary cell trap and downstream cell trap (see, Figure 3A-C), and then washing downstream through downstream array outlet channel(s) (135, 235, 435, 535), waste outlet channel(s) (138, 438, 538), and ultimately to downstream waste port(s) (140, 470, 570), and thence to an off-chip waste exhaust or receptacle. After revival of dehydrated strains, the culture medium can be swapped to a well-defined minimal medium (e.g. M9 or HM9) to ensure the purity of the medium stream and slow the growth rate of the sensing strains (See, e.g., Hasty *et al.,* "Microbial Microfluidic Biosensor," US11209412B2).

Culturing conditions within the microfluidic chip can be maintained to ensure that each strain signal has a stable baseline. Flow rates of process water and reference water and concentrated defined culture medium can be controlled by continuously measuring them using a flow meter (e.g., in a microfluidic device the sensing range can be 0-80 $\mu$l/min) and using a proportional-integral-derivative (PID) feedback loop to adjust driving pressure (e.g., headspace pressure in a closed fluidic vessel, force applied to a syringe pump, or meniscus elevation generating hydrostatic pressure). Culture temperature can be maintained at a temperature conducive to microbial physiological activity, e.g., 37°C, by measuring the temperature near the microfluidic chip using a probe (e.g. thermistor or thermocouple) and using a PID feedback loop to adjust output from a temperature control system (e.g. thermoelectric (Peltier) module, resistive heater, or compressor-based cooler). Culture pH can be maintained by including a buffer (e.g. MES, MOPS, HEPES, or PIPES) in the concentrated growth medium. With proper culturing conditions maintained, cellular responses can be continually measured within the microfluidic device for up to a few months.

In an embodiment in which an engineered recombinant biosensor strain produces a fluorescent protein reporter as the detectable marker in response to exposure to on-target water analytes of interest (e.g., contaminants), the fluorescent protein reporter can be a green fluorescent protein (GFP) variant, such as superfolder GFP (sfGFP). Cellular production of sfGFP can be measured by illuminating the cells with blue light (nominally 485 nm wavelength) and measuring the production of green light (nominally 510 nm wavelength). An appropriate sfGFP illumination source can comprise a blue light-emitting diode (LED) (e.g., Cree XLamp XP-E2 Blue, #XPEBBL-L1-0000-00301) and an excitation filter (e.g., Semrock 482/18 nm BrightLine single-band bandpass filter, #FF02-482/18). Additional spectrally-compatible fluorescent protein or dye readouts can be imaged with the use of a multi-band emission filter. For example, cellular production of the mKate2 red fluorescent protein can be imaged by illuminating the cells with an amber LED (e.g., Cree XLamp XP-E2 Amber, #XPEBAM-L1-0000-00901) through an excitation filter (e.g., Semrock 585/40 nm BrightLine single-band bandpass filter, #FF01-585/40). In addition to fluorescent light imaging, a transmitted light image of the cells within the inventive microfluidic device can be acquired by illuminating with a green LED (e.g., Cree XLamp XP-E2 Green, #XPEBGR-L1-0000-00A01). To distinguish detectable markers or reporters using multiple imaging channels, emitted fluorescence and transmitted green light should pass through a compatible emission filter (e.g., for this configuration, Semrock 527/645 nm BrightLine dual-band bandpass filter, #FF01-527/645). Monochrome images in each spectral channel can be sequentially acquired by a CCD or CMOS camera (e.g., FLIR Blackfly S Mono 1.6 MP GigE Vision, #BFS-PGE-16S2M-CS).

Experimental noise in image datasets can be minimized by employing tight temporal control of LED switching, LED warm-up, and camera triggering. For long-term continuous sensing, illumination should be stable over multi-day timescales and large temperature swings (>10°C). LED driver circuit design should be constant-current and adjustable, such that any variation in LED illumination with time or temperature can be compensated for in hardware, based on known calibration curves.

In alternative embodiments, the basis of cellular readout can be altered from fluorescence to luminance, absorbance, turbidity, or electrochemical. Compatible readout mechanisms include enzyme production (e.g. bioluminescence or chemiluminescence), cellular lysis, or cellular agglutination.

Fluid flow throughout the microfluidic chip is driven by establishing pressure differentials across the input and output ports. These pressure differentials can be applied using several methods, including pressurizing the headspace of a closed fluidic vessel with dip tube outlet, applying force to a syringe pump, or elevating an open reservoir to generate hydrostatic pressure. All of these methods are preferable to positive-displacement pump styles (e.g. rotary, piston, diaphragm, peristaltic) in that the resulting fluid flow is non-pulsatile. Macro-scale pressure pulses typically generate large flow waves within microfluidic devices that can disrupt colony stability, thereby increasing experimental noise.

In designing microfluidic chips, critical channel resistances were carefully balanced according to fluid theory. Using the Hagen-Poiseuille equation for laminar flow through low aspect ratio rectangular channels (i.e., a channel in which the height dimension is much less than the width and length dimensions), the resistance, R, of any length of channel can be estimated as in Equation I (below):

Equation I:

$$R = \frac{12\mu L}{wh^3}$$

wherein $\mu$ is the fluid viscosity, L is the channel length, w is the channel width, and h is the channel height.

Following this calculation of resistance, R, the Navier-Stokes equation can be simplified in the low Reynolds number flow regime to approximate the flow rate, Q, of a fluid through a microfluidic channel as shown in Equation II (below):

Equation II:

$$Q = \frac{\Delta P}{R}$$

wherein $\Delta P$ is the pressure differential across the channel.

For purposes of the invention, in working with aqueous solutions, we assume negligible viscosity changes across media and water sources.

Soft lithography is a common technique for rapidly prototyping microfluidic devices whereby flexible devices are molded from a rigid master mold fabricated using photolithography. Fabricating the master mold involves the sequential deposition of multiple photoresist layers on a silicon wafer, whereby each layer establishes some channel features with height corresponding to the thickness of that photoresist layer. Typically, the number of channel feature heights is designed to be minimal in order to reduce the total number of photoresist layers required, thereby simplifying master mold fabrication. As such, the "tuning" of channel resistances toward design targets can more easily be accomplished by altering channel length and width dimensions than by altering the height dimension. Resultantly, the majority of our chip design effort was focused here.

We verified microfluidic channel designs encompassed within the present invention by using a nodal analysis technique common to electrical circuit design, because the simplified $Q = \frac{\Delta P}{R}$ relationship (i.e., Equation II) applicable to microfluidic flows is directly analogous to the well-known relationship in electrical engineering (Ohm's Law), as shown in Equation III (below):

Equation III:

$$I = \frac{\Delta V}{R}$$

wherein $I$ represents the current through the resistive component and $\Delta V$ represents the voltage differential across the resistive component.

In accordance with fluid theory, each microfluidic chip design required tuning several microfluidic channel dimensions to produce channel resistances that meet the design goals. A first design goal shared by all chip designs is to ensure equivalent flows through all branch channels (210, 310, 410, 510, 610) in all microbial process and reference monitoring arrays (110, 120). This design goal ensures that convective and diffusive flows at all cell trap complexes are equivalent, which ensures the uniformity of cellular responses across the chip. This design goal is met by fabricating the channels comprising the manifold that surrounds the cell trap complexes (200, 402, 502, 602, 220, 403, 503, 603) at a height much greater than the height of the branch channels in contact with the cell trap complexes (210, 310, 410, 510, 610), such that the flow rate through each branch channel is dominated by the resistance of the branch channel rather than the resistance of the manifold channels. Embodiments of the microfluidic chips designed for housing prokaryotic microbial biosensor strains, such as but not limited to *E. coli* strains, typically have apertures and channel height ranges as shown in Table 1, below, but these height ranges can be modified for culturing eukaryotic biosensor cell lines and strains within the inventive microfluidic chip, as explained herein below.

Table 1. Typical aperture and channel heights calculated for microfluidic chips designed for housing prokaryotic cells. Note that channel heights can be increased beyond these ranges in embodiments where a person of ordinary skill in the art wishes to apply driving pressures at the chip inlets below 15 kPa in order to achieve flow rates similar to those described herein.

| Structure | Height of aperture, chamber, or channel (micrometers) |
|---|---|
| first inlet port (150, 415, 515) | 40-90 |
| first resistance-tuned inlet channel (155) | 40-90 |
| second inlet port (160, 415, 515) | 40-90 |
| second resistance-tuned inlet channel (165) | 40-90 |
| primary water inlet channel (416, 516) | 40-90 |
| water junction (400, 500) | 40-90 |
| post-water junction water channel (420, 520) | 40-90 |
| media inlet port (417, 517) | 40-90 |
| primary media inlet channel (455, 555, 655) | 40-90 |
| media junction (440, 540) | 40-90 |
| post-media junction media channel (425, 525, 625) | 40-90 |
| mixina junction (450, 550) | 40-90 |
| mixina channel (460, 560) | 40-150 |
| upstream array inlet channel (132, 432, 532) | 40-90 |
| upstream manifold channels (200, 402, 502, 602) | 90-150 |
| branch channels (210, 310, 410, 510, 610) | 40-90 |
| cell spotting region (300, 600) | about 1 to about 25 |
| primary cell trap (312, 612) | about 1 to about 25 |
| first migration channel (320, 620) | about 1 to about 25 |
| second migration channel (332, 632) | about 1 to about 25 |
| downstream cell trap (340, 640) | about 1 to about 25 |
| downstream manifold channel (220, 403, 503, 603) | 90-150 |
| downstream array outlet channel (135, 235, 435, 535) | 90-150 |
| waste outlet channel (138, 438, 538) | 90-150 |
| downstream waste port (140, 470, 570) | 90-150 |
| light emission calibration channel (427, 527, 627) | 90-150 |

For microfluidic chip embodiments in which flow(s) of concentrated growth media is mixed with flows of process and reference water on-chip (e.g. Fig. 4A, 5A, 5B), a second design goal is to balance inlet channel resistances such that, given equivalent driving pressures at the media (417, 517) and water (415, 515) inlet ports, the concentrated growth medium is diluted with the process and reference water streams to a final concentration of 1X upstream of the microbial process and reference monitoring arrays (110, 120). This ensures that the engineered sensing strains in the cell trap complexes (130, 230, 430, 530, 630) are exposed to the optimal concentration of growth medium.

Merely as an example, for the microfluidic chip embodiment described in Example 2 herein below and illustrated in Figure 5B, in which flow of a single concentrated growth medium stream is split to mix with process water and reference water streams before flowing through a pair of microbial process monitoring arrays and microbial reference monitoring arrays, the concentrated growth medium input to the combination media inlet port and media junction (517/540) could have a starting concentration of 5X the desired working concentration at the cell trap complexes (530). In this scenario, we wish to establish a 1:4 part media:water flow rate ratio at both mixing junctions (550) in order to dilute the 5X concentrated growth medium to a final concentration of 1X downstream of the mixing junctions (550) and mixing channels (560). Given equivalent driving pressures at the combination media inlet port and media junction (517/540) and the inlet ports (515), we can accomplish this by tuning the dimensions of the primary media inlet channels (555) and primary water inlet channels (516) such that the ratio of their respective resistance values (R) is 4:1.

The combined total flow rate through the chip is determined by the pressure differentials applied across the inlet and outlet ports and the effective resistances between these ports. For the microfluidic chip embodiment illustrated in Figure 5B, we applied equivalent driving pressures at the combination media inlet port and media junction (517/540) and inlet ports (515). Due to the 4:1 ratio of primary media inlet channel (555) resistance to primary water inlet channel (516) resistance, calculated as described above, process and reference water each flowed from an inlet port (515) to the downstream waste port (570) at a rate 4 times that of concentrated growth medium flow from the combination media inlet port and media junction (517/540) through each microbial monitoring array to the downstream waste port (570). Because the concentrated growth medium flow in this embodiment splits at the inlet port (517/540) and flows through both microbial monitoring arrays to establish this flow ratio through each array, the total concentrated growth medium flow rate into the combination media inlet port and media junction (517/540) is doubled (i.e., process water and reference water each flow through the chip at a rate 2 times that of the concentrated growth medium).

The combined total flow rate through the microfluidic chip is set by the desired flow rate past each cell trap complex, which is approximately equivalent across the chip. For monitoring responses from microbial strains in the microfluidic chip embodiment illustrated in Figure 5B, we fabricated the height of the cell trap complex at about 4 micrometers and the height of the branch channel at about 50 micrometers. For these feature dimensions, we discovered empirically that a flow rate of 0.5 mL/day through the branch channel adjacent to a cell trap complex supplied fresh nutrients and analyte to the microbial strains housed in the cell trap complex without disrupting the colony structure and causing cell wash-out. Because the total flow through the chip is split evenly across all cell trap complexes, and there were 100 cell trap complexes in total, the total flow through the chip was therefore 50 mL/day. Due to the flow ratios set by the channel resistances as described above, the total flow of concentrated growth medium through the chip was 10 mL/day, and the total flow of each process water and reference water was 20 mL/day. Depending on the quality of channel fabrication (i.e., defects and dimensional variability can affect channel resistances) as well as potential clogging of channels with cellular effluent over time, we found that a typical driving pressure of 14-35 kPa (sometimes 3-100 kPa) was required at the inlet ports, relative to the outlet port being maintained at atmospheric pressure, in order to establish these total flow rates.

For the microfluidic chip embodiment shown in Figure 5B, in which the flow of a concentrated growth medium is mixed with flows of process and reference water on-chip, we empirically determined the equivalent resistance of a fabricated chip as follows. We first calculated the resistance of our flow driving apparatus without the microfluidic chip connected, whereby a source reservoir was elevated to generate hydrostatic pressure to drive the flow of water sequentially through a length of tubing, a three-way splitter, and three equivalent lengths of tubing into a waste reservoir. For a source-to-waste meniscus elevation differential of 32.5 inches of water (corresponding to an applied hydrostatic pressure of 8087 Pa), we measured a flow rate of 4.8 mL of water over an elapsed time of 10 minutes, corresponding to a flow rate of 480 $\mu$L/min. The flow driving apparatus resistance is therefore 16.8 Pa*min/$\mu$L. For the same apparatus with a microfluidic chip connected between the source and waste reservoirs such that the three equivalent lengths of tubing were connected to the three chip inlets and waste was collected at the chip outlet, we measured a flow rate of 1.32 mL of water over an elapsed time of 49 minutes, corresponding to a flow rate of 26.9 $\mu$L/min. The combined resistance of the apparatus plus chip is therefore 300.2 Pa*min/$\mu$L. The equivalent resistance of the chip can be determined by subtracting the apparatus resistance from the apparatus plus chip resistance. Here we calculate it as 283.4 Pa*min/$\mu$L.

The exemplary channel dimensions provided above for the embodiment illustrated in Figure 5B and Table 1 (above) were specific to an embodiment of the invention for monitoring prokaryotic microbial cells. However, we can envision monitoring colonies of cultured eukaryotic host cells (e.g., cultured mammalian cells, e.g., HEK 293 or CHO cells, yeast cells, algal cells, or insect cells) with proper modifications to the channel dimensions. Critically, the height of the cell trap complex must be scaled to accommodate the cell size. For example, a cell trap complex height of 25 micrometers might be appropriate for monitoring cultured CHO cells. This adjustment will require other channel dimensions to scale appropriately in order to maintain channel resistance (and, resultingly, flow rate) ratios, as necessary. Because the resistance of high-aspect-ratio rectangular channels is highly sensitive to channel height as previously discussed (

$R = \frac{12\mu L}{wh^3}$ ), adjusting channel height in chip designs is often the most effective method to tune channel resistance. Note that in increasing channel dimensions to accommodate larger cell sizes, such as those of eukaryotic cells, the overall chip resistance, R, will likely decrease. This can necessitate lowering driving pressures at the chip inlet ports in order to maintain sufficiently low flow rates.

By way of further illustration, the following numbered embodiments are encompassed by the present invention:

Embodiment 1: A microfluidic chip (100) for parallel culture of recombinant biosensing microbes, comprising, encompassed by a chip solid matrix (796):

(a) one or more paired microbial process monitoring arrays (110) and microbial reference monitoring arrays (120), each reference monitoring array corresponding to one of the process monitoring array(s), each process monitoring array (110) and each reference monitoring array (120) comprising an upstream end and a downstream end, and further comprising:

(i) an upstream array inlet channel (132, 432, 532), fluidly connected and upstream to one or more upstream manifold channels (200, 402, 502, 602), and thence fluidly connected to:

(ii) a network of branch channels (210, 310, 410, 510, 610), branching in parallel within the process monitoring array(s) (110) and reference monitoring array(s) (120), wherein a cell trap complex (130, 230, 430, 530, 630) is in fluid connection to each branch channel (210, 310, 410, 510, 610), and is capable of receiving fresh liquid cell culture medium from the branch channel; and wherein each branch channel is fluidly connected via a downstream manifold channel (220, 403, 503, 603), which is configured to receive cell-conditioned medium outflow from the branch channel (210, 310, 410, 510, 610), wherein the downstream manifold channels are configured to convey the cell-conditioned medium outflow further downstream to:

(iii) a downstream array outlet channel (135, 235, 435, 535), which is configured to fluidly convey the cell-conditioned medium outflow;

(b) for conveying liquid to the process monitoring array(s) (110), a first inlet port (150, 415, 515), fluidly connected via a first resistance-tuned inlet channel (155), upstream to the first upstream array inlet channel (132, 432, 532), which is fluidly connected to the process monitoring array (110);

(c) for conveying liquid to the reference monitoring array(s) (120), a second inlet port (160, 415, 515), fluidly connected via a second resistance-tuned inlet channel (165), upstream to the second upstream array inlet channel (132, 432, 532), which is fluidly connected to the reference monitoring array (120); and

(d) a waste outlet channel (138, 438, 538), which is configured to fluidly receive the cell-conditioned medium outflow from the downstream array outlet channels (135, 235, 435, 535) and to fluidly convey the outflow to a downstream waste port (140, 470, 570).

Embodiment 2: The microfluidic chip of Embodiment 1, wherein each cell trap complex (130, 230, 430, 530, 630) comprises:

(i) a cell spotting region (300, 600);

(ii) an adjacent primary cell trap (312, 612), fluidly connected to the cell spotting region by a first migration channel (320, 620) having a broader end (322) and a narrower end (324), wherein the first migration channel tapers from the cell spotting region (300, 600) at the broader end to the primary cell trap (312, 612) at the narrower end, each primary cell trap having a side distal from the cell spotting region open to an adjacent microfluidic branch channel (210, 310, 410, 510, 610);

(iii) a second migration channel (332, 632), fluidly branching from the narrower end (324) of the tapering first migration channel; and

(iv) a plurality of downstream cell traps (340, 640) in a series adjacent to the primary cell trap, each downstream cell trap having a side with an opening to the second migration channel (332, 632) and having a side opposite the second migration channel open to the adjacent microfluidic branch channel (210, 310, 410, 510, 610), each downstream cell trap being capable of housing a viable microbial colony.

Embodiment 3: The microfluidic chip according to Embodiment 2, wherein the primary cell trap(s) (312, 612) and the downstream cell traps (340, 640) have a transparent ceiling to enable light emissions to be imaged by an external detector (180).

Embodiment 4: The microfluidic chip according to any one of Embodiments 2-3, wherein the cell spotting regions of the parallel cell trap complexes are configured to be compatible with a pin tool for delivery of microbial cells.

Embodiment 5: The microfluidic chip according to any one of Embodiments 1-4, wherein the first inlet port (150, 415, 515) and the second inlet port (160, 415, 515) are each configured to alternatively receive, at a predetermined pressure (P) at a flow rate (Q), either of:

(i) process water, or

(ii) reference water,

and wherein the first inlet port (150, 415, 515) and the second inlet port (160, 415, 515) are each configured to fluidly convey the received water to the first resistance-tuned inlet channel (155) or to the second resistance-tuned inlet channel (165), respectively.

Embodiment 6: The microfluidic chip according to any one of Embodiments 1-5, wherein the first resistance-tuned inlet channel (155) and the second resistance-tuned inlet channel (165), each further comprise:

(a) a primary water inlet channel (416, 516), fluidly connected and upstream to:
(b) a separate mixing junction (450, 550), wherein each mixing junction is fluidly connected and upstream to:

(c) a mixing channel (460, 560), which is fluidly connected and upstream to one of the plurality of the upstream array inlet channels (132, 432, 532), each of which is fluidly connected and upstream to one of the microbial process monitoring arrays (110) or one of the microbial reference monitoring arrays (120);
whereby the chip is configured for on-chip mixing of the process water and the reference water, with one or more concentrated defined liquid cell culture media.

Embodiment 7: The microfluidic chip according to Embodiment 6, wherein the first resistance-tuned inlet channel (155) and the second resistance-tuned inlet channel (165), each further comprise, fluidly connected and downstream to the primary water inlet channel (416, 516):
(d) a water junction (400, 500), fluidly connected and upstream to:

(e) more than one post-water junction water channels (420, 520), each of which is fluidly connected and upstream to the separate mixing junction of subpart (b) (450, 550);
wherein the water junction (400, 500) is configured to split the flow of liquid to the more than one post-water junction water channel(s).

Embodiment 8: The microfluidic chip according to any one of Embodiments 6-7, comprising:
(f) one or more media inlet ports (417, 517), each media inlet port being configured for fluidly receiving a single concentrated defined liquid cell culture medium from a source outside the chip, and being upstream and fluidly connected to:
(g) a primary media inlet channel (455, 555, 655), each primary media inlet channel being upstream and fluidly connected to:
(h) a media junction (440, 540), being upstream and fluidly connected to:
(i) a plurality of post-media junction media channels (425, 525, 625), each being upstream and fluidly connected to one of the separate mixing junctions of subpart (b) (450, 550).

Embodiment 9: The microfluidic chip according to any one of Embodiments 3-8, wherein the external detector (180) is configured to monitor the primary cell trap(s) (312, 612) and the downstream cell traps (340, 640) in each process monitoring array (110) and each reference monitoring array (120).

Embodiment 10: The microfluidic chip according to any one of Embodiments 1-9, comprising a light emission calibration channel (427, 527, 627).

Embodiment 11: The microfluidic chip according to any one of Embodiments 1-10, comprising a plurality of data matrices (195, 495, 595, 795) fabricated into an external surface of the chip solid matrix (796), for orientation of an image of light emissions to be collected from the paired microbial process monitoring arrays (110) and microbial reference monitoring arrays (120).

Embodiment 12: The microfluidic chip according to Embodiment 11, wherein the plurality of data matrices (195, 495, 595, 795) is configured to register the image of light emissions by affine transformation.

Embodiment 13: The microfluidic chip according to any one of Embodiments 1-12, wherein the microfluidic chip is designed to house colonies of recombinant biosensing microbial cells and the microbial cells are prokaryotic cells; and wherein the height of each aperture, port, channel, junction, cell spotting region, or cell trap is within a range set forth in Table 1 herein.

The following working examples are illustrative and not to be construed in any way as limiting the scope of the invention.

EXAMPLES

Example 1. Manufacture of Microbial Biosensor Strains and the Microfluidic Devices of the Invention

Engineered biosensor strains. Recombinantly engineered biosensor strain designs that we employed, for exemplary purposes, were based on promoters native to *Escherichia coli* and other bacteria that were identified to be sensitive to the analytes of interest. We designed 152 plasmid variants based on 23 promoter elements. (See, Cookson et al., "Engineered Biosensor Strains of *E. Coli* for Continuous Aerobic Detection of Analytes," PCT/US2024/025712).

Plasmid variants of a single promoter element typically included variations on the promoter sequence, ribosomal binding site (RBS), or other regulatory elements or genes. Of this set, we constructed 87 plasmid variants, either by *de novo* DNA synthesis, or by using standard cloning methods such as Gibson assembly. (D. G. Gibson, L. Young, R. Y. Chuang, J. C. Venter, C. A. Hutchison, and H. O. Smith, "Enzymatic assembly of DNA molecules up to several hundred kilobases," Nat Methods, vol. 6, no. 5, pp. 343-345, (2009), doi: 10.1038/nmeth.1318). When native gene sequences were required for amplification, *E. coli* K-12 MG1655 was used. Other microbial species and other recombinant means for engineering microbial biosensor strains are also known to the skilled artisan. (See, e.g., Hasty et al., "Microbial Microfluidic Biosensor," US11209412B2 and US2022/057378A1; Daunert et al., "Spores for the stabilization and on-site application of bacterial whole-cell biosensing systems," US8389263B2 and US2010/0304379A1).

<u>Microfluidic devices</u>. Microfluidic devices according to the invention contain networks of fluidic channels with cross-sectional dimensions on the micron ($\mu$m) scale. These small dimensions result in a low Reynolds number (i.e., in the field of fluid dynamics, a dimensionless quantity that predicts fluid flow patterns by quantifying the ratio between inertial and viscous forces; typically much less than 100 for microfluidic flows) that produces laminar (as opposed to turbulent) fluid flow within the microchannels. Devices with such small features can be manufactured using a variety of materials and methods. For example, rapid and inexpensive prototyping of microfluidic devices (or "chips") is often performed using soft lithography techniques, wherein an elastomer is molded from a patterned mold. (See, e.g., Duffy et al., "Rapid Prototyping of Microfluidic Systems in Poly(dimethylsiloxane)," Analytical Chemistry 1998 70 (23), 4974-4984, doi: 10.1021/ac980656z). Typically, the "master mold" is generated by patterning multiple layers of photoresist (i.e., SU-8) on a silicon wafer using photolithography to create channel structures of various heights. A two-part silicone elastomer (e.g. polydimethylsiloxane (PDMS), commercially available as SYLGARD®184, Dow Corning Corp.) is then mixed, poured, degassed, and cured against the master mold. Upon demolding, the cured elastomer monolith takes the inverse shape of the master mold, such that the channel structure formed by photoresist in the master mold becomes negative space in the elastomer replica. Finally fluidic ports are bored in the elastomer, and it is bonded to a flat surface (e.g., to glass following exposure to oxygen plasma) to seal the channel structure. After establishing fluidic connections at the device ports, external pressure can be applied to drive fluid flow through the microfluidic channels. All of the embodiments illustrated in Figure 1, Figure 4A, and Figure 5A-B were made by this process. As illustrated schematically in Figure 3A-C and Figure 6A, some embodiments of the chip can include support pillars (314, 614), present within the cell spotting regions (300, 600), to prevent the collapse of the chip solid matrix (796) in what is a relatively wide and shallow region of the chip. Such embodiments of the chip in which support pillars (314, 614) are typically employed are those made from elastomeric or other soft materials.

For intermediate-scale manufacture of the inventive microfluidic devices (e.g., a quantity of about 100 to about 1,000), embossing techniques are typically employed. These techniques place a soft or rigid "stamp" against a thermoplastic material (e.g., cyclic olefin copolymer (COC), cyclic olefin polymer (COP), or polymethyl methacrylate (PMMA)), typically under heat and/or pressure, to transfer the features from the stamp (in positive relief) into the thermoplastic (in negative relief). Embossed thermoplastic can then be sealed with pressure-sensitive adhesive (PSA) or by chemically bonding to elastomer or thermoplastic. When a rigid stamp is required, but patterned silicon master molds are too fragile to serve as the stamp, a daughter mold can be replicated from the master in PDMS, and then a granddaughter mold can be replicated from the PDMS in epoxy and used as the stamp. The chip shown in Figure 1 was made using this particular process.

Another intermediate-scale method for manufacturing the inventive microfluidic devices involves etching microchannels into glass. This can be accomplished using several techniques, which typically include some form of chemical etching and/or micromachining. Similar to embossed thermoplastic chips, etched glass chips can be sealed with pressure-sensitive adhesive (PSA) or by chemically bonding to elastomer or thermoplastic.

For mass-manufacture of the inventive microfluidic chips (e.g., a quantity of 1,000 or greater), injection molding is typically employed. In this technique, a multi-piece mold is typically micromachined from metal, and thermoplastic pellets are heated, injected, and cooled within the machined mold under pressure. Typically, the mold design must incorporate additional considerations such as feature aspect ratio, thermoplastic shrinkage, mold filling and purging, part release, and mold wear.

In embodiments where microfluidic chips are fabricated from thermoplastic or glass, gas permeability through the bulk material is typically much lower than through chips fabricated from PDMS. This can limit the diffusion rate of oxygen to aerobic cells cultured in the cell trap complexes and may necessitate delivering the required oxygen by other means (e.g., saturating the water and/or media inlet fluids with dissolved oxygen). Conversely, chips fabricated using gas-impermeable materials (e.g., glass or thermoplastic materials) may be preferable when culturing anaerobic cells in the cell trap complexes under low-oxygen conditions. In this scenario, the chip may be placed in an anaerobic chamber to ensure hypoxic conditions.

Example 2. <u>Use of the inventive microfluidic chip for in-lab characterization of *E. coli* biosensor strains</u>

An embodiment of the inventive microfluidic chip (illustrated in Figure 5B) was used to house and characterize the real-time sensing abilities of several exemplary recombinant *Escherichia coli* biosensor strains in the appropriate growth medium background, as described in Cookson et al., PCT/US2024/025712. recombinant *Escherichia coli* biosensor strains were operated to assay various analytes when incorporated into the microfluidic chip designed to sample process water, which was mixed and diluted with concentrated defined culture medium. The plurality of *E. coli* strains in the platform were useful to detect and quantify a signal coming from the expression of a detectable marker in response to the presence of the analyte of interest. For example, in some embodiments, fluidic, optical, and electronic systems were designed to continuously draw and mix process water with a concentrated defined culture medium within the microfluidic chip housing the cells and periodically acquire and process images of the microfluidic chip to detect and quantify the expression of a detectable marker, such as green fluorescent protein (GFP); then the GFP signal for each strain was calibrated to a concentration of the on-target analyte of interest, e.g., a contaminant. In this manner, a panel of microbial biosensor strains, e.g., *E. coli* biosensor strains, can be employed to continuously monitor a process water for a variety of analytes in the aqueous samples. Optionally, the marker signal data can be gathered and transmitted to an operator or database in real time.

A major challenge to implementing microfluidic technology lies in developing suitable interfaces with macro-scale flow systems. In some embodiments of the invention, we generated flow through the microfluidic chip using a combination of peristaltic pumps and sealed reservoirs with pressurized headspaces. For drawing the process water into the chip from a source external to the sensor enclosure, we used a commercially available 8-roller peristaltic pump (e.g., Bio-Chem Fluidics, Boonton, NJ, Product # PE-WX18-S21-1), with appropriate chemically inert small-bore tubing (e.g., PharMed® BPT, 0.02" ID, 0.09" OD; Saint-Gobain Performance Plastics Corporation) to minimize pulsatility in the flow stream. For driving the flow of reference water and reagents housed in the microfluidic chip enclosure, we stored the liquids in gas-sealed bottles with ported caps. Tubing was threaded through each cap port to access the bottle gas headspace (for gas flow in) and the bottle liquid contents (for reference water and reagent flow out). A commercially available electro-pneumatic air pressure regulator (e.g., SMC, Product # ITV1010-211L4-X26) was used to control the headspace pressure in each bottle, whereby increased headspace pressure corresponded to increased liquid flow out of the bottle into the chip. A tank of compressed dinitrogen gas was used to supply chemically-inert headspace pressure.

In some embodiments, commercially available small-bore tubing (e.g. PFA, 0.02" ID, 1/16" OD, IDEX Health & Science #1512L; or Tygon® formulation ND-100-80, 0.02" ID, 0.06" OD, VWR #MFLX06419-01, Saint-Gobain Performance Plastics Corporation) was used to connect additional fluidic components between the sources of process water, reference water, and reagents and the chip without introducing significant dead volume. For example, a PEEK filter assembly with 0.5-$\mu$m pore size (IDEX Health & Science #A-356) was connected inline to remove small particles that could clog the chip microchannels. A non-metallic check valve (IDEX Health & Science #CV-3321) was connected inline to maintain forward flow. A passive (e.g., Omnifit® Bubble Trap, PEEK with 10-$\mu$m PTFE filter, Diba #006BT, Diba Industries Limited) or active (e.g., 2-Channel OEM Mini Degassing Module, IDEX Health & Science # 0001-6274) degasser was connected inline to each flow stream to remove gas bubbles. Valving (e.g., either a rotary valve (e.g., TitanEX® 2 Position/6 Port valve with control PCB, IDEX Health & Science #MLP777-601 valve with #7770-067 PCB) or a custom manifold incorporating 2-way solenoid valves (as shown in Figure 8; e.g., LVM Series 3-Port Solenoid Valve with Power Saving Circuit, SMC #LVM095RY-5C-KOZ)) was connected in-line with the process water and reference water flow streams to alternate their flows into the paired inlet ports of the chip. Finally, the flow rate of each fluid into the chip was measured by connecting a liquid flow meter (e.g., Sension® # LG16-0431D, 0-80 $\mu$L/min, Sensirion AG) in-line.

In some embodiments, the flow rate at each chip inlet was maintained at a target value by implementing a standard PID feedback control scheme. Here, the flow rate into the chip (i.e. "process value") as measured by a liquid flow meter was compared to the target flow rate ("set point"). Deviations between these values were used in conjunction with $K_p$, $K_i$, and $K_d$ constants to calculate correction terms to be applied to the pressure source driving the flow (i.e., "control variable," represented by either peristaltic pump rate or bottle headspace pressure). For controlling flows into the inlet ports of the chip, for example as shown in Figure 5B, we used a peristaltic pump to flow process water into a (first) inlet port (515) and a bottle with pressurized headspace to flow reference water into the other (second) inlet port (515). We used another bottle with pressurized headspace to flow 5X concentrated bacterial growth medium into the combination media inlet port and media junction (517/540). We used PID control to establish 1X process water flow at 20 mL/day, 1X reference water flow at 20 mL/day, and 5X concentrated growth medium flow at 10 mL/day. At these inlet flow rates, the concentrations of process water, reference water, and growth medium at the microbial process and reference monitoring arrays (110, 120) downstream of on-chip mixing at the mixing junctions (550) and mixing channels (560) were 0.8X, 0.8X, and 1X, respectively.

In an embodiment where the recombinant microbial strains in the chip produce a fluorescent protein reporter as the detectable marker in response to exposure to on-target water analytes of interest (e.g. contaminants), the fluorescent protein reporter can be a green fluorescent protein (GFP) variant, such as superfolder GFP (sfGFP). Cellular production of sfGFP can be measured by illuminating the cells with blue light (nominally 485-nm wavelength) and measuring the production of green light (nominally 510-nm wavelength). An appropriate sfGFP illumination source can comprise a blue light-emitting diode (LED) (e.g., XLamp® XP-E2 Blue, Cree LED #XPEBBL-L1-0000- 00301) and an excitation filter (e.g., 482/18 nm BrightLine® single-band bandpass filter, Semrock® #FF02-482/18). Additional spectrally-compatible fluorescent protein or dye readouts can be imaged with the use of a multi-band emission filter. For example, cellular production of the mKate2 red fluorescent protein can be imaged by illuminating the cells with an amber light-emitting diode (LED) (e.g., XLamp® XP-E2 Amber, Cree LED #XPEBAM-L1-0000-00901) through an excitation filter (e.g., 585/40 nm BrightLine® single-band bandpass filter, Semrock® #FF01-585/40). In addition to fluorescent light imaging, a transmitted light image of the cells within the chip can be acquired by illuminating with a green LED (e.g., XLamp® XP-E2 Green, Cree LED #XPEBGR-L1-0000- 00A01). To distinguish detectable markers or reporters using multiple imaging channels, emitted fluorescence and transmitted green light should pass through a compatible emission filter (e.g., for this configuration, 527/645 nm BrightLine® dual-band bandpass filter, Semrock® #FF01-527/645). Monochrome images in each spectral channel can be sequentially acquired by a CCD or CMOS camera (e.g., Blackfly® S Mono 1.6 MP GigE Vision, FLIR #BFS-PGE-16S2M-CS). Sets of spectral images can be acquired periodically (e.g. every 5 minutes) over days or weeks to track the dynamics of fluorescent protein production (correlating to analyte concentration) over time.

Experimental noise in image datasets can be minimized by employing tight temporal control of light-emitting diode (LED) switching, LED warm-up, and camera triggering. For long-term continuous sensing, illumination should be stable over multi-day timescales and large temperature swings (>10°C). LED driver circuit design should be constant-current and adjustable, such any variation in LED illumination with time or temperature can be compensated for in hardware, based on known calibration curves.

In some embodiments, custom electronics were developed to power and control all fluidic, optic, and thermal systems. Custom Printed Circuit Board Assemblies (PCBAs) were designed to electrically interface with the hardware components of the microfluidic chip sensor system. Fluidic hardware components with electronic interfaces included peristaltic pumps, electro-pneumatic air pressure regulators, air pressure sensors, active degassers, valves (e.g. solenoid or rotary), and liquid flow meters. Optic hardware components with electronic interfaces included illumination sources (e.g., light-emitting diodes (LEDs)) and cameras. Thermal hardware components with electronic interfaces included resistive heaters, compressor-based coolers, thermoelectric modules, thermistors, thermocouples, and fans.

Custom firmware was developed to run on the microprocessors of the custom PCBAs to orchestrate control of the hardware components to construct a functional sensor. The custom firmware was written to receive operational commands from a connected System on a Chip (SoC) computer using the Modbus RTU protocol, and a graphical user interface (GUI) was developed in HTML to run on the SoC. In this configuration, the user of the inventive microfluidic chip can connect via a computer interface (e.g., via a desktop or laptop computer, a cellular phone, a tablet, or the like) to the SoC over Wi-Fi, Bluetooth, or a similar communication protocol, within a web browser application, to send commands to the custom PCBAs to control the biosensor operation of the microfluidic chip remotely, if desired.

The E. coli biosensor strains were loaded into predetermined addressable locations (i.e., the cell trap complexes) arrayed in the microfluidic device, or "chip." Briefly, an "Alternating Conditions" ("AC") sensor mode of operation was used, by which cells were alternatingly exposed to 2-hour pulses of water containing the analyte (termed process water), followed by 2-hour pulses of pure water (representing a negative control baseline response). In an example of one such experiment, in which an ammonium sensing glnA strain was subjected to 2-hour on-off pulses of ammonium spanning 0-7 ppm, the raw fluorescence signal was band-pass filtered and normalized relative to the mean to produce a processed fluorescence signal. Sequential "AC" step inductions for three iron-responsive strains were also identified by screening an E. coli fluorescent promoter library as described in PCT/US2024/025712. The E. coli strains codB and fes were sensitive to iron concentrations as low as 10 ppb, whereas the LOD for strain ugpB was around 50 ppb. Arraying multiple strains, with various sensitivities within a sensing array of the microfluidic chip allowed us to expand our sensing range. For example, the responses of strains mntP and mntP-mntH(KO) saturate at 1000 ppb Mn(II); strain mntP+-mntH(KO) extended the useful dynamic range upwards for sensing up to 3000 ppb Mn(II).

In another experiment, a nickel dication-sensitive rcnAB recombinant E. coli strain responded to nickel dication in the microfluidic chip embodiment illustrated in Figure 5B, as we probed the strain detection limit. We loaded rcnAB into all microbial process monitoring arrays and microbial reference monitoring arrays and booted the chip on LB medium. After transitioning to HM9 medium, we monitored strain fluorescence at all positions while we alternated exposure between pure water (our "reference water" stream) and pure water spiked with various concentrations of Ni(II) (our "process water" stream) every 2 h (see "Alternating Conditions" sensor mode of operation). The Ni(II) concentration in the process water was spiked to 122 ppb and then stepped down to 60, 30, and 15 ppb every 24 h (data not shown). At 15 ppb the response became indistinguishable from 0; therefore our detection limit is approximately 30 ppb. We subsequently spiked to 972 ppb, likely saturating the response of the genetic regulatory circuit.

We subsequently performed a fast Fourier transform (FFT) with 4 h period (i.e., one complete "AC" cycle) on the strain response signals to calculate the oscillation amplitude during these AC inductions (i.e., the distance between the minimum and maximum values for each on-off cycle), which can be calibrated to analyte concentration. Several biosensing strains were characterized or calibrated in this way, with each strain subjected to at least three "AC" inductions at a minimum of three concentrations of the target analyte.

For all the tested microbial strains, the amplitude of the fluorescence response to "AC" inductions increased with analyte concentration, although not always linearly. Laboratory calibration data can alternatively be presented as sequential "AC" inductions, where analyte concentration steps up and down. The amplitude of the fluorescence strain response correspondingly increases and decreases at each step in analyte concentration. To calibrate each microbial biosensor strain, we performed a regression analysis between the magnitude of the strain amplitude and the analyte concentration.

Example 3. "Real-world" trial of the inventive microfluidic chip with engineered *E. coli* strain in a commercial algae pond

To evaluate the performance of our microfluidic chip and biosensor strains outside the laboratory, we deployed a hardware prototype to a prominent algae research facility in Mesa, Arizona, as described in Cookson et al., PCT/US2024/025712. The optimal growth conditions for algae biomass production were studied in 1,100-liter outdoor raceway ponds in the challenging desert climate of the southwestern United States of America, where ambient temperature can exceed 115°F. We enclosed a microfluidic device designed to monitor analytes in up to four process water sources, optionally different from one another or the same (e.g., as illustrated in Figure 1), and loaded with our *E. coli* sensing strain glnA in all microbial process and reference monitoring arrays (110 and 120) within a robust hardware package designed to orchestrate all sensor operations, including imaging, valving, flow control, temperature control, and remote communications. (See, e.g., Hasty et al., "Microbial Microfluidic Biosensor," US11209412B2). We constructed a recirculation loop with peristaltic metering pump, mesh strainers, and 500-kDa tangential flow filter (TFF) to rapidly deliver fresh filtered pond culture to our sensor hardware and return the unused majority fraction to the pond, thereby minimizing sensing delay. Continuous real-time field data were gathered for outdoor algae culturing throughout two pond growth experiments each spanning 22 days. The pond was inoculated with *Scenedesmus obliquus* (UTEX 393) culture in "brackish" (5 parts-per-thousand salinity) BG-11 medium and grew denser as it consumed available nutrients. To maintain sunlight penetration and rapid growth, algae biomass was periodically partially harvested from the pond, and fresh BG-11 medium was added to supply fresh nutrients. Alternatively, the culture was dosed with fresh BG-11 medium without biomass harvesting. Raw fluorescence response of the ammonium-sensitive glnA engineered *E. coli* strain to changing ammonium concentration in the managed pond culture was calibrated to triplicate pond grab samples analyzed by Hach ammonium test kits. Biosensor ammonium measurements agreed with Hach measurements well within the $\pm 15\%$ accuracy window deemed acceptable by site management. These long-term, continuous, and quantitative analyte data demonstrate the effectiveness of the inventive microfluidic chip platform for the culture of *E. coli* biosensor strains in a commercial biomass production application. We noted diurnal oscillations in our $NH_4^+$ measurements, which may be explainable in view of the particular embodiment of the microfluidic biosensor hardware employed; with this embodiment of the hardware we continuously actively degassed the inlet stream of filtered pond culture before diluting it with a custom additive and pumping it to the microfluidic chip. We found that the sensed $NH_4^+$ concentration diurnally dipped at mid-day, when both temperature and photosynthetic productivity peaked. Because $NH_4^+$ solubility decreases at higher temperatures, $NH_4^+$ outgassing at elevated temperature and vacuum degasser pressure could explain our observations. Additionally, or alternatively, high algae productivity generates high dissolved oxygen (DO) levels that could have "scrubbed" $NH_4^+$ from solution during continuous degassing. Whatever the cause, we subsequently improved the microfluidic chip hardware by 1) adding temperature control to stabilize $NH_4^+$ solubility, and by 2) only briefly degassing the diluted aqueous culture medium further downstream in transit to the microfluidic chip. These hardware improvements eliminated diurnal oscillations in our $NH_4^+$ measurements.

Example 4. Panel of recombinant *Escherichia coli* biosensor strains incorporated into the inventive microfluidic chip for analyte assays

The inventive microfluidic chip can be used to house recombinant microbial biosensors strains, e.g., *Escherichia coli* biosensor strains (see, e.g., Cookson et al., PCT/US2024/025712), or other microbial biosensor strains, to operate as an assay of analytes when incorporated into the inventive microfluidic chip, designed to sample a process water, which is mixed and diluted with concentrated defined culture medium. Previous examples show how some *E. coli* strains in the microfluidic platform are useful to detect and quantify a signal coming from the expression of a detectable marker in response to the presence of the analyte of interest. For example, in some embodiments, fluidic, electronic, and optical systems can be designed to continuously draw and mix process water with a concentrated defined culture medium within the inventive microfluidic chip housing the cells and periodically acquire and process images of the microfluidic chip to detect and quantify the expression of a detectable marker, such as GFP; then the GFP signal for each strain can be calibrated to a concentration of the on-target analyte of interest, e.g., a contaminant. In this manner, a panel of *E. coli* biosensor strains on the microfluidic chip can be employed to continuously monitor a process water for a variety of analytes in the aqueous sample. Optionally, the marker signal data can be gathered and transmitted to an external operator or database in real time.

The microfluidic chip contains predetermined addressable locations (i.e., the cell spotting regions of the cell trap complexes) in which *E. coli* biosensor strains can be loaded during its manufacture, or post-manufacture. *E. coli* culture biomass can be transferred from an agar plate or liquid culture into an array of spotting "reservoirs" in the device, either individually and sequentially by hand or in parallel by pin tool. For example, the pin tool can be automated to robotically select and align the source and target colony arrays, retrieve biomass from the source array, and deposit it upon the target array. Following "loading" of the spotting reservoirs in the microfluidic device, strain viability can be preserved either by air drying or freeze drying. For air drying, the colonies are allowed to dry naturally through evaporation. For freeze drying, the hardware device (e.g., microfluidic chips) are frozen, and vacuum pressure is applied to sublimate water vapor. For example, the microfluidic chip monolith containing the loaded spotting reservoirs can be sealed against a flat surface to form the microfluidic channels using various methods; both chip halves can be treated with oxygen plasma to activate the surfaces before spotting and then brought into contact to form a covalent bond. Alternatively, a pressure-sensitive adhesive can be applied to one chip half and then compressed against the other to rupture adhesive vesicles at the contacting surfaces but not along the open microfluidic channels.

The microfluidic device typically maintains *E. coli* viability when shielded from light at refrigerator temperature (4°C) to room temperature (20°C) for at least 30 days. When the microfluidic chip is booted in preparation for sensing, rich medium (e.g. Lysogeny Broth (LB)) is flowed into the chip to mix with the process water at a predetermined defined dilution ratio and hydrates the arrayed spotting reservoirs. Strains spotted within each reservoir grow to confluence throughout the following 12-24 hours, with excess culture overflowing each reservoir and washing downstream to an off-chip waste receptacle. After revival of the dehydrated strains, the culture medium can be swapped to a well-defined minimal medium (e.g. M9 or HM9) to ensure the purity of the medium stream and slow the growth rate of the sensing strains. (See, e.g., Hasty et al., "Microbial Microfluidic Biosensor," US11209412B2.)

Culturing conditions within the microfluidic chip can be maintained to ensure that each strain signal has a stable baseline. Flow rates of process water and concentrated defined culture medium can be controlled by continuously measuring them using a flow meter (e.g., sensing range can be 0-80 $\mu$l/min) and using a proportional-integral-derivative (PID) feedback loop to adjust driving pressure (e.g., headspace pressure in a closed fluidic vessel, force applied to a syringe pump, or meniscus elevation generating hydrostatic pressure). Culture temperature can be maintained, preferably at 37°C, by measuring the temperature near the microfluidic chip using a probe (e.g. thermistor or thermocouple) and using a PID feedback loop to adjust output from a temperature control system (e.g. thermoelectric (Peltier) module, resistive heater, or compressor-based cooler). Culture pH can be maintained by including a buffer (e.g. MES, MOPS, HEPES, or PIPES) in the concentrated growth medium. With proper culturing conditions maintained, cellular responses can be continually measured within the microfluidic device for up to a few months.

Fluid flow throughout the microfluidic chip is driven by establishing pressure differentials across the first and second inlet ports (150, 160, 415, 515), the media inlet ports (417, 517), and the downstream waste port(s) (140, 470, 570). These pressure differentials can be applied using several methods, including pressurizing the headspace of a closed fluidic vessel with dip tube outlet, applying force to a syringe pump, or elevating an open reservoir to generate hydrostatic pressure. All of these methods are preferable to positive-displacement pump styles (e.g. rotary, piston, diaphragm, peristaltic) in that the resulting fluid flow is non-pulsatile. Macro-scale pressure pulses typically generate large flow waves within microfluidic devices that can disrupt colony stability, thereby increasing experimental noise.

In one useful embodiment, a microfluidic flow control system employs compressed gas and electro-pneumatic regulators to pressurize the headspaces of sealed fluidic vessels with dip tubes. Such a system can produce controlled non-pulsatile flows by adjusting headspace pressure based on feedback from a PID control loop and microfluidic flow meter. In some embodiments, electropneumatic regulators and peristaltic pumps can be combined to mix media and reference water flow streams from pressurized vessels with a process water flow stream delivered from an open vessel by peristaltic pump. The set points of multiple PID control loops can be configured to achieve proper mixing ratios of the flow streams within the microfluidic device. Figure 8 illustrates the fluidic schematic for a hardware assembly configuration appropriate for driving flows through the microfluidic chip, such as the embodiment shown in Figure 1. This embodiment of the chip is designed to sense analytes in four process waters, which may each be different from one another or the same in any combination, using four chip lanes with culture media pre-mixed off-chip. In this embodiment, four process waters were drawn from a source external to the microfluidic chip sensor system by peristaltic pump. In parallel, flows of four reference waters were driven from sealed bottles by applying headspace pressure. Each flow passed through a 0.5-micron filter to remove any unexpected particulates, a liquid flow meter was used to measure flow, an active or passive degasser was employed to remove any bubbles in the flow stream, and a check valve was used to ensure forward flow. Each pair of process and reference waters then passed through a pair of valves that were activated out of phase; for example, in Figure 8, process water and reference waters are directed to opposite halves of a chip lane by a pair of 2-way valves (each labeled "2V" and having two inlets and one outlet, where the activation state of the valve determines which inlet flows to the outlet). At each point in time, one 2-way valve is not activated (e.g., thereby opening the fluidic path for process water connected to the left inlet to flow through to the valve outlet) and the other 2-way valve is activated (e.g., thereby opening the fluidic path for reference water connected to the right inlet to flow through to the valve outlet). The activation states of the valves switch in synchrony to enable operation in "Alternating Conditions" (AC) mode, whereby the process and reference water flows are redirected to opposite microbial strain arrays within a single chip lane upon each valve switch. After flowing through both halves of all four chip lanes, the strain array effluents merged into a central waste outlet channel, which conveyed fluid flow to a downstream waste port connected to an optional waste reservoir for collection, or to a free exhaust of the waste fluid.

Using an "AC" sensor mode of operation enabled by the microfluidic chip, the fluidic input to the sensing *E. coli,* or another microbial biosensor strain panel in the inventive microfluidic device, can be alternated between pulses of: 1) "process" water containing an unknown concentration of the target analyte, and 2) "reference" water with a similar background composition to the process water but not containing the analyte of interest. In practice, a microfluidic chip design can be fluidically split into replicate arrays of sensing recombinant *E. coli* strains, and these process and reference water streams can be periodically alternated between them using automated valving. When measuring responses from transcriptional reporters with timescales of tens of minutes, the "AC" pulse period can vary from around 1 to 8 hours, with a nominal value of around 4 hours, typically. The "AC" mode is essentially a series of precisely timed inductions, where cells do not return to baseline levels of fluorescence. This leads to periodic modulation of cell fluorescence, where the oscillation amplitude can be calculated and calibrated to analyte concentration using a number of methods.

The following useful method of calculating the instantaneous oscillation amplitude, $G_{AC}$, can be easily implemented in both traditional computing environments and web browser environments. For each engineered recombinant *E. coli* biosensor strain, we first calculate either the mean or median fluorescence response across replicate observation regions in the microfluidic device, where response in this case is band pass filtered response. This response leads to signal $g_n$ at a discrete set of time points $t_n$, with n being an integer that labels the time points. We then perform a least-square regression for $g_n$ using a function $f_n$ with unknown fitting parameters A, B, and C, according to Equation IV (below).

Equation IV:

$$f_n = A + B \sin(\omega t_n) + C \cos(\omega t_n)$$

where $\omega = 2\pi/T$ is the natural frequency for period $T$ (a specified parameter). When calculating the amplitude response at time t, we perform this regression for time points $t_n$ satisfying $t - t_H < t_n \leq t$ for history duration $t_H$ (a second specified parameter). The required least-square regression is then a standard calculation that depends on straightforward linear algebra. Application of this process leads to time-dependent fitting parameters A(t), B(t), and C(t) that can be used to quantify the baseline, phase, and amplitude of response. In particular, we define the oscillation amplitude $G_{AC}$ according to Equation V (below).

Equation V:

$$G_{AC}(t) = [B(t)^2 + C(t)^2]^{1/2}$$

The calculation of $G_{AC}$ in this manner is rather fast and is readily generalized to other regression methods if desired.

There are two primary parameters that must be specified to calculate $G_{AC}$. The period parameter T determines the frequency of cellular response that is to be sampled, and T should almost always be set to the experimental drive period used in "AC" mode. The history parameter $t_H$ determines how many time points are to be used in the regression. Very short $t_H$ integrates over very little data and is susceptible to noise, while very long $t_H$ may miss important time-dependent features of analyte concentration. We have found empirically that values $t_H = T$ and $t_H = 1.5T$ produce reasonably time-localized and low-fluctuation amplitude response.

As a final step in quantifying measurements of strain fluorescence response, the strain response amplitude, $G_{AC}$, can be calibrated to the concentration of the target analyte. The "AC" response amplitude can be fit to the induction concentrations to generate a calibration curve, as previously described in Cookson et al., PCT/US2024/025712. Given this calibration curve, the strain response during a future induction with process water containing unknown analyte concentration can be mapped to an analyte concentration.

The previous embodiment of the Alternating Conditions (AC) sensor mode of operation for the sensing recombinant *E. coli* strain panel is particularly employed to measure analytes in a relatively pure "process" water stream (e.g., laboratory water containing an unknown concentration of the target analyte of interest). However, most biosensing applications of interest will involve quantitatively measuring analyte concentrations in an environmental or industrial process water stream (e.g., groundwater, surface water, wastewater). In these scenarios, the composition of the "reference" water stream used in the "AC" analysis technique is matched to the background composition of the process water stream but omits the target analyte to be sensed. This decreases noise in the strain response by reducing biological artifacts due to sudden shifts in extracellular pH, osmolarity, and ionic strength. Ideally, a formulation for the reference water should be optimized such that several key water quality parameters match the process water background. Example key water quality parameters include pH, bicarbonate, carbonate, total organic carbon (TOC), alkalinity, hardness, calcium, chloride, magnesium, manganese, ammonium, nitrate, nitrite, potassium, sodium, and sulfate.

An important application of the inventive microfluidic chip with a panel of recombinant *Escherichia coli* strains is for sensing nutrient loads in impure water streams. Such process waters can include high nutrient loads in wastewaters or agricultural surface waters as well as nutrients added to culture media in closed or open bioreactors used to produce biomass and other biological products. In the case of algal biomass production in photobioreactors, nutrients are added to algal growth media, and their concentrations are depleted as the culture density increases. The biosensor panel of recombinant *E. coli* strains can also be used to inform the periodic dosing of nutrients to optimize biomass or bioproduct production.

When using the inventive microfluidic chip with a biosensing panel of *E. coli* or other microbial biosensor strains to quantify nutrient concentrations in algal cultures, the "reference" water stream used in the "AC" analysis technique should be matched to the algal growth medium, while omitting the sensing target analyte. Common algal defined growth media used in commercial production include (in order of increasing salinity) Zarrouk's Medium, BG-11 Medium, and F/2 Medium.

## Claims

1.     A microfluidic chip (100) for parallel culture of recombinant biosensing microbes, comprising, encompassed by a chip solid matrix (796):

(a) one or more paired microbial process monitoring arrays (110) and microbial reference monitoring arrays (120), each reference monitoring array corresponding to one of the process monitoring array(s), each process monitoring array (110) and each reference monitoring array (120) comprising an upstream end and a downstream end, and further comprising:

(i) an upstream array inlet channel (132, 432, 532), fluidly connected and upstream to one or more upstream manifold channels (200, 402, 502, 602), and thence fluidly connected to:
(ii) a network of branch channels (210, 310, 410, 510, 610), branching in parallel within the process monitoring array(s) (110) and reference monitoring array(s) (120), wherein a cell trap complex (130, 230, 430, 530, 630) is in fluid connection to each branch channel (210, 310, 410, 510, 610), and is capable of receiving fresh liquid cell culture medium from the branch channel; and wherein each branch channel is fluidly connected via a downstream manifold channel (220, 403, 503, 603), which is configured to receive cell-conditioned medium outflow from the branch channel (210, 310, 410, 510, 610), wherein the downstream manifold channels are configured to convey the cell-conditioned medium outflow further downstream to:
(iii) a downstream array outlet channel (135, 235, 435, 535), which is configured to fluidly convey the cell-conditioned medium outflow;

(b) for conveying liquid to the process monitoring array(s) (110), a first inlet port (150, 415, 515), fluidly connected via a first resistance-tuned inlet channel (155), upstream to the first upstream array inlet channel (132, 432, 532), which is fluidly connected to the process monitoring array (110);
(c) for conveying liquid to the reference monitoring array(s) (120), a second inlet port (160, 415, 515), fluidly connected via a second resistance-tuned inlet channel (165), upstream to the second upstream array inlet channel (132, 432, 532), which is fluidly connected to the reference monitoring array (120); and
(d) a waste outlet channel (138, 438, 538), which is configured to fluidly receive the cell-conditioned medium outflow from the downstream array outlet channels (135, 235, 435, 535) and to fluidly convey the outflow to a downstream waste port (140, 470, 570).

2.     The microfluidic chip of Claim 1, wherein each cell trap complex (130, 230, 430, 530, 630) comprises:

(i) a cell spotting region (300, 600);
(ii) an adjacent primary cell trap (312, 612), fluidly connected to the cell spotting region by a first migration channel (320, 620) having a broader end (322) and a narrower end (324), wherein the first migration channel tapers from the cell spotting region (300, 600) at the broader end to the primary cell trap (312, 612) at the narrower end, each primary cell trap having a side distal from the cell spotting region open to an adjacent microfluidic branch channel (210, 310, 410, 510, 610);

(iii) a second migration channel (332, 632), fluidly branching from the narrower end (324) of the tapering first migration channel; and

(iv) a plurality of downstream cell traps (340, 640) in a series adjacent to the primary cell trap, each downstream cell trap having a side with an opening to the second migration channel (332, 632) and having a side opposite the second migration channel open to the adjacent microfluidic branch channel (210, 310, 410, 510, 610), each downstream cell trap being capable of housing a viable microbial colony.

3. The microfluidic chip according to Claim 2, wherein the primary cell trap(s) (312, 612) and the downstream cell traps (340, 640) have a transparent ceiling to enable light emissions to be imaged by an external detector (180).

4. The microfluidic chip according to any one of Claims 2-3, wherein the cell spotting regions of the parallel cell trap complexes are configured to be compatible with a pin tool for delivery of microbial cells.

5. The microfluidic chip according to any one of Claims 1-4, wherein the first inlet port (150, 415, 515) and the second inlet port (160, 415, 515) are each configured to alternatively receive, at a predetermined pressure (P) at a flow rate (Q), either of:

(i) process water,
or
(ii) reference water,

and wherein the first inlet port (150, 415, 515) and the second inlet port (160, 415, 515) are each configured to fluidly convey the received water to the first resistance-tuned inlet channel (155) or to the second resistance-tuned inlet channel (165), respectively.

6. The microfluidic chip according to any one of Claims 1-5, wherein the first resistance-tuned inlet channel (155) and the second resistance-tuned inlet channel (165), each further comprise:

(a) a primary water inlet channel (416, 516), fluidly connected and upstream to:
(b) a separate mixing junction (450, 550), wherein each mixing junction is fluidly connected and upstream to:
(c) a mixing channel (460, 560), which is fluidly connected and upstream to one of the plurality of the upstream array inlet channels (132, 432, 532), each of which is fluidly connected and upstream to one of the microbial process monitoring arrays (110) or one of the microbial reference monitoring arrays (120);

whereby the chip is configured for on-chip mixing of the process water and the reference water, with one or more concentrated defined liquid cell culture media.

7. The microfluidic chip according to Claim 6, wherein the first resistance-tuned inlet channel (155) and the second resistance-tuned inlet channel (165), each further comprise, fluidly connected and downstream to the primary water inlet channel (416, 516):

(d) a water junction (400, 500), fluidly connected and upstream to:
(e) more than one post-water junction water channels (420, 520), each of which is fluidly connected and upstream to the separate mixing junction of Claim 6, subpart (b) (450, 550); wherein the water junction is configured to split the flow of liquid to the more than one post-water junction water channel(s).

8. The microfluidic chip according to any one of Claims 6-7, comprising:

(f) one or more media inlet ports (417, 517), each media inlet port being configured for fluidly receiving a single concentrated defined liquid cell culture medium from a source outside the chip, and being upstream and fluidly connected to:

(g) a primary media inlet channel (455, 555, 655), each primary media inlet channel being upstream and fluidly connected to:

(h) a media junction (440, 540), being upstream and fluidly connected to:

(i) a plurality of post-media junction media channels (425, 525, 625), each being upstream and fluidly connected to one of the separate mixing junctions of Claim 6, subpart (b) (450, 550).

9. The microfluidic chip according to any one of Claims 3-8, wherein the external detector (180) is configured to monitor the primary cell trap(s) (312, 612) and the downstream cell traps (340, 640) in each process monitoring array (110) and each reference monitoring array (120).

10. The microfluidic chip according to any one of Claims 1-9, comprising a light emission calibration channel (427, 527, 627).

11. The microfluidic chip according to any one of Claims 1-10, comprising a plurality of data matrices (195, 495, 595, 795) fabricated into an external surface of the chip solid matrix (796), for orientation of an image of light emissions to be collected from the paired microbial process monitoring arrays (110) and microbial reference monitoring arrays (120).

12. The microfluidic chip according to Claim 11, wherein the plurality of data matrices (195, 495, 595, 795) is configured to register the image of light emissions by affine transformation.

FIG. 1

**Zoom of One Process or Reference Monitoring Array**

▱ 4 μm, Cell Trap Complexes

▨ 50 μm Branch Channels

▩ 150 μm Manifold Channels

**FIG. 2**

EP 4 711 442 A1

230

## Cell Trap Complex

FIG. 3A

## i) Cells Deposited in Spotting Region

Cells

ΓOA

Nutrient Diffusion

Laminar Flow of Growth Medium

## ii) Dividing Cells Push Each Other Towards Trap Opening

ΓOA

Cell Growth

## iii) Cells Fill Downstream Cell Traps

ΓOA

## iv) Cell Traps Monitored Until Experiment End

ΓOA

Cell Debris Continuously Washed Away

## FIG. 3B

**FIG. 3C**

Solid Matrix

Fresh medium

Nutrient deprived

Nutrient rich

Heterogeneous starved/rich cells

FIG. 4A

**FIG. 4B**

427

ii

Media

To top
array

425

440

To
bottom
array

425

**FIG. 4C**

iii

To middle array

420

420

400

To left
array

To right
array

416

415

Water

**FIG. 4D**

FIG. 5A

FIG. 5B

**FIG. 6A**

FIG. 6B

EP 4 711 442 A1

FIG. 7A

FIG. 7B

Clear

Mask

Textured Transparent Zone

EP 4 711 442 A1

**FIG. 8**

# FIG. 9A

a   Old Woman Creek water (PPM units)

# FIG. 9B

b   Old Woman Creek water (mM units)

# EP 4 711 442 A1

## EUROPEAN SEARCH REPORT

**Application Number**

EP 25 19 7101

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHU ZHEN ET AL: "A microfluidic single-cell array for in situ laminar-flow-based comparative culturing of budding yeast cells", TALANTA, ELSEVIER, AMSTERDAM, NL, vol. 231, 20 April 2021 (2021-04-20), XP086566221, ISSN: 0039-9140, DOI: 10.1016/J.TALANTA.2021.122401 [retrieved on 2021-04-20] * the whole document * | 1-12 | INV.<br>C12M3/06<br>C12M1/00 |
| A | US 2018/149633 A1 (HASTY JEFF [US] ET AL) 31 May 2018 (2018-05-31) * paragraphs [0062], [0066], [0069], [0199] - [0342]; claims 1, 34, 43, 58; figures 5A, 5B, 6A-6C, 7A-7G, 50, 54, 57 * | 1-12 | |
| A | US 2024/247300 A1 (HAN ARUM [US] ET AL) 25 July 2024 (2024-07-25) * paragraphs [0039] - [0052]; claims 22, 25-27, 30-34; figures 1, 2A-2C, 3, 4A-4D, 8 * | 1-12 | **TECHNICAL FIELDS SEARCHED (IPC)**<br><br>C12M |
| A | WO 2013/090818 A1 (UNIV CALIFORNIA [US]; PRINDLE ARTHUR [US] ET AL.) 20 June 2013 (2013-06-20) * paragraphs [0007], [0032], [0104]; claims 1, 50-55, 145; figures 3A, 3B * | 1-12 | |

The present search report has been drawn up for all claims

| Place of search | Date of completion of the search | Examiner |
|---|---|---|
| The Hague | 28 January 2026 | González Ferreiro, M |

EPO FORM 1503 03.82 (P04C01)

## ANNEX TO THE EUROPEAN SEARCH REPORT
## ON EUROPEAN PATENT APPLICATION NO.

EP 25 19 7101

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2026

| Patent document cited in search report | Publication date | Patent family member(s) | | Publication date |
|---|---|---|---|---|
| US 2018149633 A1 | 31-05-2018 | CN | 107636160 A | 26-01-2018 |
| | | EP | 3259366 A1 | 27-12-2017 |
| | | EP | 4071252 A1 | 12-10-2022 |
| | | US | 2018149633 A1 | 31-05-2018 |
| | | US | 2022057378 A1 | 24-02-2022 |
| | | WO | 2016133830 A1 | 25-08-2016 |
| | | ZA | 201705584 B | 23-02-2022 |
| US 2024247300 A1 | 25-07-2024 | EP | 4337260 A1 | 20-03-2024 |
| | | US | 2024247300 A1 | 25-07-2024 |
| | | WO | 2022241202 A1 | 17-11-2022 |
| WO 2013090818 A1 | 20-06-2013 | US | 2015133339 A1 | 14-05-2015 |
| | | WO | 2013090818 A1 | 20-06-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 11209412 B2, Hasty
- US 2022057378 A1
- US 8389263 B2, Daunert et al.
- US 20100304379 A1
- US 2024025712 W, Cookson
- US 20090176269 A, Giovagnoli
- US 20160244506 A
- US 9359629 B
- EP 2235197 A
- EP 2574676 A
- US 5302697 A, Goodey, Andrew R
- US 6022952 A
- US 6335178 B
- US 2018149633 A1, Hasty
- WO 2016133830 A1, Hasty
- WO 2014093661 A2, , Zhang
- US 9228208 B2, Frendewey
- WO 2008052101 A2, Church
- US 8153432 B2

- US 20150079680 A1, Bradley
- WO 2017141173 A2, Begemann
- US 9982279 B1, Gill
- US 9428767 B2, Minshull
- US 9580697 B2
- US 9574209 B2
- US 10041077 B2, Minshull
- US 11028410 B2, McGrew
- US 11098310 B2, McGrew
- US 20190185881 A1, McGrew
- US 4767704 A
- US 4657866 A
- US 4927762 A
- US 4560655 A
- US 5122469 A
- WO 90103430 A
- WO 8700195 A
- US 8119368 B, Zijlstra
- US 8222001 B
- US 8440458 B
- US 8206981 B, Crowley

### Non-patent literature cited in the description

- **MUELLER et al.** The widespread and unjust drinking water and clean water crisis in the United States. *Nature Commun.*, 2021, vol. 12, 3544
- **ALLAIRE et al.** National trends in drinking water quality violations. *PNAS*, 2018, vol. 115 (9), 2078-2083
- **CHISLOCK, M. F.** ; **DOSTER, E** ; **ZITOMER, R. A.** ; **WILSON, A. E.** Eutrophication: Causes, Consequences, and Controls in Aquatic Ecosystems. *Nature Education Knowledge*, 2013, vol. 4 (4), 10
- **DODDS et al.** Eutrophication of U.S. Freshwaters: Analysis of Potential Economic Damages. *Environ. Sci. & Technol.*, 2009, vol. 43 (1)
- **CARDEMIL, C. et al.** Bioluminescent Escherichia coli strains for the quantitative detection of phosphate and ammonia in coastal and suburban watersheds. *DNA Cell Biol*, 2010, vol. 29 (9), 519-31
- **DEANGELIS et al.** Two novel bacterial biosensors for detection of nitrate availability in the rhizosphere. *Appl. Environ. Microbiol.*, 2005, vol. 71 (12), 8537-47

- **DIAWARA et al.** Arsenic, cadmium, lead, and mercury in surface soils, Pueblo, Colorado: implications for population health risk. *Environ. Geochem. Health*, 04 June 2006, vol. 28 (4), 297-315
- Alternatives for Managing the Nation's Complex Contaminated Groundwater Sites. *National Research Council of the National Academies*, 2013
- **BRICKER et al.** Effects of nutrient enrichment in the nation's estuaries: A decade of change. *Harmful Algae*, 2008, vol. 8 (1), 21-32
- **DAUNERT et al.** Genetically engineered whole-cell sensing systems: coupling biological recognition with reporter genes. *Chemical Reviews*, 2000, vol. 100 (7), 2705-2737
- **OGUCHI et al.** pH Condition in temperature shift cultivation enhances cell longevity and specific hMab productivity in CHO culture. *Cytotechnology*, 2006, vol. 52 (3), 199-207
- **AL-FAGEEH et al.** The cold-shock response in cultured mammalian cells: Harnessing the response for the improvement of recombinant protein production. *Biotechnol. Bioeng*, 2006, vol. 93, 829-835

- **MARCHANT, R.J. et al.** Metabolic rates, growth phase, and mRNA levels influence cell-specific antibody production levels from in vitro cultured mammalian cells at sub-physiological temperatures. *Mol. Biotechnol*, 2008, vol. 39, 69-77
- **PATTISON et al.** *Biotechnol. Prog.*, 2000, vol. 16, 769-774
- **MANIATIS et al.** Molecular Cloning. A Laboratory Manual. Cold Spring Harbor Laboratory, 1982
- **MANIATIS et al.** *Science*, 1987, vol. 236, 1237
- **VOSS et al.** *Trends Biochem. Sci.*, 1986, vol. 11, 287
- **STOLPER et al.** Aerobic growth at nanomolar oxygen concentrations. *PNAS*, 2010, vol. 107 (44), 18755-18760
- **M. SØNDERGAARD**. Encyclopedia of Inland Waters. Academic Press, 852-859
- **C. TOBIAS et al.** Coastal Wetlands: An Integrated Ecosystem Approach. Elsevier, 2019, 539-596
- Computational Molecular Biology. Oxford University Press, 1988
- Biocomputing Informatics and Genome Projects. Academic Press, 1993
- Computer Analysis of Sequence Data. Humana Press, 1994
- **VON HEINJE, G.** Sequence Analysis in Molecular Biology. Academic Press, 1987
- Sequence Analysis Primer. Stockton Press, 1991
- **CARILLO et al.** *SIAM J. Applied Math.*, 1988, vol. 48, 1073
- **DAYHOFF et al.** *in Atlas of Protein Sequence and Structure*, 1978, vol. 5
- **DEVEREUX et al.** *Nucl. Acid Res.*, 1984, vol. 12, 387
- Genetics Computer Group. University of Wisconsin
- **DAYHOFF et al.** *Atlas of Protein Sequence and Structure*, 1978, vol. 5, 345-352
- **HENIKOFF et al.** *Proc. Natl. Acad. Sci. U.S.A.*, 1992, vol. 89, 10915-10919

- **NEEDLEMAN et al.** *J. Mol. Biol.*, 1970, vol. 48, 443-453
- **HAN et al.** Novel signal peptides improve the secretion of recombinant Staphylococcus aureus Alpha toxinH35L in Escherichia coli,''. *AMB Express*, 12 May 2017, vol. 7, 93
- **GRAHAM et al.** *Virology*, 1973, vol. 52, 456
- **SAMBROOK et al.** *Molecular Cloning: A Laboratory Manual*, 2001
- **DAVIS et al.** Basic Methods in Molecular Biology. Elsevier, 1986
- **CHU et al.** *Gene*, 1981, vol. 13, 197
- **CAYLEY S et al.** Large changes in cytoplasmic biopolymer concentration with osmolality indicate that macromolecular crowding may regulate protein-DNA interactions and growth rate in osmotically stressed Escherichia coli K-12. *J. Mol. Recognit.*, 2004, vol. 17 (5), 488-96
- **SAMBROOK et al.** Molecular Cloning: A Laboratory Guide. Cold Spring Harbor Press, 1989, vol. 1-3
- **SANGER, F. et al.** *Proc. Natl. Acad. Sci. USA*, 1977, vol. 74, 5463-5467
- **BUERMANS, H. P. J.** ; **DEN DUNNEN, J. T**. Next generation sequencing technology: Advances and applications. *Biochimica et Biophysica Acta - Molecular Basis of Disease*, 2014, vol. 1842 (10), 1932-1941
- **HAM et al.** *Meth. Enz.*, 1979, vol. 58, 44
- **BARNES et al.** *Anal. Biochem.*, 1980, vol. 102, 255
- **CHALLENGER, C.A.** An acoustic wave-based technology for cell harvesting applications may help enable continuous manufacturing,. *BioPharm International*, 2017, vol. 30 (9), 30
- **D. G. GIBSON** ; **L. YOUNG** ; **R. Y. CHUANG** ; **J. C. VENTER** ; **C. A. HUTCHISON** ; **H. O. SMITH**. Enzymatic assembly of DNA molecules up to several hundred kilobases. *Nat Methods*, 2009, vol. 6 (5), 343-345
- **DUFFY et al.** Rapid Prototyping of Microfluidic Systems in Poly(dimethylsiloxane). *Analytical Chemistry*, 1998, vol. 70 (23), 4974-4984